# EUROPEAN PATENT SPECIFICATION

(11) **EP 2 903 078 B1**
(45) Date of publication and mention of the grant of the patent: **25.10.2017**
(21) Application number: 13842105.2
(22) Date of filing: 25.09.2013
(51) Int. Cl.: H01M 14/00, C07D 213/79, C07D 333/16, C07D 333/20, C07D 401/14, C07D 409/04, C07D 409/06, C07D 409/14, C07D 417/14, C07D 495/04, C09B 57/10, C09B 67/44, H01L 31/04, C07F 15/00

(54) **METAL COMPLEX DYE, PHOTOELECTRIC CONVERSION ELEMENT, DYE-SENSITIZED SOLAR CELL, DYE SOLUTION, AND DYE-ADSORBED ELECTRODE**
METALLKOMPLEXFARBSTOFF, FOTOELEKTRISCHES UMWANDLUNGSELEMENT, FARBSTOFFSENSIBILISIERTE SOLARZELLE, FARBSTOFFLÖSUNG UND FARBSTOFFADSORBIERTE ELEKTRODE
COLORANT À COMPLEXE MÉTALLIQUE, ÉLÉMENT DE CONVERSION PHOTOÉLECTRIQUE, CELLULE SOLAIRE À COLORANT, SOLUTION DE COLORANT, ET ÉLECTRODE À COLORANT ADSORBÉ

(30) Priority: 28.09.2012 JP 2012218755; 29.07.2013 JP 2013156801
(43) Date of publication of application: 05.08.2015
(73) Proprietor: FUJIFILM Corporation, Tokyo 106-8620 (JP)
(72) Inventor: KOBAYASHI, Katsumi, Ashigarakami-gun Kanagawa 258-8577 (JP); NOMURA, Kimiatsu, Ashigarakami-gun Kanagawa 258-8577 (JP)
(74) Representative: Hoffmann Eitle
(86) International application number: PCT/JP2013/075950
(87) International publication number: WO 2014/050911

(56) References cited:
- EP-A2- 2 372 734
- JP-A- 2006 298 882
- JP-A- 2010 013 500
- JP-A- 2011 195 745
- JP-A- 2013 178 968
- US-A1- 2005 081 911
- US-A1- 2007 265 443
- US-A1- 2007 277 871
- OSMAN DAYAN ET AL.: 'Synthesis and catalytic activity of ruthenium(II) complexes containing pyridine-based tridentate triamines ('NNN') and pyridine carboxylate ligands (NO)' INORGANICA CHIMICA ACTA vol. 392, 08 March 2012, pages 246 - 253, XP055240127
- CHUN-CHENG CHOU ET AL.: 'Ruthenium(II) Sensitizers with Heteroleptic Tridentate Chelates for Dye-Sensitized Solar Cells' ANGEWANDTE CHEMIE INTERNATIONAL EDITION vol. 50, 2011, pages 2054 - 2058, XP055195744
- BO-SO CHEN ET AL.: 'Neutral, panchromatic Ru(II) terpyridine sensitizers bearing pyridine pyrazolate chelates with superior DSSC performance' CHEMICAL COMMUNICATIONS 08 September 2009, pages 5844 - 5846, XP055195747
- TAKASHI FUNAKI ET AL.: 'Efficient Panchromatic Sensitization of Nanocrystalline Ti02-based Solar Cells Using 2-Pyridinecarboxylate- substituted Ruthenium(II) Complexes' CHEMISTRY LETTERS vol. 38, no. 1, 2009, pages 62 - 63, XP055240272
- TAKASHI FUNAKI ET AL.: 'A 2- quinolinecarboxylate-substituted ruthenium(II) complex as a new type of sensitizer for dye- sensitized solar cells' INORGANICA CHEMICA ACTA vol. 362, 29 October 2008, pages 2519 - 2522, XP026020376

## Description

### TECHNICAL FIELD

The present invention relates to a metal complex dye, a photoelectric conversion element, a dye-sensitized solar cell, a dye solution, and dye-adsorbed electrode.

### BACKGROUND ART

Photoelectric conversion elements are used in various photosensors, copying machines, solar cells or the like. These photoelectric conversion elements have adopted various systems to be put into use, such as elements utilizing metals, elements utilizing semiconductors, elements utilizing organic pigments or dyes, or elements utilizing combinations of these. In particular, solar cells that make use of non-exhaustive solar energy do not necessitate fuels, and full-fledged practicalization of solar cells as an inexhaustible clean energy is being highly expected. Above all, research and development of silicon-based solar cells have long been in progress. Based on a policy-wise consideration in each country, widespread use thereof is still in progress. However, silicon is an inorganic material, and has its own limitations in terms of improving throughput and cost.

Under such circumstances, research is being vigorously carried out on dye-sensitized solar cells. Especially, to have built momentum toward such research is research results by Graetzel et al. of École Polytechnique Fédérale de Lausanne in Switzerland. They employed a structure in which a dye formed from a ruthenium complex was fixed at the surface of a porous titanium oxide thin film, and realized a photoelectric conversion efficiency that was comparable to that of amorphous silicon. Thus, the dye-sensitized solar cells that can be produced even without using an expensive vacuum apparatus instantly attracted the attention of researchers all over the world.

Hitherto, as metal complex dyes to be used in photoelectric conversion elements, dyes generally called as N3, N719, Z907, and J2 have been developed. However, conventional dye-sensitized solar cells did not have sufficient production stability required for actual application.

Metal complex dyes usually have a ligand in which a nitrogen atom of a pyridine ring such as bipyridyl or terpyridyl is a coordinating atom. Recently, studies have been also made on ligands which can substitute them (see, Patent Literature 1 to 3, for example). However, the results are not necessarily satisfactory. Further, solar cells get attention to and are highly expected as energy sources in place of nuclear power generation, and thus early practical application as solar sell has been demanded.

Further metal complex dyes to be used in photoelectric conversion elements are described in JP 2006 298882; Angewandte Chemie International Edition, vol. 50, 2011, pages 2054-2058; Chemical Communications. 8 September 2009. pages 5844-5846; Chemistry Letters, vol. 38, no. 1, 2009, pages 62-63; and Inorganic Chemica Acta, vol. 362, 29 October 2008, pages 2519-2522.

### CITATION LIST

### Patent Literatures

Patent Literature 1: US Patent Application Publication No. 2005/0081911 A1
Patent Literature 2: JP-A-2010-13500 ("JP-A" means unexamined published Japanese patent application)
Patent Literature 3: JP-A-2011-195745

### SUMMARY OF THE INVENTION

### TECHNICAL PROBLEM

With regard to electrode unevenness, thermal deterioration, properties at high speed, and initial properties, which would become issues in terms of production stability required for practical application, conventional dyes are not sufficient, and thus there has been a demand for further improvements.

Thus, the present invention aims to provide a photoelectric conversion element which has improved production stability in addition to an improvement of those performances. That is, the present invention aims to provide a photoelectric conversion element which is further improved with photoelectric conversion efficiency and durability, shows stable performance reproducibility and little deviation in product quality, has suitability for high speed production (that is, high speed suitability), and causes less thermal deterioration; a metal complex dye for obtaining the same, a dye-adsorbed electrode and a dye solution for producing the same, and a dye-sensitized solar cell.

### SOLUTION TO PROBLEM

In order to improve photoelectric conversion efficiency (η), it is necessary to improve both of short-circuit current density (Jsc) and open-circuit voltage (Voc). The present inventors made investigations on metal complex dyes, by sorting out possible factors for improvements: coating property and adsorptivity of a metal complex dye to a surface of fine semiconductor particles such as titanium oxide; stability of a metal complex dye as a complex; prevention of reverse electron transport of electrons injected to titanium oxide, to an electrolyte (for example, I₃⁻); adsorption or desorption of a metal complex dye to a surface of fine semiconductor particles and the rate thereof; and bimolecular or multi-molecular association of metal complex dye.

As a result, the present inventors found that, with regard to improvement of coordination power to a center metal, coating of a metal complex dye to the surface of fine semiconductor particles, a dye association property, and stability of a metal complex dye per se, these performances greatly change according to a type of a coordinating atom and of a substituent substituted onto a dye skeleton, and made the present invention.

That is, the tasks of the present invention can be achieved by the following means.

(1) A photoelectric conversion element, containing:
   an electrically conductive support;
   a photoconductor layer containing an electrolyte;
   a charge-transfer layer containing an electrolyte; and
   a counter electrode;
   wherein the photoconductor layer contains semiconductor fine-particles carrying a metal complex dye represented by Formula (I):

   M(LD)(LA)(X)_{mX}·(CI)_{mY} Formula (I)

   wherein M represents Fe²⁺, Ru²⁺ or Os²⁺;
   LD represents a ligand represented by Formula (DL);
   LA represents a bidentate or tridentate ligand having a nitrogen-containing aromatic heterocyclic skeleton and at least one acidic group;
   X represents a monodentate or bidentate ligand; mX represents an integer of 0 to 3;
   CI represents a counter ion necessary for neutralizing an electric charge; and
   mY represents an integer of 0 to 2; 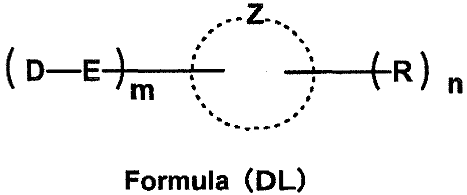 wherein Z represents a group of nonmetallic atoms necessary for forming a ring, and it may contain, as a ring-forming atom, a coordinating atom which coordinates to the aforementioned M; D represents a coordinating atom selected from an oxygen atom and a nitrogen atom and coordinates to the aforementioned M; E represents a single bond, -O-, -N(R¹)-, -C(R²)₂-, -C(=R³)-, -C(=O)-, -C(=NR¹)-, or -C(R²)₂-C(=O)-, in which R¹ and R² each independently represent a hydrogen atom, an alkyl group, an aryl group, a heteroaryl group, an alkoxy group, or an aryloxy group; R³ represents an alkylidene group, an alkenylidene group, or a cycloalkylidene group; two R²s may be the same or different from one another; R represents a substituent; m and n each independently represent an integer of 1 or more;
   wherein at least one coordinating atom is a coordinating atom A and, among Rs in the number of n, at least one is a substituent W, and D never forms a ring with E or Z;
   herein, the coordinating atom A is an oxygen or nitrogen atom which does not have an unsaturated bonding arm, and the substituent W is an ethenyl group, an ethynyl group, an aryl group, or a heteroaryl group, each of which is substituted with a monovalent alkyl group having 6 to 18 carbon atoms or with a substituent having the alkyl group.
(2) The photoelectric conversion element described in the above item (1), wherein the ligand represented by Formula (DL) is represented by any one of Formulas (DL-1) to (DL-3): 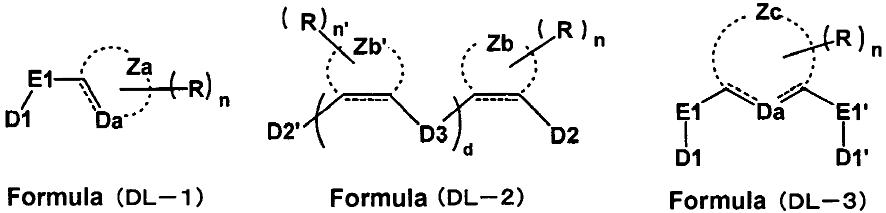 wherein R and n each have the same meaning as R and n in Formula (DL); n' represents an integer of 0 or more; Za, Zb, Zb' and Zc each represent a group of nonmetallic atoms necessary for forming a ring; the bond linking the carbon atom to which -E1-D1 is attached and Da, the bond linking the carbon atom to which D2 or D2' is attached and the carbon atom to which D3 is attached, and the bond linking the carbon atom to which -E1-D1 or -E1'-D1' is attached and Da may be either a single bond or a double bond; D1 to D3, D1', D2' and Da each independently represent a coordinating atom which coordinates to M; D1 to D3, D1' and D2' each represent an atom selected from the group consisting of an oxygen atom and a nitrogen atom; Da represents an atom selected from the group consisting of an oxygen atom, a nitrogen atom, and a carbon atom; E1 and E1' each independently represent a single bond, -O-,-N(R¹)-, -C(R²)₂-, -C(=R³)-, -C(=O)-, -C(=NR¹)-, or -C(R²)₂-C(=O)-, in which R¹ and R² each independently represent a hydrogen atom, an alkyl group, an aryl group, a heteroaryl group, an alkoxy group, or an aryloxy group; R³ represents an alkylidene group, an alkenylidene group, or a cycloalkylidene group; two R²s may be the same or different from one another; d represents 0 or 1; when d is 1, Zb and Zb' may be bonded to each other to form a ring; and when n and n' each are an integer of 2 or more, plural Rs may be bonded with each other to form a ring.
(3) The photoelectric conversion element described in the above item (1) or (2), wherein the ligand represented by Formula (DL) is represented by any one of Formulas (DL-1a), (DL-1b), (DL-2a), (DL-2b), (DL-3a), and (DL-3b): 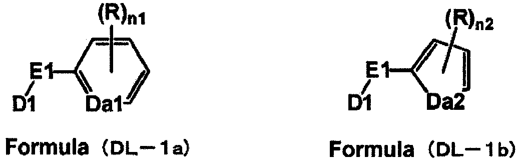 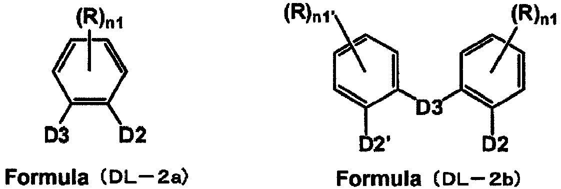 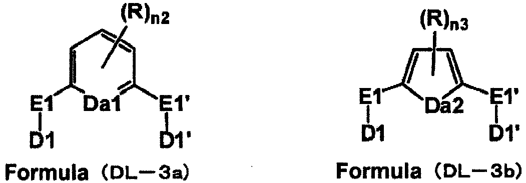 wherein R has the same meaning as R in Formula (DL); n1 represents an integer of 1 to 4; n2 represents an integer of 1 to 3; n3 represents 1 or 2; n1' represents an integer of 0 to 4; D1 to D3, D1', D2', Da1, and Da2 each independently represent a coordinating atom which coordinates to M; D1 to D3, D1', and D2' each represent an atom selected from the group consisting of an oxygen atom and a nitrogen atom; Da1 and Da2 each represent an atom selected from the group consisting of an oxygen atom, a nitrogen atom and a carbon atom; E1 and E1' each independently represent a single bond, -O-, -N(R¹)-, -C(R²)₂-, -C(=R³)-, -C(=O)-, -C(=NR¹)-, or -C(R²)₂-C(=O)-, in which R¹ and R² each independently represent a hydrogen atom, an alkyl group, an aryl group, a heteroaryl group, an alkoxy group, or an aryloxy group; R³ represents an alkylidene group, an alkenylidene group, or a cycloalkylidene group; two R²s may be the same or different from one another; and when n1 to n3 and n1' each are an integer of 2 or more, plural Rs may be bonded with each other to form a ring.
(4) The photoelectric conversion element described in any one of the above items (1) to (3), wherein the substituent W is 1) an ethenyl group or ethynyl group having an aryl group or heteroaryl group which is substituted with an alkyl group having 6 to 18 carbon atoms, an alkylamino group having 6 to 18 carbon atoms, an alkoxy group having 6 to 18 carbon atoms, or an alkylthio group having 6 to 18 carbon atoms, at position 2, or 2) an aryl group or heteroaryl group substituted with an alkyl group having 6 to 18 carbon atoms, an alkylamino group having 6 to 18 carbon atoms, an alkoxy group having 6 to 18 carbon atoms, or an alkylthio group having 6 to 18 carbon atoms.
(5) The photoelectric conversion element described in any one of the above items (1) to (4), wherein the substituent W is represented by any one of Formulas (W-1) to (W-6): 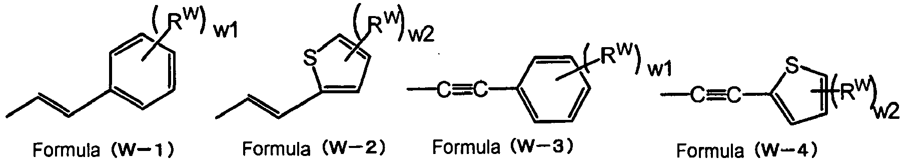 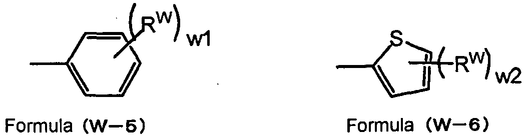 wherein R^{W} represents a monovalent alkyl group having 6 to 18 carbon atoms or a group having the alkyl group; w1 represents an integer of 1 to 5; and w2 represents an integer of 1 to 3.
(6) The photoelectric conversion element described in any one of the above items (1) to (5), wherein LA is represented by any one of Formulas (AL-1) to (AL-6): 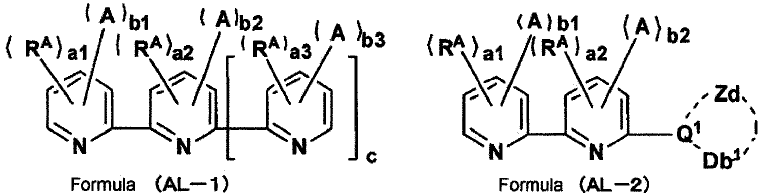 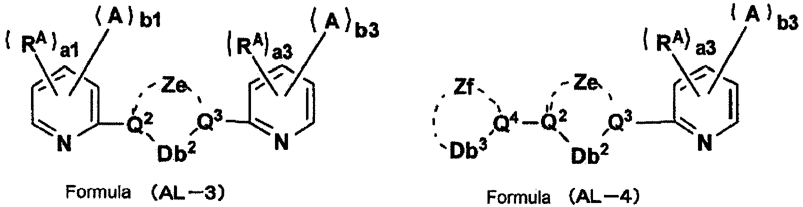 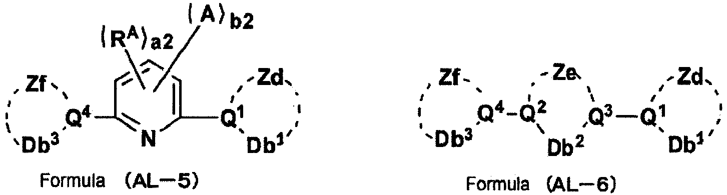 wherein Zd, Ze, and Zf each independently represent a benzene ring, a pyrrole ring, an imidazole ring, a pyrazole ring, a pyrazine ring, a pyrimidine ring, a pyridazine ring, a triazole ring, an oxazole ring, a triazine ring, a thiazole ring, an isothiazole ring, an oxazole ring, an isoxazol ring, a furan ring, a thiophene ring, a pyrrolidine ring, a piperidine ring, a morpholine ring, a piperazine ring, a tetrahydrofuran ring, a tetrahydropyran ring, a 4H-pyran ring, a 1,4-dihydropyridine ring, a tetradehydromorpholine ring, or a benzene-fused ring of these rings; A represents an acidic group; Q¹ to Q⁴ each independently represent a carbon atom or a nitrogen atom; Db¹ to Db³ each independently represent a nitrogen atom having a lone electron pair, an anionic nitrogen atom, or an anionic carbon atom; R^{A} represents a substituent; a1, a3, b1, and b3 each independently represent an integer of 0 to 4; a2 and b2 each independently represent an integer of 0 to 3; c represents 0 or 1; and
   the ligands represented by Formulas (AL-1) to (AL-6) have at least one acidic group.
(7) The photoelectric conversion element described in any one of the above items (1) to (6), wherein LA is represented by Formula (AL-1): 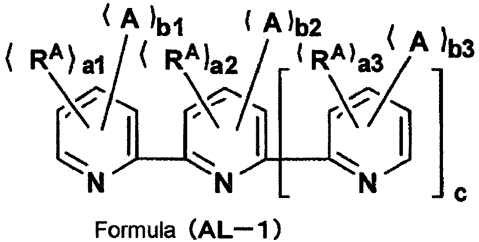 wherein A represents an acidic group; R^{A} represents a substituent; a1, a3, b1 and b3 each independently represent an integer of 0 to 4; a2 and b2 each independently represent an integer of 0 to 3; c represents 0 or 1; and
   the ligand represented by Formula (AL-1) has at least one acidic group.
(8) The photoelectric conversion element described in any one of the above items (1) to (7), wherein X in Formula (I) is NCS⁻, Cl⁻, Br⁻, I⁻, CN⁻, NCO⁻, or H₂O.
(9) The photoelectric conversion element described in any one of the above items (1) to (8), wherein CI in Formula (I) is a halogen ion, an arylsulfonate ion, an aryldisulfonate ion, an alkylsulfate ion, a sulfate ion, a thiocyanate ion, a perchlorate ion, a tetrafluoroborate ion, a hexafluorophosphate ion, an acetate ion, a trifluoromethanesulfonate ion, an ammonium ion, an alkali metal ion, or a hydrogen ion.
(10) A dye-sensitized solar cell, having the photoelectric conversion element described in any one of the above items (1) to (9).
(11) A metal complex dye represented by Formula (I):

   M(LD)(LA)(X)_{mX}·(CI)_{mY} Formula (I)

   wherein M represents Fe²⁺, Ru²⁺ or Os²⁺;
   LD represents a ligand represented by Formula (DL);
   LA represents a bidentate or tridentate ligand having a nitrogen-containing aromatic heterocyclic skeleton and at least one acidic group.
   X represents a monodentate or bidentate ligand; mX represents an integer of 0 to 3;
   CI represents a counter ion necessary for neutralizing an electric charge; and
   mY represents an integer of 0 to 2; 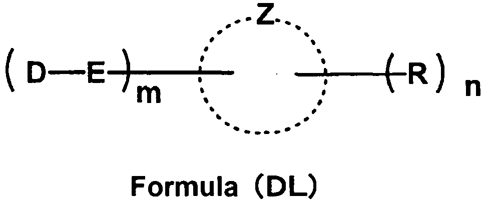 wherein Z represents a group of nonmetallic atoms necessary for forming a ring, and it may contain, as a ring-forming atom, a coordinating atom which coordinates to the aforementioned M; D represents a coordinating atom selected from an oxygen atom and a nitrogen atom and coordinates to the aforementioned M; E represents a single bond, -O-, -N(R¹)-, -C(R²)₂-, -C(=R³)-, -C(=O)-, -C(=NR¹)-, or -C(R²)₂-C(=O)-, in which R¹ and R² each independently represent a hydrogen atom, an alkyl group, an aryl group, a heteroaryl group, an alkoxy group, or an aryloxy group; R³ represents an alkylidene group, an alkenylidene group, or a cycloalkylidene group; two R²s may be the same or different from one another; R represents a substituent; m and n each independently represent an integer of 1 or more;
   wherein at least one coordinating atom is a coordinating atom A and, among Rs in the number of n, at least one is a substituent W, and D never forms a ring with E or Z; herein, the coordinating atom A is an oxygen or nitrogen atom which does not have an unsaturated bonding arm, and the substituent W is an ethenyl group, an ethynyl group, an aryl group, or a heteroaryl group, each of which is substituted with a monovalent alkyl group having 6 to 18 carbon atoms or with a substituent having the alkyl group.
(12) The metal complex dye described in the above item (11), wherein the ligand represented by Formula (DL) is represented by any one of Formulas (DL-1) to (DL-3): 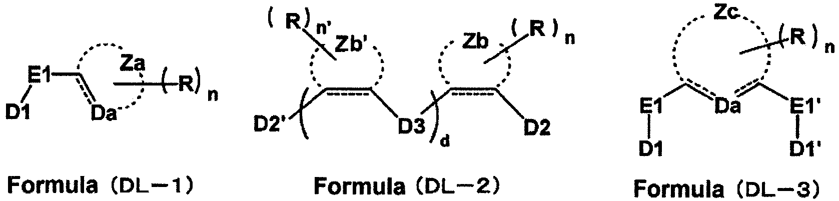 wherein R and n each have the same meaning as R and n in Formula (DL); n' represents an integer of 0 or more; Za, Zb, Zb' and Zc each represent a group of nonmetallic atoms necessary for forming a ring; the bond linking the carbon atom to which -E1-D1 is attached and Da, the bond linking the carbon atom to which D2 or D2' is attached and the carbon atom to which D3 is attached, and the bond linking the carbon atom to which -E1-D1 or -E1'-D1' is attached and Da may be either a single bond or a double bond; D1 to D3, D1', D2' and Da each independently represent a coordinating atom which coordinates to M; D1 to D3, D1' and D2' each represent an atom selected from the group consisting of an oxygen atom and a nitrogen atom; Da represents an atom selected from the group consisting of an oxygen atom, a nitrogen atom, and a carbon atom; E1 and E1' each independently represent a single bond, -O-,-N(R¹)-, -C(R²)₂-, -C(=R³)-, -C(=O)-, -C(=NR¹)-, or -C(R²)₂-C(=O)-, in which R¹ and R² each independently represent a hydrogen atom, an alkyl group, an aryl group, a heteroaryl group, an alkoxy group, or an aryloxy group; R³ represents an alkylidene group, an alkenylidene group, or a cycloalkylidene group; two R²s may be the same or different from one another; d represents 0 or 1; when d is 1, Zb and Zb' may be bonded to each other to form a ring; and when n and n' each are an integer of 2 or more, plural Rs may be bonded with each other to form a ring.
(13) The metal complex dye described in the above item (11) or (12), wherein the ligand represented by Formula (DL) is represented by any one of Formulas (DL-1a), (DL-1b), (DL-2a), (DL-2b), (DL-3a) and (DL-3b): 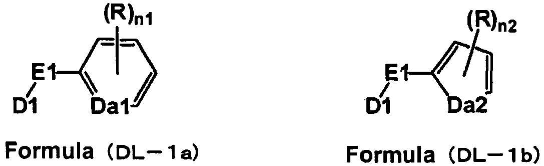 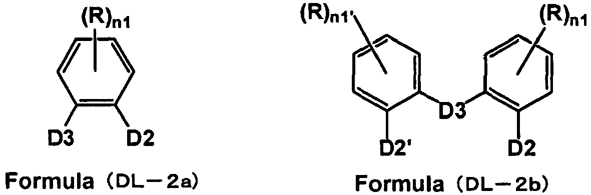 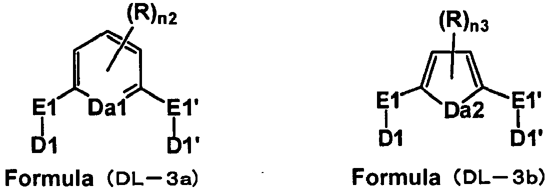 wherein R has the same meaning as R in Formula (DL); n1 represents an integer of 1 to 4; n2 represents an integer of 1 to 3; n3 represents 1 or 2; n1' represents an integer of 0 to 4; D1 to D3, D1', D2', Da1, and Da2 each independently represent a coordinating atom which coordinates to M; D1 to D3, D1', and D2' each represent an atom selected from the group consisting of an oxygen atom and a nitrogen atom; Da1 and Da2 each represent an atom selected from the group consisting of an oxygen atom, a nitrogen atom and a carbon atom; E1 and E1' each independently represent a single bond, -O-, -N(R¹)-, -C(R²)₂-, -C(=R³)-, -C(=O)-, -C(=NR¹)-, or -C(R²)₂-C(=O)-, in which R¹ and R² each independently represent a hydrogen atom, an alkyl group, an aryl group, a heteroaryl group, an alkoxy group, or an aryloxy group; R³ represents an alkylidene group, an alkenylidene group, or a cycloalkylidene group; two R²s may be the same or different from one another; and when n1 to n3 and n1' each are an integer of 2 or more, plural Rs may be bonded with each other to form a ring.
(14) The metal complex dye described in any one of the above items (11) to (13), wherein the substituent W is 1) an ethenyl group or ethynyl group having, at position 2, an aryl group or heteroaryl group which is substituted with an alkyl group having 6 to 18 carbon atoms, an alkylamino group having 6 to 18 carbon atoms, an alkoxy group having 6 to 18 carbon atoms, or an alkylthio group having 6 to 18 carbon atoms, or 2) an aryl group or heteroaryl group substituted with an alkyl group having 6 to 18 carbon atoms, an alkylamino group having 6 to 18 carbon atoms, an alkoxy group having 6 to 18 carbon atoms, or an alkylthio group having 6 to 18 carbon atoms.
(15) The metal complex dye described in any one of the above items (11) to (14), wherein the substituent W is represented by any one of Formulas (W-1) to (W-6): 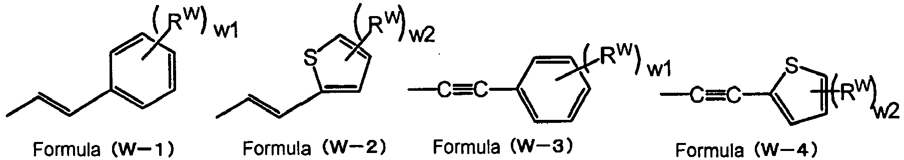 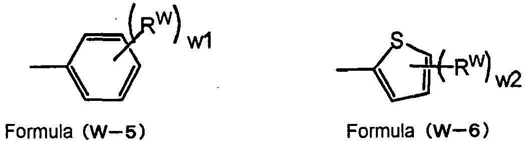 wherein R^{W} represents a monovalent alkyl group having 6 to 18 carbon atoms or a group having the alkyl group; w1 represents an integer of 1 to 5; and w2 represents an integer of 1 to 3.
(16) The metal complex dye described in any one of the above items (11) to (15), wherein LA is represented by any one of Formulas (AL-1) to (AL-6): 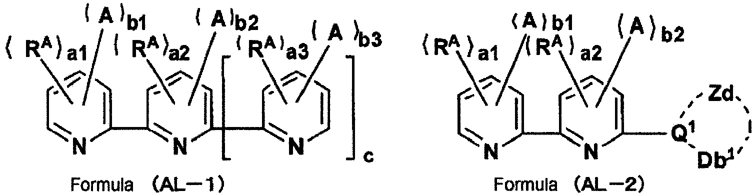 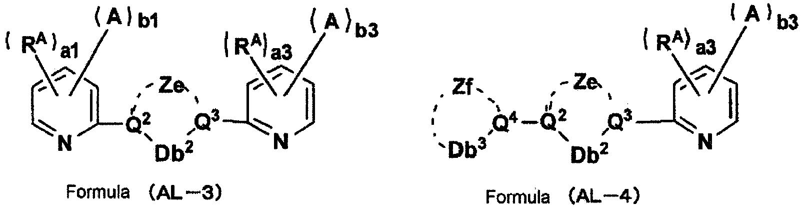 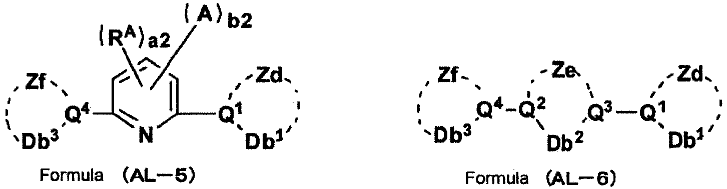 wherein Zd, Ze, and Zf each independently represent a benzene ring, a pyrrole ring, an imidazole ring, a pyrazole ring, a pyrazine ring, a pyrimidine ring, a pyridazine ring, a triazole ring, an oxazole ring, a triazine ring, a thiazole ring, an isothiazole ring, an oxazole ring, an isoxazol ring, a furan ring, a thiophene ring, a pyrrolidine ring, a piperidine ring, a morpholine ring, a piperazine ring, a tetrahydrofuran ring, a tetrahydropyran ring, a 4H-pyran ring, a 1,4-dihydropyridine ring, a tetradehydromorpholine ring, or a benzene-fused ring of these rings; A represents an acidic group; Q¹ to Q⁴ each independently represent a carbon atom or a nitrogen atom; Db¹ to Db³ each independently represent a nitrogen atom having a lone electron pair, an anionic nitrogen atom, or an anionic carbon atom; R^{A} represents a substituent; a1, a3, b1, and b3 each independently represent an integer of 0 to 4; a2 and b2 each independently represent an integer of 0 to 3; c represents 0 or 1; and
   the ligands represented by Formulas (AL-1) to (AL-6) have at least one acidic group.
(17) The metal complex dye described in any one of the above items (11) to (16), wherein LA is represented by Formula (AL-1): 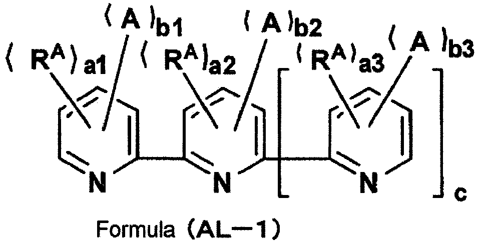 wherein A represents an acidic group; R^{A} represents a substituent; a1, a3, b1 and b3 each independently represent an integer of 0 to 4; a2 and b2 each independently represent an integer of 0 to 3; c represents 0 or 1; and
   the ligand represented by Formula (AL-1) has at least one acidic group.
(18) A dye solution, containing the metal complex dye described in any one of the above items (11) to (17) dissolved in an organic solvent.
(19) The dye solution described in the above item (18), containing, in the organic solvent, the metal complex dye in an amount of 0.001 to 0.1 mass% and water in an amount controlled to 0.1 mass% or less.
(20) A dye-adsorbed electrode for a dye-sensitized solar cell, having a photoconductor layer which is obtained by coating an electrically-conductive support which has been applied with semiconductor fine particles with a dye solution described in (18) or (19), followed by reaction and curing.

In the present specification, unless otherwise specified, with respect to the carbon-carbon double bond, in a case where the E configuration or the Z configuration exists in the molecule, it may be either one of the two configurations or a mixture thereof. When there are two or more substituents, linking groups, ligands or the like (hereinafter referred to as substituents or the like) represented by a specific symbol, or when two or more substituents or the like are defined at the same time or alternatively, each of the substituents or the like may be the same or different from one another, unless otherwise specified. This also applies to definition of the number of substituents or the like. Further, when a plurality of substituents or the like are close to one another (particularly adjacent to each other), they may be linked to one another to form a ring, unless otherwise specified. Further, a ring, for example, an aliphatic ring, an aromatic ring, or a heterocycle, may be ring-fused to form a fused ring.

In the present specification, each substituent may further be substituted with another substituent, unless otherwise specified.

### ADVANTAGEOUS EFFECTS OF INVENTION

According to the present invention, a photoelectric conversion element which has an additional improvement of photoelectric conversion efficiency and an improvement of durability, and also stable performance reproducibility and little deviation in product quality, a metal complex dye for obtaining the same, a dye-adsorbed electrode and a dye solution for producing the same, and a dye-sensitized solar cell can be provided.

Other and further features and advantages of the invention will appear more fully from the following description, appropriately referring to the accompanying drawings.

### BRIEF DESCRIPTION OF THE DRAWINGS

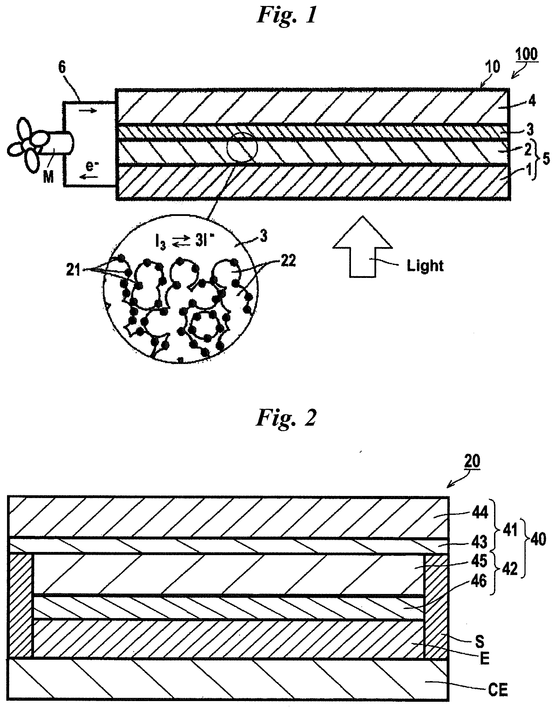

{Fig. 1} Fig. 1 is a cross-sectional view schematically showing one embodiment of the photoelectric conversion element of the present invention, including an enlarged view of the circular portion in the layer thereof.
{Fig. 2} Fig. 2 is a cross-sectional view schematically showing a dye-sensitized solar cell of the second embodiment of the photoelectric conversion element of the present invention.

### MODE FOR CARRYING OUT THE INVENTION

### «Photoelectric conversion element and dye-sensitized solar cell»

In the photoelectric conversion element of the present invention, for example, as shown in Fig. 1, the photoelectric conversion element 10 is composed of: an electrically-conductive support 1; a photoconductor layer 2 containing semiconductor fine particles which has been sensitized by a dye (metal complex dye) 21; a charge-transfer layer 3 which is a hole-transport layer, and a counter electrode 4.

In the present invention, it is preferred that a co-adsorbent has been adsorbed, together with the dye (metal complex dye) 21, onto semiconductor fine particles 22. The electrically conductive support 1 in which the photoconductor layer 2 has been provided functions as a working electrode in the photoelectric conversion element 10. In this embodiment, the photoelectric conversion element 10 is shown as a system 100 utilizing a dye-sensitized solar cell, in which the photoelectric conversion element 10 is made usable for a cell purpose which makes an operation means M to work via an external circuit 6.

In this embodiment, the light-receiving electrode 5 is an electrode having an electrically-conductive support 1, and a photoconductor layer 2 containing semiconductor fine-particles 22 to which a dye (metal complex dye) 21 has been adsorbed. The photoconductor layer 2 is designed according to the intended purpose, and it may have a single-layer structure or a multilayer structure. The dye (metal complex dye) 21 in one photoconductor layer may be a single species or a mixture, but as at least one of them, the metal complex dye of the present invention is used. Light incident to the photoconductor layer 2 excites the dye (metal complex dye) 21. The excited dye has electrons having high energy, and these electrons are transferred from the dye (metal complex dye) 21 to a conduction band of the semiconductor fine particles 22, and further reach the electrically conductive support 1 by diffusion. At this time, the dye (metal complex dye) 21 is in an oxidized form. The electrons on the electrode, while working in an external circuit 6, return through the counter electrode 4 to the photoconductor layer 2 in which the oxidized form of the dye (metal complex dye) 21 and the electrolyte exist, to function as the solar cell.

In the present invention, regarding materials for use in the photoelectric conversion element and the dye-sensitized solar cell, and a method of producing each member, ordinary ones in this kind may be adopted, and reference can be made to, for example, U.S. Patent No. 4,927,721, U.S. Patent No. 4,684,537, U.S. Patent No. 5,084,365, U.S. Patent No. 5,350,644, U.S. Patent No. 5,463,057, U.S. Patent No. 5,525,440, JP-A-7-249790, JP-A-2004-220974 or JP-A-2008-135197. Hereinafter, an outline of main members will be described.

### <Photoconductor layer>

The photoconductor layer is a layer that contains an electrolyte described later and semiconductor fine-particles carrying a sensitizing dye including the metal complex dye of the present invention described later.

First, a metal complex dye for use in the present invention will be described in detail.

### <<Metal complex dye>>

A metal complex dye according to the present invention is represented by Formula (I).

M(LD)(LA)(X)_{mX}·(CI)_{mY} Formula (I)

M represents Fe²⁺, Ru²⁺ or Os²⁺.

LD represents a ligand represented by Formula (DL) described below.

LA represents a bidentate or tridentate ligand having a nitrogen-containing aromatic heterocyclic skeleton and at least one acidic group.

X represents a monodentate or bidentate ligand. mX represents an integer of 0 to 3.

CI represents a counter ion necessary for neutralizing an electric charge.

mY represents an integer of 0 to 2;

### -Ligand LD-

In the present invention, the ligand LD is categorized as a donor ligand, and is represented by the following Formula (DL).

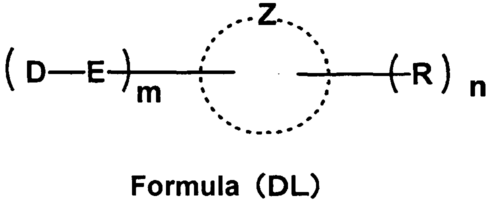

In formula, Z represents a group of nonmetallic atoms necessary for forming a ring, and it may contain, as a ring-forming atom, a coordinating atom which coordinates to the aforementioned M. D represents a coordinating atom selected from an oxygen atom and a nitrogen atom and coordinates to the aforementioned M. E represents a single bond, -O-, -N(R¹)-, -C(R²)₂-, -C(=R³)-, -C(=O)-, -C(=NR¹)-, or -C(R²)₂-C(=O)-. Herein, R¹ and R² each independently represent a hydrogen atom, an alkyl group, an aryl group, a heteroaryl group, an alkoxy group, or an aryloxy group. R³ represents an alkylidene group, an alkenylidene group, or a cycloalkylidene group. Two R²s may be the same or different from one another. R represents a substituent. m and n each independently represent an integer of 1 or more.

However, at least one coordinating atom is the coordinating atom A and; among Rs in the number of n, at least one is the substituent W; and D never forms a ring with E or Z.

Herein, the coordinating atom A is an oxygen atom or nitrogen atom which does not have an unsaturated bonding arm; and the substituent W is an ethenyl group, an ethynyl group, an aryl group, or a heteroaryl group, each of which is substituted with a monovalent alkyl group having 6 to 18 carbon atoms or with a substituent having the alkyl group.

m is preferably an integer of 1 to 3, and more preferably 1 or 2.

n is preferably an integer of 1 to 3, more preferably 1 or 2, and further preferably 1.

### (Coordinating atom A)

In the present invention, the ligand LD has at least one coordinating atom A.

The coordinating atom A is an oxygen atom or nitrogen atom which does not have an unsaturated bonding arm. "Not having an unsaturated bonding arm" means that the bond to an adjacent atom is not an unsaturated bond, as carbonyl (>C=O) for an oxygen atom or a nitrogen atom (=N-) in a pyridine ring for a nitrogen atom, for example. In the case of an oxygen atom, for example, the oxygen atom of -OH and -O⁻, and -O⁻ of -C(=O)-O⁻ can be mentioned. In the case of a nitrogen atom, each nitrogen atom of -NH₂, -NH⁻, -C(=O)N⁻C(=O)-, -C(=O)N⁻SO₂-, and -N⁻SO₂- can be mentioned. Herein, the bonding arm (-) at the right side is substituted with a hydrogen atom or a substituent.

Since the ligand LD is a bidentate or tridentate ligand, it is possible to have one to three coordinating atoms A. To strengthen the coordination to the metal M, it is preferable that one or two coordinating atoms be the coordinating atom A.

The coordinating atom D binds to the ring Z via E (i.e., -E-D).

E represents a single bond, -O-, -N(R¹)-, -C(R²)₂-, -C(=R³)-, -C(=O)-,-C(=NR¹)-, or -C(R²)₂-C(=O)-. Herein, R¹ and R² each independently represent a hydrogen atom, an alkyl group, an aryl group, a heteroaryl group, an alkoxy group, or an aryloxy group. R³ represents an alkylidene group, an alkenylidene group, or a cycloalkylidene group. Two R²s may be the same or different from one another. R represents a substituent.

The number of carbon atoms of the alkyl group for R¹ and R² is preferably 1 to 6, more preferably 1 to 4, and further preferably 1 or 2. Examples of the alkyl group include methyl, ethyl, n-propyl, isopropyl, and n-hexyl.

The number of carbon atoms of the aryl group for R¹ and R² is preferably 6 to 12, more preferably 6 or 7, and further preferably 6. Examples of the aryl group include phenyl, tolyl, and naphthyl.

The number of carbon atoms of the heteroaryl group for R¹ and R² is preferably 0 to 12, more preferably 0 to 6, and further preferably 1 to 5. The heteroatom for forming the ring of the heteroaryl group is preferably an oxygen atom, a nitrogen atom, or a sulfur atom, and a 5-membered ring or a 6-membered ring is preferable. Examples of the heteroaromatic ring of the heteroaryl group include a thiophene ring, a furan ring, pyrrole ring, a pyrazole ring, an imidazole ring, a pyridine ring, and a pyrazine ring.

The number of carbon atoms of the alkoxy group for R¹ and R² is preferably 1 to 6, more preferably 1 to 4, and further preferably 1 or 2. Examples of the alkoxy group include methoxy, ethoxy, n-propoxy, isopropoxy, and n-hexyloxy.

The number of carbon atoms of the aryloxy group for R¹ and R² is preferably 6 to 12, more preferably 6 to 12, and further preferably 6 to 8. Examples of the aryloxy group include phenoxy, tolyloxy, and naphthoxymethoxy.

R¹ and R² each are preferably a hydrogen atom or an alkyl group, and more preferably a hydrogen atom.

The number of carbon atoms of the alkylidene group for R³ is preferably 1 to 6, and more preferably 1 to 4. Examples of the alkylidene group include methylidene, ethylidene, n-propylidene, isopropylidene, and n-hexylidene.

The number of carbon atoms of the alkenylidene group for R³ is preferably 2 to 6, and more preferably 2 to 4. Examples of the alkenylidene group include vinylidene and allylidene.

The number of carbon atoms of the cycloalkylidene group for R³ is preferably 3 to 8, more preferably 3 to 6, and further preferably 5 or 6. Examples of the cycloalkylidene group include cyclopropylidene, cyclopentylidene, and cyclohexylidene.

E is preferably a single bond, -O-, -N(R¹)-, -C(R²)₂-, or -C(R²)₂-C(=O)-; and more preferably a single bond, -O-, -C(R²)₂-, or -C(R²)₂-C(=O)-.

### (Substituent W)

The ligand LD has a substituent W.

The substituent W is an ethenyl group, an ethynyl group, an aryl group, or a heteroaryl group, each of which is substituted with a monovalent alkyl group having 6 to 18 carbon atoms or a group having the alkyl group.

As described herein, "a group having the alkyl group" means a group which contains a monovalent alkyl group having 6 to 18 carbon atoms in any portion thereof, and examples thereof include 2-(monovalent alkyl having 6 to 18 carbon atoms)ethenyl group, 2-(monovalent alkyl having 6 to 18 carbon atoms)ethynyl group, an aryl group substituted with a monovalent alkyl group having 6 to 18 carbon atoms, a heteroaryl group substituted with a monovalent alkyl group having 6 to 18 carbon atoms, an alkoxy group having an alkyl group having 6 to 18 carbon atoms, an alkylthio group having an alkyl group having 6 to 18 carbon atoms, an alkylamino group having an alkyl group having 6 to 18 carbon atoms, and an aryl group or a heteroaryl group each substituted with these.

Herein, the alkylamino group having an alkyl group having 6 to 18 carbon atoms is preferably a dialkylamino group.

If an ethenyl group, an ethynyl group, an aryl group, or a heteroaryl group, each of which is substituted with "a group having the alkyl group" is explained with taking an ethenyl group substituted with "a group having the alkyl group" as an example, for a case in which "a group having the alkyl group" is "2-(monovalent alkyl having 6 to 18 carbon atoms)ethenyl group" described as the first example in the above, it becomes a 2-[2-(monovalent alkyl having 6 to 18 carbon atoms)ethenyl]ethenyl group, that is, (alkyl having 6 to 18 carbon atoms)-CH=CH-CH=CH-.

The monovalent alkyl group having 6 to 18 carbon atoms is a linear or branched alkyl group, and the number of carbon atoms is preferably 8 to 18.

That is, the substituent W is preferably 1) an ethenyl group or ethynyl group "having an aryl group or heteroaryl group which is substituted with an alkyl group having 6 to 18 carbon atoms, an alkylamino group having 6 to 18 carbon atoms, an alkoxy group having 6 to 18 carbon atoms, or an alkylthio group having 6 to 18 carbon atoms" at position 2, or 2) an aryl group or heteroaryl group "substituted with an alkyl group having 6 to 18 carbon atoms, an alkylamino group having 6 to 18 carbon atoms, an alkoxy group having 6 to 18 carbon atoms, or an alkylthio group having 6 to 18 carbon atoms".

Preferred examples of the substituent W include those represented by the following (W-1) to (W-6).

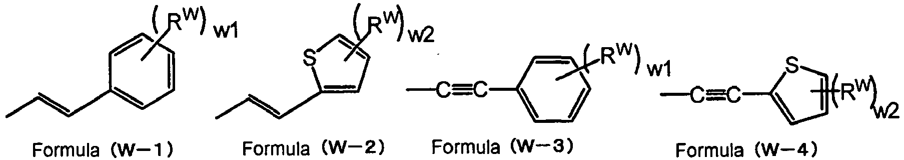

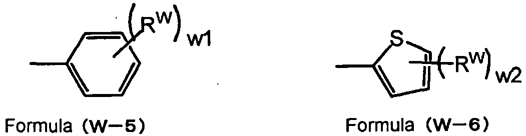

In the formulas, R^{W} represents a monovalent alkyl group having 6 to 18 carbon atoms or a group having the alkyl group; w1 represents an integer of 1 to 5; and w2 represents an integer of 1 to 3.

Herein, "a substituent having the alkyl group" is the group as described above, and the preferred range is also the same.

R^{W} is preferably an alkyl group having 6 to 18 carbon atoms, an alkoxy group having 6 to 18 carbon atoms, an alkylthio group having 6 to 18 carbon atoms, or an alkylamino group having 6 to 18 carbon atoms.

Herein, R^{W} is preferably substituted on a position adjacent to the position at which a bonding arm, an ethynyl group, or an ethenyl group is present (α position), or on a position next to it (β position).

Meanwhile, with regard to the substituent W, R in the above formula (DL) has at least one substituent W, and R also represents a substituent other than that. Examples of the substituent for R include the substituent T which is described later.

However, among the substituent T, a substituent other than the acidic group described later is preferable.

In Formula (DL), Z represents a group of nonmetallic atoms necessary for forming a ring.

The ring formed by Z can be any ring. Examples thereof include an alicycle, an aromatic ring, a non-aromatic hetero ring, a heteroaromatic ring, and a ring in which a different ring or an identical ring is condensed to those rings. The ring formed by Z is preferably an aromatic ring or a heteroaromatic ring, or a ring in which an aromatic ring or a heteroaromatic ring is condensed to those rings. A 5- or 6-membered aromatic ring or a hetero aromatic ring is preferable. The aromatic ring is preferably a benzene ring, and the heteroaromatic ring is preferably a nitrogen-containing heteroaromatic ring. Examples thereof include a pyrrole ring, a pyrazole ring, an imidazole ring, a pyridine ring, a pyrazine ring, a pyrimidine ring, a pyridazine ring, a triazine ring, an indole ring, and a furan ring. Of these, a pyrrole ring and a pyridine ring are preferable.

Meanwhile, when the ligand LD is a tridentate ligand, it is preferable to have the coordinating atom A as coordinating atoms at two ends instead of the center than to have the coordinating atom A for all three of the coordinating atoms.

Among the ligand represented by Formula (DL), a ligand represented by any one of Formulas (DL-1) to (DL-3) is more preferable, and a ligand represented by any one of Formulas (DL-1a), (DL-1b), (DL-2a), (DL-2b), (DL-3a) and (DL-3b) is further preferable.

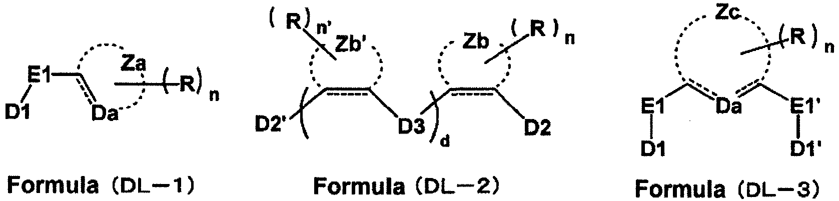

In the formulas, R and n each have the same meaning as R and n in Formula (DL). n' represents an integer of 0 or more. Za, Zb, Zb', and Zc each represent a group of nonmetallic atoms necessary for forming a ring. The bond linking the carbon atom to which -E1-D1 is attached and Da, the bond linking the carbon atom to which D2 or D2' is attached and the carbon atom to which D3 is attached, and the bond linking the carbon atom to which -E1-D1 or -E1'-D1' is attached and Da may be either a single bond or a double bond. D1 to D3, D1', D2' and Da each represent a coordinating atom which coordinates to the above M; and D1 to D3, D1' and D2' each represent an atom selected from the group consisting of an oxygen atom and a nitrogen atom. Da represents an atom selected from the group consisting of an oxygen atom, a nitrogen atom and a carbon atom. E1 and E1' each independently represent a single bond, -O-,-N(R¹)-, -C(R²)₂-, -C(=R³)-, -C(=O)-, -C(=NR¹)-, or -C(R²)₂-C(=O)-. Herein, R¹ and R² each independently represent a hydrogen atom, an alkyl group, an aryl group, a heteroaryl group, an alkoxy group, or an aryloxy group. R³ represents an alkylidene group, an alkenylidene group, or a cycloalkylidene group. Two R²s may be the same or different from one another. d represents 0 or 1. When d is 1, Zb and Zb' may be bonded to each other to form a ring. When n and n' each are an integer of 2 or more, plural R's may be bonded with each other to form a ring.

The ring formed by each of Za and Zc is preferably a heteroaromatic ring containing an oxygen atom or a nitrogen atom, and preferably a 5- or 6-membered ring. Examples of such a ring include a pyrrole ring, a furan ring, a pyridine ring, a pyrazole ring, an imidazole ring, a pyridine ring, a pyrazine ring, a pyrimidine ring, a pyridazine ring, a triazine ring, and an indole ring. Of these, a pyrrole ring and a pyridine ring are preferable.

The rings formed by Zb and Zb' may be the same or different from each other. The ring formed by Zb or Zb' is preferably a benzene ring or a ring in which a heterocycle or an aromatic ring is condensed to a benzene ring. The benzene ring is more preferable.

In E1 and E1', -O-, -N(R¹)-, -C(R²)₂-, -C(=R³)-, -C(=O)-, -C(=NR¹)-, and -C(R²)₂-C(=O)- each have the same meaning as -O-, -N(R¹)-, -C(R²)₂-, -C(=R³)-, -C(=O)-, -C(=NR¹)-, and -C(R²)₂-C(=O)- for E of Formula (DL); and preferable ranges thereof are also the same.

D1 and D1' may be the same or different from one another, and D2 and D2' may be the same or different from one another.

D1, D1', D2 and D2' each are preferably -OH, -NH₂, -O-, -NH⁻, -N⁻SO₂R^{α}, -N⁻(C=O)OR^{α}, or -N⁻(C=O)R^{α}. Herein, R^{α} represents a substituent. Specific examples of such a substituent include the substituent T described later. Of these, an alkyl group is preferable.

D3 is preferably -O- or -N(R¹)-, more preferably -N(R¹)-. R¹ has the same meaning as R¹ of -N(R¹)- in E described above; and a preferable range thereof is also the same.

As -E-D1 and -E'-D1', -CH₂OH, -CH₂-NH₂, -C(=O)-OH, -C(=O)-O⁻, and -C(=O)NH₂ are particularly preferable.

n' is preferably an integer of 0 to 2, more preferably 0 or 1.

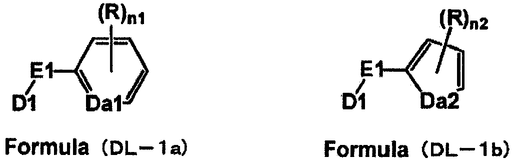

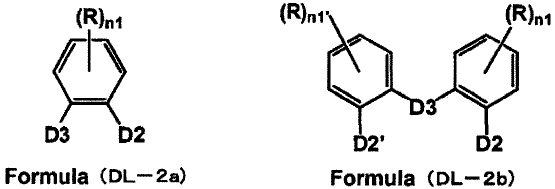

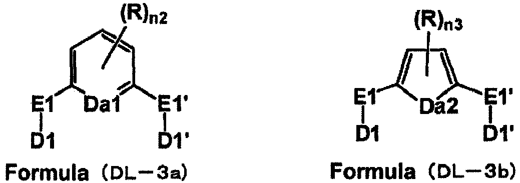

In the formulas, R has the same meaning as R in Formula (DL). n1 represents an integer of 1 to 4. n2 represents an integer of 1 to 3. n3 represents 1 or 2. n1' represents an integer of 0 to 4. D1 to D3, D1', D2', Da1, and Da2 each independently represent a coordinating atom which coordinates to the above M. D1 to D3, D1' and D2' each represent an atom selected from the group consisting of an oxygen atom and a nitrogen atom. Da1 and Da2 each represent an atom selected from the group consisting of an oxygen atom, a nitrogen atom, and a carbon atom. E1 and E1' each independently represent a single bond, -O-. -N(R¹)-, -C(R²)₂-, -C(=R³)-, -C(=O)-, -C(=NR¹)-, or-C(R²)₂-C(=O)-. Herein, R¹ and R² each independently represent a hydrogen atom, an alkyl group, an aryl group, a heteroaryl group, an alkoxy group, or an aryloxy group. R³ represents an alkylidene group, an alkenylidene group, or a cycloalkylidene group. Two R²s may be the same or different from one another. When n1 to n3 and n1' each are an integer of 2 or more, plural R's may be bonded with each other to form a ring.

Among the ligands represented by Formulas (DL-1) to (DL-3), a ligand represented by Formula (DL-1) or (DL-3) is preferable, and a ligand represented by Formula (DL-3) is more preferable.

Further, the ligand represented by Formula (DL-1) is preferably the ligand represented by Formula (DL-1a), and the ligand represented by Formula (DL-3) is preferably the ligand represented by Formula (DL-3a).

Specific examples of the ligand LD are shown below, but the present invention is not limited to these.

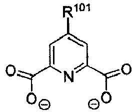

| | R¹⁰¹ |
|---|---|
| LD-1-1 | 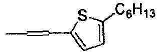 |
| LD-1-2 | 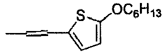 |
| LD-1-3 | 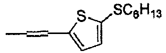 |
| LD-1-4 | 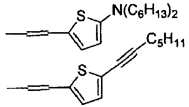 |
| LD-1-5 | |
| LD-1-6 | 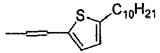 |
| LD-1-7 | 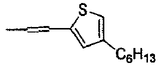 |
| LD-1-8 | 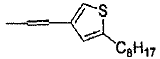 |
| LD-1-9 | 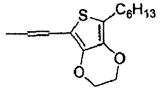 |
| LD-1-10 | 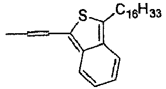 |
| LD-1-11 | 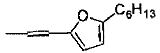 |
| LD-1-12 | 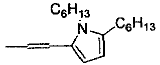 |
| LD-1-13 | 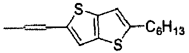 |
| LD-1-14 | 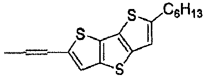 |
| LD-1-15 | 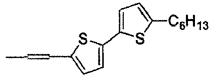 |
| LD-1-16 | 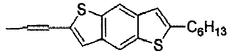 |
| LD-1-17 | 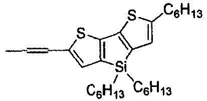 |
| LD-1-18 | 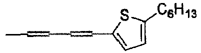 |
| LD-1-19 | 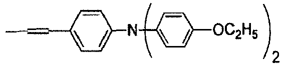 |
| LD-1-20 | 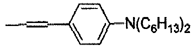 |
| LD-1-21 | 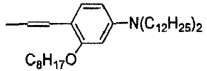 |
| LD-1-22 | 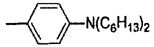 |
| LD-1-23 | 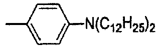 |
| LD-1-24 | 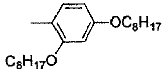 |
| LD-1-25 | 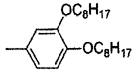 |
| LD-1-26 | 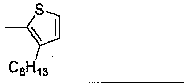 |

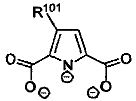

| | R¹⁰¹ |
|---|---|
| LD-1-27 | 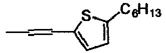 |
| LD-1-28 | 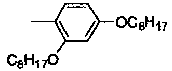 |
| LD-1-29 | 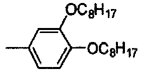 |
| LD-1-30 | 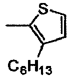 |
| LD-1-31 | 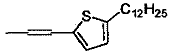 |

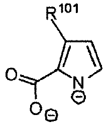

| | R¹⁰¹ |
|---|---|
| LD-1-32 | 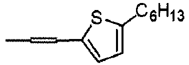 |
| LD-1-33 | 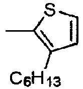 |

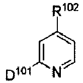

| | D¹⁰¹ | R¹⁰² |
|---|---|---|
| LD-3-1 | -COO^{⊖} | 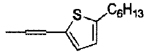 |
| LD-3-2 | -CH₂-O^{⊖} | 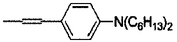 |
| LD-3-3 | -CH₂-O^{⊖} | 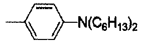 |
| LD-3-4 | -CH₂-O^{⊖} | 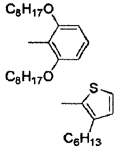 |
| LD-3-5 | -COO^{⊖} | |
| LD-3-6 | 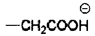 | 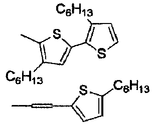 |
| LD-3-7 | -CH₂-NH₂ | |
| LD-3-8 | -CH₂-NH₂ | 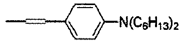 |
| LD-3-9 | -CH₂-NH₂ | 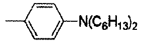 |
| LD-3-10 | -CH₂-NH₂ | 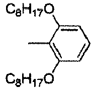 |
| LD-3-11 | -CH₂-NH₂ | 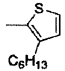 |
| LD-3-12 | 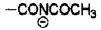 | 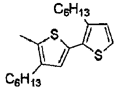 |
| LD-3-13 | 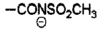 | 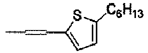 |

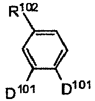

| | D¹⁰¹ | R¹⁰² |
|---|---|---|
| LD-4-1 | -O^{⊖} | 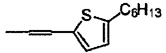 |
| LD-4-2 | -O^{⊖} | 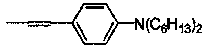 |
| LD-4-3 | -O^{⊖} | 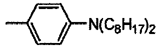 |
| LD-4-4 | -O^{⊖} | 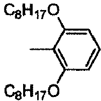 |
| LD-4-5 | -O^{⊖} | 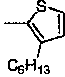 |
| LD-4-6 | -O^{⊖} | 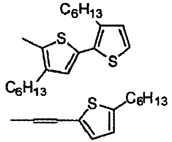 |
| LD-4-7 | -NH₂ | |
| LD-4-8 | -NH₂ | 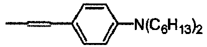 |
| LD-4-9 | -NH₂ | 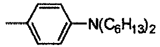 |
| LD-4-10 | -NH₂ | 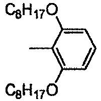 |
| LD-4-11 | -NH₂ | 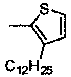 |
| LD-4-12 | 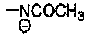 | 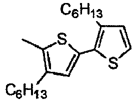 |
| LD-4-13 | 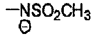 | 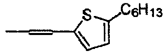 |

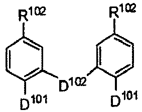

| | D¹⁰¹ | D¹⁰² | R¹⁰² |
|---|---|---|---|
| LD-5-1 | -O^{⊖} | -NH- | 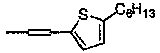 |
| LD-5-2 | -O^{⊖} | -NH- | 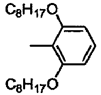 |
| LD-5-3 | -O^{⊖} | -NH- | 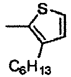 |

These ligands LD can be readily synthesized by methods described in US 2005/0081911 A1, JP-A-2010-13500, JP-A-2011-195745, US 2010/0258175 A1, Japanese Patent No. 4298799, and Angew. Chem. Int. Ed., 2011, 50, 2054-2058, methods described in references cited in these literatures, or methods according to these methods.

### - Ligand LA -

The ligand LA represents a bidentate or a tridentate ligand which has a nitrogen-containing aromatic heterocyclic skeleton and at least one acidic group.

It is preferable that the ligand has at least two acidic groups.

### (Acidic group)

The acidic group means a substituent having a dissociative proton and having a pKa of 11 or lower. Examples thereof include: an acid group which shows an acid property, such as a carboxyl group, a phosphonyl group, a phosphoryl group, a sulfo group, and a boric acid group; or a group having any of these groups; and from the viewpoint of electron injection, a carboxyl group or a group having the same is preferred. Further, the acidic group may be in a dissociation form due to release of a proton, or may be a salt thereof.

The acidic group may be a group in which an acid group connects via a linking group, and the linking group includes an alkylene group. The alkylene group preferably has 1 to 4 carbon atoms.

Further, in a case where the acidic group is a salt thereof, a counter ion which forms the salt is not limited in particular. Examples thereof include positive ions mentioned as a counter ion CI in Formula (I) described above.

In the present invention, from the viewpoint of electron transfer, an acidic group having no linking group is preferred, and a carboxyl group or a salt thereof is preferred in particular.

The ligand LA is preferably a ligand represented by any one of Formulas (AL-1) to (AL-6).

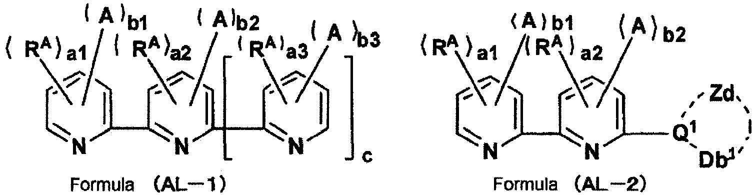

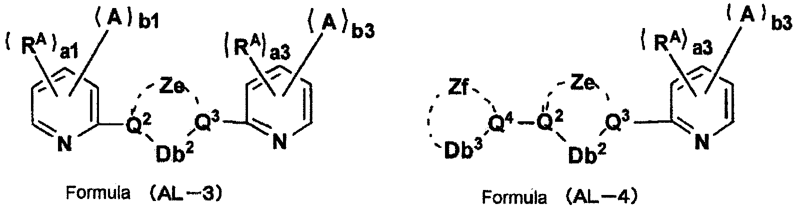

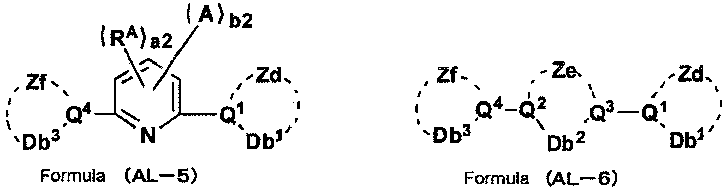

In the formulas, Zd, Ze, and Zf each independently represent a benzene ring, a pyrrole ring, an imidazole ring, a pyrazole ring, a pyrazine ring, a pyrimidine ring, a pyridazine ring, a triazole ring, an oxazole ring, a triazine ring, a thiazole ring, an isothiazole ring, an oxazole ring, an isoxazol ring, a furan ring, a thiophene ring, a pyrrolidine ring, a piperidine ring, a morpholine ring, a piperazine ring, a tetrahydrofuran ring, a tetrahydropyran ring, a 4H-pyran ring, a 1,4-dihydropyridine ring, a tetradehydromorpholine ring, or a benzene-fused ring of these rings. A represents an acidic group. Q¹ to Q⁴ each independently represent a carbon atom or a nitrogen atom. Db¹ to Db³ each independently represent a nitrogen atom having a lone electron pair, an anionic nitrogen atom, or an anionic carbon atom. R^{A} represents a substituent. a1, a3, b1, and b3 each independently represent an integer of 0 to 4. a2 and b2 each independently represent an integer of 0 to 3. c represents 0 or 1.

Herein, the ligands represented by Formulas (AL-1) to (AL-6) have at least one acidic group.

Specific examples of the substituent of R^{A} include the substituent T described later.

Among those substituents, an electron-withdrawing group is preferable. Meanwhile, the electron-withdrawing group is preferably a group which has Hammett's σp value of 0 or higher.

R^{A} is preferably an aryl group or a heteroaryl group. As the aryl group, preferred is a phenyl group which may have a substituent. As the heteroaryl group, it is preferable that the heterocycle of the heteroaryl group be a 5- or 6-membered ring and the ring-forming hetero atom be a nitrogen atom or a sulfur atom. The heterocycle may be condensed with an aromatic ring or a heterocycle.

The aryl group and the heteroaryl group of R^{A} may have a substituent. Specific examples of such a substituent include the substituent T described later.

R^{A} is further preferably a phenyl group, a thienyl group, a pyrimidinyl group or a benzothiazolyl group, each of which may have a substituent.

### c is preferably 1.

Among the ligands represented by Formulas (AL-1) to (AL-6), a ligand represented by Formula (AL-1) is particularly preferable.

Specific examples of the ligand LA are shown below, but the present invention is not limited to these.

Herein, "Ph" represents a phenyl group.

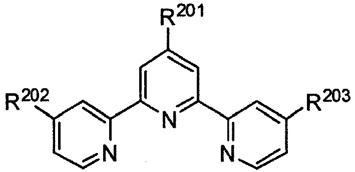

| | R²⁰¹ | R²⁰² | R²⁰³ |
|---|---|---|---|
| LA-1-1 | -COOH | -COOH | -COOH |
| LA-1-2 | -COOH | -COOH | -H |
| LA-1-3 | -H | -COOH | -H |
| LA-1-4 | -H | -PO₃H₂ | -H |
| LA-1-5 | -H | -SO₃H | -H |

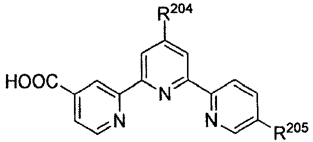

| | R²⁰⁴ | R²⁰⁵ |
|---|---|---|
| LA-2-1 | -COOH | 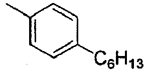 |
| LA-2-2 | -COOH | 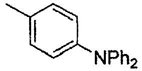 |
| LA-2-3 | -COOH | 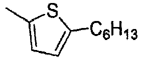 |
| LA-2-4 | -COOH | 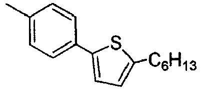 |
| LA-2-5 | -COOH | -CN |
| LA-2-6 | -COOH | -CF₃ |
| LA-2-7 | -COOH | -F |
| LA-2-8 | -COOH | 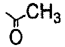 |

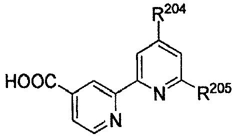

| | R²⁰⁴ | R²⁰⁵ |
|---|---|---|
| LA-2-13 | -COOH | 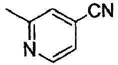 |
| LA-2-14 | -COOH | 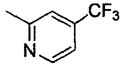 |
| LA-2-15 | -COOH | 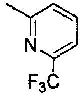 |
| LA-2-16 | -COOH | 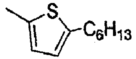 |
| LA-2-17 | -COOH | 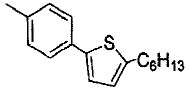 |
| LA-2-18 | -COOH | 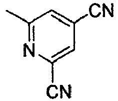 |

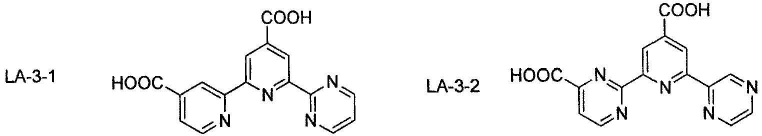

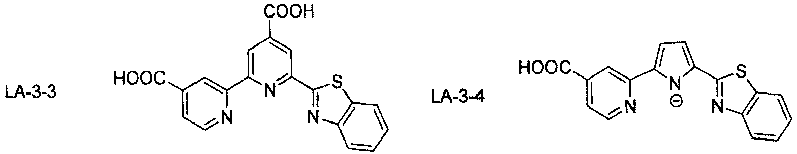

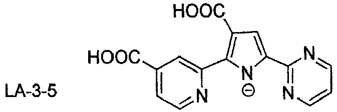

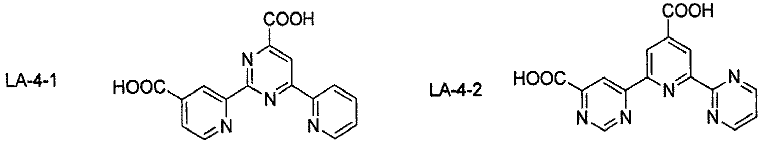

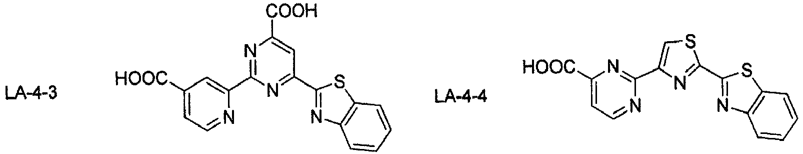

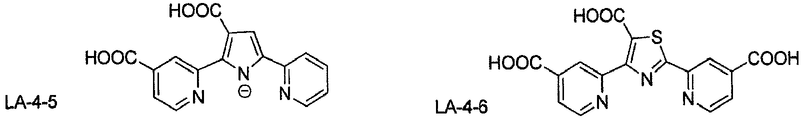

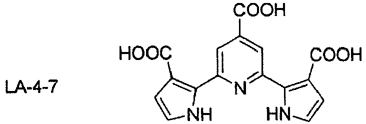

The ligand LA can be readily synthesized in the same manner as the ligand LD.

### - Ligand X -

X represents a monodentate or bidentate ligand.

The ligand X represents a monodentate or bidentate ligand coordinating at a group selected from the group consisting of an acyloxy group (preferably an acyloxy group having 1 to 20 carbon atoms, for example, acetyloxy, benzoyloxy, salicylic acid, glycyloxy, N,N-dimethylglycyloxy, oxalylene (-OC(O)C(O)O-), and the like), an acylthio group (preferably an acylthio group having 1 to 20 carbon atoms, for example, acetylthio, benzoylthio, and the like), a thioacyloxy group (preferably a thioacyloxy group having 1 to 20 carbon atoms, for example, thioacetyloxy (CH₃C(S)O-) and the like), a thioacylthio group (preferably a thioacylthio group having 1 to 20 carbon atoms, for example, thioacetylthio (CH₃C(S)S-), thiobenzoylthio (PhC(S)S-) and the like), an acylaminooxy group (preferably an acylaminooxy group having 1 to 20 carbon atoms, for example, N-methylbenzoyl aminooxy (PhC(O)N(CH₃)O-), acetylaminooxy (CH₃C(O)NHO-) and the like), a thiocarbamate group (preferably a thiocarbamate group having 1 to 20 carbon atoms, for example, N,N-diethylthiocarbamate and the like), a dithiocarbamate group (preferably a dithiocarbamate group having 1 to 20 carbon atoms, for example, N-phenydithio carbamate, N,N-dimethyldithiocarbamate, N,N-diethyldithiocarbamate, N,N-dibenzyldithiocarbamate and the like), a thiocarbonate group (preferably a thiocarbonate group having 1 to 20 carbon atoms, for example, ethylthiocarbonate and the like), a dithiocarbonate group (preferably a dithiocarbonate group having 1 to 20 carbon atoms, for example, ethyldithiocarbonate (C₂H₅OC(S)S-) and the like), a trithiocarbonate group (preferably a trithiocarbonate group having 1 to 20 carbon atoms, for example, ethyltrithiocarbonate (C₂H₅SC(S)S-) and the like), an acyl group (preferably an acyl group having 1 to 20 carbon atoms, for example, acetyl, benzoyl and the like), a thiocyanate group, an isothiocyanate group, a cyanate group, an isocyanate group, a cyano group, an alkylthio group (preferably an alkylthio group having 1 to 20 carbon atoms, for example, methylthio, ethylenedithio, and the like), an arylthio group (preferably an arylthio group having 6 to 20 carbon atoms, for example, phenylthio, 1,2-phenylenedithio and the like), an alkoxy group (preferably an alkoxy group having 1 to 20 carbon atoms, for example, methoxy and the like) and an aryloxy group (preferably an aryloxy group having 6 to 20 carbon atoms, for example, phenoxy, quinoline-8-hydroxyl and the like), or a ligand selected from the group consisting of a halogen atom (preferably a chlorine atom, a bromine atom, an iodine atom and the like), carbonyl (···CO), a dialkylketone (preferably a dialkylketone having 3 to 20 carbon atoms, for example, acetone ((CH₃)₂CO···) and the like), a 1,3-diketone (preferably a 1,3-diketone having 3 to 20 carbon atoms, for example, acetylacetone (CH₃C(O···)CH=C(O-)CH₃), a trifluoro acetylacetone (CF₃C(O···)CH=C(O-)CH₃), dipivaloylmethane (t-C₄H₉C(O···)CH=C(O-)t-C₄H₉), dibenzoylmethane (PhC(O···)CH=C(O-)Ph), 3-chloroacetylacetone (CH₃C(O···)CCl=C(O-)CH₃) and the like), a carbonamide (preferably a carbonamide group having 1 to 20 carbon atoms, for example, CH₃N=C(CH₃)O-, -OC(=NH)-C(=NH)O- and the like), a thiocarbonamide (preferably a thiocarbonamide group having 1 to 20 carbon atoms, for example, CH₃N=C(CH₃)S- and the like), and a thiourea (preferably a thiourea having 1 to 20 carbon atoms, for example, (NH(···)=C(S-)NH₂, CH₃N(···)=C(S-)NHCH₃, (CH₃)₂N-C(S···)N (CH₃)₂ and the like). Note that "···" indicates a coordinate bond.

The ligand X is preferably a ligand that coordinates at a group selected from the group consisting of an acyloxy group, a thioacylthio group, an acylaminooxy group, a dithiocarbamate group, a dithiocarbonate group, a trithiocarbonate group, a thiocyanate group, an isothiocyanate group, a cyanate group, an isocyanate group, a cyano group, an alkylthio group, an arylthio group, an alkoxy group and an aryloxy group, or a ligand selected from the group consisting of a halogen atom, carbonyl, a 1,3-diketone and a thiourea; more preferably a ligand that coordinates at a group selected from the group consisting of an acyloxy group, an acylaminooxy group, a dithiocarbamate group, a thiocyanate group, an isothiocyanate group, a cyanate group, an isocyanate group, a cyano group and an arylthio group, or a ligand selected from the group consisting of a halogen atom, a 1,3-diketone and a thiourea; especially preferably a ligand that coordinates at a group selected from the group consisting of a dithiocarbamate group, a thiocyanate group, an isothiocyanate group, a cyanate group and an isocyanate group, or a ligand selected from the group consisting of a halogen atom and a 1,3-diketone; and most preferably a ligand that coordinates at a group selected from the group consisting of a dithiocarbamate group, a thiocyanate group and an isothiocyanate group, or a ligand composed of a 1,3-diketone. Note that in the case where the ligand X contains an alkyl group, an alkenyl group, an alkynyl group, an alkylene group or the like, these groups may be linear or branched, and may be substituted or unsubstituted. In the case where the ligand X contains an aryl group, a heterocyclic group, a cycloalkyl group, or the like, these groups may be substituted or unsubstituted, and may be a single ring or a fused ring.

In the case where the ligand X is a bidentate ligand, the ligand X is preferably a ligand that coordinates at a group selected from the group consisting of an acyloxy group, an acylthio group, a thioacyloxy group, a thioacylthio group, an acylaminooxy group, a thiocarbamate group, a dithiocarbamate group, a thiocarbonate group, a dithiocarbonate group, a trithiocarbonate group, an acyl group, an alkylthio group, an arylthio group, an alkoxy group and an aryloxy group, or a ligand selected from the group consisting of a 1,3-diketone, a carbonamide group, a thiocarbonamide group and a thiourea. In the case where the ligand X is a monodentate ligand, the ligand X is preferably a ligand that coordinates at a group selected from the group consisting of a thiocyanate group, an isothiocyanate group, a cyanate group, an isocyanate group, a cyano group, an alkylthio group and an arylthio group, or a ligand selected from the group consisting of a halogen atom, carbonyl, a dialkylketone and a thiourea.

In the present invention, X is preferably NCS⁻, Cl⁻, Br⁻, I⁻, CN⁻, NCO⁻, or H₂O.

### - Metal atom M -

M is a center metal of the metal complex dye, and M represents Fe²⁺, Ru²⁺, or Os²⁺. In the present invention, M is preferably Ru²⁺. Meanwhile, when it is in a state of being incorporated into a photoelectric conversion element, the valency of M can vary according to an oxidation and reduction reaction with neighboring materials.

### -Counter ion CI for neutralizing electric charge-

CI represents a counter ion in the case where the counter ion is necessary to neutralize a charge. Generally, whether the dye is cationic or anionic, or has a net ionic charge, depends on the metal, the ligand, and the substituent, in the metal complex dye.

In the case where the substituent has a dissociative group or the like, the metal complex dye represented by Formula (I) may have a negative charge arising from dissociation. In this case, an electric charge of the metal complex dye represented by Formula (I) as a whole is electrically neutralized by CI.

When the counter ion CI is a positive counter ion, examples of the counter ion CI include an inorganic or organic ammonium ion (for example, tetraalkyl ammonium ion, pyridinium ion, and the like), a phosphonium ion (for example, a tetralkylphosphonium ion, an alkyltriphenylphosphonium ion, and the like), an alkali metal ion, and a proton (a hydrogen ion).

When the counter ion CI is a negative counter ion, the counter ion CI may be an inorganic negative ion or an organic negative ion. Examples thereof include a halogen negative ion (for example, fluoride ion, chloride ion, bromide ion, iodide ion), a substituted arylsulfonate ion (for example, p-toluene sulfonate ion, p-chlorobenzene sulfonate ion), an aryldisulfonate ion (for example, 1,3-benzene disulfonate ion, 1,5-naphthalene disulfonate ion, 2,6-naphthalene disulfonate ion), an alkylsulfate ion (for example, methylsulfate ion), a sulfate ion, a thiocyanate ion, a perchlorate ion, a tetrafluoroborate ion, a hexafluorophosphae ion, a picrate ion, an acetate ion, and a trifluoromethane sulfonate ion. Alternatively, as a charge balance counter ion, an ionic polymer or another dye with the opposite charge from the dye in interest may be used. Alternatively, a metal complex ion (for example, bisbenzene-1,2-dithiolatonickel (III) and the like) may be used.

In the present invention, CI is preferably a halogen ion, an arylsulfonate ion, an aryldisulfonate ion, an alkylsulfate ion, a sulfate ion, a thiocyanate ion, a perchlorate ion, a tetrafluoroborate ion, a hexafluorophosphate ion, an acetate ion, a trifluoromethanesulfonate ion, an ammonium ion, an alkali metal ion or a hydrogen ion, or an inorganic or organic ammonium ion; particularly preferably tetrabutylammonium ion, sodium ion, or proton

### - mX and mY -

In Formula (I), mX represents an integer of 0 to 3, preferably 0 or 1.

In Formula (I), mY represents an integer of 0 to 2, preferably 0.

Specific examples of the metal complex dye of the present invention are shown below, but the present invention is not limited to these.

In this connection, in the exemplified structures shown below, in the ligand, -CO₂H or an ammonio group, is shown in a non-dissociating form or with omitting a counter ion. The counter ion (CI in Formula (I) above) can be a tetrabutylammonium ion (⁺NBu₄), a sodium ion, PF₆⁻, or a halogen ion such as Cl⁻, which are described as representing examples.

### •Notation system for specific examples of metal complex dye

The compound represented by Formula (I) of the present invention is M(LD)(LA)(X)mX·(CI)mY, and when Ru²⁺ as M is described as Ru^{II}, the ligand LD is the above-exemplified tridentate ligand LD-1-1, the ligand LA is the above-exemplified tridentate ligand LA-1-1, and both mX and mY are 0, it is described as Ru^{II}(LD-1-1)(LA-1-1), which corresponds to the metal complex dye shown in the following Example 1.

Further, when the ligand LD is the above-exemplified tridentate ligand LD-2-7, the ligand LA is the above-exemplified tridentate ligand LA-1-1, mX is 0, mY is 2, and X is Cl⁻, it is described as Ru^{II}(LD-2-7)(LA-1-1)Cl₂, which corresponds to the metal complex dye shown in the following Example 2.

Further, when the ligand LD is the above-exemplified bidentate ligand LD-3-5, the ligand LA is the above-exemplified tridentate ligand LA-1-1, X is NCS, mX is 1, and mY is 0, it is described as Ru^{II}(LD-3-5)(LA-1-1)(NCS), which corresponds to the metal complex dye shown in the following Example 3.

Notation system for metal complex dye

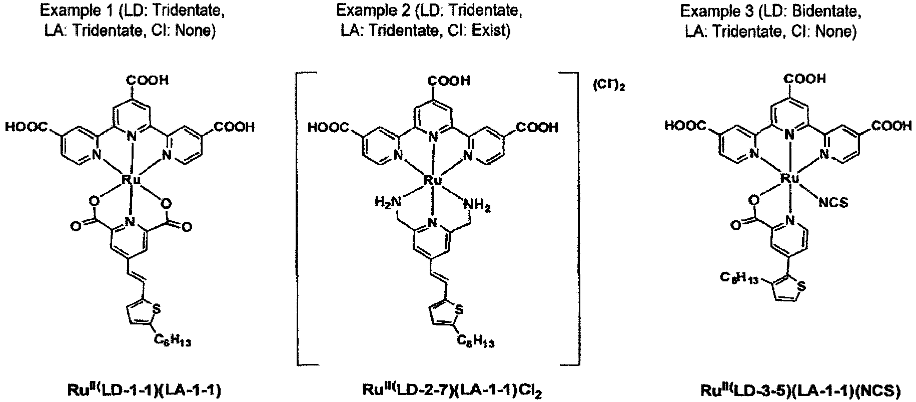

Hereinbelow, specific examples of the metal complex dye of the present invention are shown according to the notation system described above.

| | |
|---|---|
| Metal complex dye 1 | Ru^{II}(LD-1-1)(LA-1-1) |
| Metal complex dye 2 | Ru^{II}(LD-1-1)(LA-1-3) |
| Metal complex dye 3 | Ru^{II}(LD-1-2)(LA-1-1) |
| Metal complex dye 4 | Ru^{II}(LD-1-3)(LA-1-1) |
| Metal complex dye 5 | Ru^{II}(LD-1-4)(LA-1-1) |
| Metal complex dye 6 | Ru^{II}(LD-1-6)(LA-1-1) |
| Metal complex dye 7 | Ru^{II}(LD-1-9)(LA-1-1) |
| Metal complex dye 8 | Ru^{II}(LD-1-12)(LA-1-1) |
| Metal complex dye 9 | Ru^{II}(LD-1-13)(LA-1-1) |
| Metal complex dye 10 | Ru^{II}(LD-1-15)(LA-1-1) |
| Metal complex dye 11 | Ru^{II}(LD-1-16)(LA-1-1) |
| Metal complex dye 12 | Ru^{II}(LD-1-17)(LA-1-1) |
| Metal complex dye 13 | Ru^{II}(LD-1-21)(LA-1-1) |
| Metal complex dye 14 | Ru^{II}(LD-1-24)(LA-1-1) |
| Metal complex dye 15 | Ru^{II}(LD-1-26)(LA-1-1) |
| Metal complex dye 16 | Ru^{II}(LD-1-30)(LA-1-1)N⁺(C₄H₉)₄ |
| Metal complex dye 17 | Ru^{II}(LD-1-1)(LA-2-1) |
| Metal complex dye 18 | Ru^{II}(LD-1-1)(LA-2-2) |
| Metal complex dye 19 | Ru^{II}(LD-1-1)(LA-2-3) |
| Metal complex dye 20 | Ru^{II}(LD-1-1)(LA-2-9) |
| Metal complex dye 21 | Ru^{II}(LD-1-1)(LA-2-12) |
| Metal complex dye 22 | Ru^{II}(LD-1-6)(LA-2-4) |
| Metal complex dye 23 | Ru^{II}(LD-1-15)(LA-2-3) |
| Metal complex dye 24 | Ru^{II}(LD-1-17)(LA-2-4) |
| Metal complex dye 25 | Ru^{II}(LD-1-21)(LA-2-4) |
| Metal complex dye 26 | Ru^{II}(LD-1-24)(LA-2-3) |
| Metal complex dye 27 | Ru^{II}(LD-1-26)(LA-2-3) |
| Metal complex dye 28 | Ru^{II}(LD-1-26)(LA-2-4) |
| Metal complex dye 29 | Ru^{II}(LD-1-1)(LA-3-1) |
| Metal complex dye 30 | Ru^{II}(LD-1-15)(LA-3-2) |
| Metal complex dye 31 | Ru^{II}(LD-1-24)(LA-3-4)N⁺(C₄H₉)₄ |
| Metal complex dye 32 | Ru^{II}(LD-1-26)(LA-3-4)N⁺(C₄H₉)₄ |
| Metal complex dye 33 | Ru^{II}(LD-2-1)(LA-1-1) |
| Metal complex dye 34 | Ru^{II}(LD-2-1)(LA-1-2) |
| Metal complex dye 35 | Ru^{II}(LD-2-2)(LA-1-1) |
| Metal complex dye 36 | Ru^{II}(LD-2-2)(LA-1-2) |
| Metal complex dye 37 | Ru^{II}(LD-2-3)(LA-1-1) |
| Metal complex dye 38 | Ru^{II}(LD-2-3)(LA-1-2) |
| Metal complex dye 39 | Ru^{II}(LD-2-4)(LA-1-1) |
| Metal complex dye 40 | Ru^{II}(LD-2-6)(LA-1-1) |
| Metal complex dye 41 | Ru^{II}(LD-2-6)(LA-1-2) |
| Metal complex dye 42 | Ru^{II}(LD-2-7)(LA-1-1)Cl₂ |
| Metal complex dye 43 | Ru^{II}(LD-2-7)(LA-1-2)Cl₂ |
| Metal complex dye 44 | Ru^{II}(LD-2-9)(LA-1-1)(NCS)₂ |
| Metal complex dye 45 | Ru^{II}(LD-2-12)(LA-1-1) |
| Metal complex dye 46 | Ru^{II}(LD-2-12)(LA-1-2) |
| Metal complex dye 47 | Ru^{II}(LD-2-12)(LA-1-1) |
| Metal complex dye 48 | Ru^{II}(LD-2-13)(LA-1-1) |
| Metal complex dye 49 | Ru^{II}(LD-2-13)(LA-1-2) |
| Metal complex dye 50 | Ru^{II}(LD-2-13)(LA-1-3) |
| Metal complex dye 51 | Ru^{II}(LD-2-1)(LA-2-4) |
| Metal complex dye 52 | Ru^{II}(LD-2-2)(LA-2-1) |
| Metal complex dye 53 | Ru^{II}(LD-2-3)(LA-2-3) |
| Metal complex dye 54 | Ru^{II}(LD-2-6)(LA-2-4) |
| Metal complex dye 55 | Ru^{II}(LD-3-1)(LA-1-1)NCS |
| Metal complex dye 56 | Ru^{II}(LD-3-1)(LA-1-2)NCS |
| Metal complex dye 57 | Ru^{II}(LD-3-2)(LA-1-1)NCS |
| Metal complex dye 58 | Ru^{II}(LD-3-2)(LA-1-3)NCS |
| Metal complex dye 59 | Ru^{II}(LD-3-3)(LA-1-1)NCS |
| Metal complex dye 60 | Ru^{II}(LD-3-5)(LA-1-1)NCS |
| Metal complex dye 61 | Ru^{II}(LD-3-6)(LA-1-1)NCS |
| Metal complex dye 62 | Ru^{II}(LD-3-7)(LA-1-1)(NCS)₂ |
| Metal complex dye 63 | Ru^{II}(LD-3-12)(LA-1-1)NCS |
| Metal complex dye 64 | Ru^{II}(LD-3-13)(LA-1-1)NCS |
| Metal complex dye 65 | Ru^{II}(LD-3-3)(LA-2-1)NCS |
| Metal complex dye 66 | Ru^{II}(LD-3-5)(LA-2-2)NCS |
| Metal complex dye 67 | Ru^{II}(LD-3-6)(LA-2-2)NCS |
| Metal complex dye 68 | Ru^{II}(LD-3-7)(LA-2-3)(NCS)₂ |
| Metal complex dye 69 | Ru^{II}(LD-3-12)(LA-2-4)NCS |
| Metal complex dye 70 | Ru^{II}(LD-3-13)(LA-2-4)NCS |
| Metal complex dye 71 | Ru^{II}(LD-4-1)(LA-1-1)(NCS)N⁺(C₄H₉)₄ |
| Metal complex dye 72 | Ru^{II}(LD-4-1)(LA-1-2)(NCS)N⁺(C₄H₉)₄ |
| Metal complex dye 73 | Ru^{II}(LD-4-2)(LA-1-1)(NCS)N⁺(C₄H₉)₄ |
| Metal complex dye 74 | Ru^{II}(LD-4-2)(LA-1-3)(NCS)N⁺(C₄H₉)₄ |
| Metal complex dye 75 | Ru^{II}(LD-4-3)(LA-1-1)(NCS)N⁺(C₄H₉)₄ |
| Metal complex dye 76 | Ru^{II}(LD-4-3)(LA-1-5)(NCS)N⁺(C₄H₉)₄ |
| Metal complex dye 77 | Ru^{II}(LD-4-4)(LA-1-1)(NCS)N⁺(C₄H₉)₄ |
| Metal complex dye 78 | Ru^{II}(LD-4-4)(LA-1-2)(NCS)N⁺(C₄H₉)₄ |
| Metal complex dye 79 | Ru^{II}(LD-4-6)(LA-1-1)(NCS)N⁺(C₄H₉)₄ |
| Metal complex dye 80 | Ru^{II}(LD-4-7)(LA-1-1)(NCS)₂ |
| Metal complex dye 81 | Ru^{II}(LD-4-12)(LA-1-1)(NCS)N⁺(C₄H₉)₄ |
| Metal complex dye 82 | Ru^{II}(LD-4-13)(LA-1-1)(NCS)N⁺(C₄H₉)₄ |
| Metal complex dye 83 | Ru^{II}(LD-4-6)(LA-2-3)(NCS)N⁺(C₄H₉)₄ |
| Metal complex dye 84 | Ru^{II}(LD-4-7)(LA-2-1)(NCS)₂ |
| Metal complex dye 85 | Ru^{II}(LD-4-12)(LA-2-3)(NCS)N⁺(C₄H₉)₄ |
| Metal complex dye 86 | Ru^{II}(LD-4-13)(LA-2-3)(NCS)N⁺(C₄H₉)₄ |
| Metal complex dye 87 | Ru^{II}(LD-5-1)(LA-3-1) |
| Metal complex dye 88 | Ru^{II}(LD-5-3)(LA-3-4) |
| Metal complex dye 89 | Ru^{II}(LD-1-24)(LA-3-1) |
| Metal complex dye 90 | Ru^{II}(LD-1-26)(LA-3-3)N⁺(C₄H₉)₄ |

The metal complex dye according to the present invention can be easily synthesized by methods described in US 2005/0081911 A1, JP-A-2010-13500, JP-A-2011-195745, JP-A-2001-291534, and Chem. Commun., 2009, 5844 to 5846, reference literatures listed in these literatures, or in accordance with methods described in these patents or literatures.

The metal complex dye of the present invention has the maximum absorption wavelength in a solution, preferably in a range from 300 to 1,000 nm, more preferably in a range from 350 to 950 nm, and particularly preferably in a range from 370 to 900 nm.

### - Electrically-conductive support -

The electrically-conductive support is preferably a support having electroconductivity per se, such as a metal, or a glass or plastic support having an electrically-conductive film layer on the surface. As the plastic support, a transparent polymer film described in paragraph No. 0153 of JP-A-2001-291534 can be mentioned. In addition to the glass and plastic, ceramic (JP-A-2005-135902), an electrically-conductive resin (JP-A-2001-160425) or the like may be used as the support. The electrically-conductive support may be provided with a light management function at the surface, and for example, the anti-reflective film having a high refractive index film and a low refractive index oxide film alternately laminated as described in JP-A-2003-123859, and the light guide function as described in JP-A-2002-260746 may be mentioned.

The thickness of the electrically-conductive film layer is preferably 0.01 to 30 µm, more preferably 0.03 to 25 µm, and particularly preferably 0.05 to 20 µm.

It is preferable that the electrically-conductive support is substantially transparent. The term "substantially transparent" means that the transmittance of light is 10% or more, preferably 50% or more, and particularly preferably 80% or more. As the transparent electrically-conductive support, a support formed from glass or plastic and coated with an electrically-conductive metal oxide is preferable. As a metal oxide, tin oxide is preferable, and indium-tin oxide and fluorine-doped oxide are particularly preferable. In this case, the coating amount of the electrically-conductive metal oxide is preferably 0.1 to 100 g, per square meter of the support made of glass or plastic. In the case of using a transparent electrically-conductive support, it is preferable that light is incident from the support side.

### - Semiconductor fine-particles -

Regarding the semiconductor fine-particles, fine-particles of chalcogenides of metals (for example, oxides, sulfides and selenides), or fine-particles of perovskites may be used with preference. Preferred examples of the chalcogenides of metals include oxides of titanium, tin, zinc, tungsten, zirconium, hafnium, strontium, indium, cerium, yttrium, lanthanum, vanadium, niobium or tantalum, cadmium sulfide, and cadmium selenide. Preferred examples of the perovskites include strontium titanate, and calcium titanate. Among these, titanium oxide (titania), zinc oxide, tin oxide, and tungsten oxide are particularly preferred.

Examples of the crystal structure of titania include structures of anatase type, brookite type and rutile type, and anatase type and brookite type structures are preferred. A titania nanotube, nanowire, or nanorod may be mixed with titania fine-particles or may be used as a semiconductor electrode.

A particle size of the semiconductor fine-particles is expressed in terms of an average particle size using a diameter when a projected area is converted into a circle, and is preferably 0.001 to 1 µm as primary particles, and 0.01 to 100 µm as an average particle size of dispersions. Examples of the method for coating the semiconductor fine-particles on the electrically-conductive support include a wet method, a dry method or other methods.

It is preferable to form a short circuit-preventing layer between the transparent electrically-conductive film and the semiconductor layer ("photoconductor layer"), so as to prevent reverse current due to a direct contact between the electrolyte and the electrode. It is preferable to employ a spacer or a separator, so as to prevent contact between the photoelectrode and the counter electrode. It is preferable for the semiconductor fine-particles to have a large surface area, so that a large amount of dye can adsorb to the surface. For example, in a state of the semiconductor fine-particles being coated on the support, the surface area is preferably 10 times or more, and more preferably 100 times or more, relative to the projected surface area. The upper limit of this value is not particularly limited, and the upper limit is generally about 5,000 times. In general, as the thickness of the layer containing semiconductor fine particles increases, the amount of dye that can be supported (carried) per unit area increases, and therefore, the light absorption efficiency increases. However, since the diffusion distance of generated electrons increases along, the loss due to charge recombination also increases. Although a preferred thickness of the photoconductor layer being the semiconductor layer may vary depending on the utility of the element, the thickness is typically 0.1 to 100 µm. In the case of using the photoelectric conversion element for a dye-sensitized solar cell, the thickness of the photoconductor layer is preferably 1 to 50 µm, and more preferably 3 to 30 µm. The semiconductor fine-particles may be calcined after being applied on the support, at a temperature of 100 to 800°C for 10 minutes to 10 hours, so as to bring about cohesion of the particles. When a glass support is used, the film-forming temperature is preferably 60 to 400°C.

The coating amount of the semiconductor fine-particles per square meter of the support is preferably 0.5 to 500 g, and more preferably 5 to 100 g. The overall amount of use of the dye is preferably 0.01 to 100 millimoles, more preferably 0.1 to 50 millimoles, and particularly preferably 0.1 to 10 millimoles, per square meter of the support. In this case, the amount of use of the metal complex dye of the present invention is preferably set to 5% by mole or more. The amount of the dye adsorbed to the semiconductor fine-particles is preferably 0.001 to 1 millimole, and more preferably 0.1 to 0.5 millimoles, based on 1 g of the semiconductor fine-particles. When the amount of the dye is set to such a range, the sensitization effect on the semiconductor fine-particles can be sufficiently obtained.

When the dye is a salt, a counter ion of the specific metal complex dye is not particularly limited. Examples thereof include an alkali metal ion and a quaternary ammonium ion.

After the dye has been adsorbed, the surface of the semiconductor fine-particles may be treated using amines. Preferred examples of the amines include pyridines (e.g., 4-tert-butylpyridine, and polyvinylpyridine). These may be used directly when the compounds are liquids, or may be used in a state of being dissolved in an organic solvent.

In the photoelectric conversion element (for example, photoelectric conversion element 10) and the dye-sensitized solar cell (for example, dye-sensitized solar cell 20) of the present invention, at least the metal complex dye of the present invention is used.

In the present invention, the metal complex dye of the present invention and another dye may be used in combination.

The dye to be used in combination includes: a Ru complex dye described in JP-T-7-500630 (the term "JP-T" means a published Japanese translation of a PCT patent application) (in particular, dyes synthesized in Examples 1 to 19 described in from line 5 on left lower column on page 5 to line 7 on right upper column on page 7), a Ru complex dye described in JP-T-2002-512729 (in particular, dyes synthesized in Examples 1 to 16 described in line 3 from the bottom of page 20 to line 23 on page 29), a Ru complex dye described in JP-A-2001-59062 (in particular, dyes described in paragraph Nos. 0087 to 0104), a Ru complex dye described in JP-A-2001-6760 (in particular, dyes described in paragraph Nos. 0093 to 0102), a Ru complex dye described in JP-A-2001-253894 (in particular, dyes described in paragraph Nos. 0009 to 0010), a Ru complex dye described in JP-A-2003-212851 (in particular, dyes described in paragraph No. 0005), a Ru complex dye described in WO 2007/91525 pamphlet (in particular, dyes described in paragraph No. [0067]), a Ru complex dye described in JP-A-2001-291534 (in particular, dyes described in paragraph Nos. 0120 to 0144), a Ru complex dye described in JP-A-2012-012570 (in particular, dyes described in paragraph Nos. 0095 to 0103), a Ru complex dye described in JP-A-2013-084594 (in particular, dyes described in paragraph Nos. 0072 to 0081 and the like), a squarylium cyanine dye described in JP-A-11-214730 (in particular, dyes described in paragraph Nos. 0036 to 0047), a squarylium cyanine dye described in JP-A-2012-144688 (in particular, dyes described in paragraph Nos. 0039 to 0046 and 0054 to 0060), a squarylium cyanine dye described in JP-A-2012-84503 (in particular, dyes described in paragraph Nos. 0066 to 0076 and the like), an organic dye described in JP-A-2004-063274 (in particular, dyes described in paragraph Nos. 0017 to 0021), an organic dye described in JP-A-2005-123033 (in particular, dyes described in paragraph Nos. 0021 to 0028), an organic dye described in JP-A-2007-287694 (in particular, dyes described in paragraph Nos. 0091 to 0096), an organic dye described in JP-A-2008-71648 (in particular, dyes described in paragraph Nos. 0030 to 0034), an organic dye described in WO 2007/119525 pamphlet (in particular, dyes described in paragraph No. [0024]), a porphyrine dye described in Angew. Chem. Int. Ed., 49, 1 to 5 (2010), and a phthalocyanine dye described in Angew. Chem. Int. Ed., 46, 8358 (2007), or the like.

Preferable dyes to be used in combination include Ru complex dyes, squaryrium cyanine dyes, or organic dyes.

In a case where the metal complex dye of the present invention and another dye are used in combination, a ratio of mass of the metal complex dye of the present invention/mass of another dye is preferably from 95/5 to 10/90, more preferably from 95/5 to 50/50, still more preferably from 95/5 to 60/40, particularly preferably from 95/5 to 65/35, and most preferably from 95/5 to 70/30.

### - Charge transfer layer -

The charge transfer layer for use in the photoelectric conversion element of the present invention is a layer having a function to replenish electrons to the oxidized form of the dye, and it is provided between the light-receiving electrode and the counter electrode (an opposite electrode). The charge-transfer layer contains an electrolyte. Examples of the electrolyte include a liquid electrolyte having a redox pair dissolved in an organic solvent, a so-called gel electrolyte in which a liquid having a redox pair dissolved in an organic solvent is impregnated in a polymer matrix, and a molten salt containing a redox pair. In order to enhance photoelectric conversion efficiency, a liquid electrolyte is preferred. As an organic solvent of the liquid electrolyte, a nitrile compound, an ether compound, an ester compound, or the like, is used, and a nitrile compound is preferred, and acetonitrile and methoxypropionitrile are particularly preferred.

Examples of the redox pair include a combination of iodine and an iodide (preferably an iodide salt, or an iodide ionic liquid; more preferably lithium iodide, tetrabutylammonium iodide, tetrapropylammonium iodide, or methylpropylimidazolium iodide), a combination of an alkylviologen (for example, methylviologen chloride, hexylviologen bromide, or benzylviologen tetrafluoroborate) and a reductant thereof, a combination of a polyhydroxybenzene (for example, hydroquinone, naphthohydroquinone, or the like) and an oxidized form thereof, a combination of a divalent iron complex and a trivalent iron complex (for example, a combination of potassium ferricyanide and potassium ferrocyanide), and a combination of a divalent cobalt complex and a trivalent cobalt complex. Among these, a combination of iodine and an iodide, and a combination of a divalent cobalt complex and a trivalent cobalt complex, are preferred.

The cobalt complex is preferably a complex represented by Formula (CC).

Co(LL)ma(X)mb·CI Formula (CC)

In Formula (CC), LL represents a bidentate or tridentate ligand. X represents a monodentate ligand. ma represents an integer of 0 to 3. mb represents an integer of 0 to 6. CI represents a counter ion in the case where the counter ion is necessary to neutralize a charge in Formula (CC).

Examples of CI include those of CI in Formula (I).

LL is preferably a ligand represented by Formula (LC).

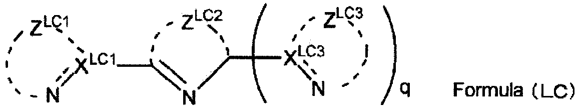

In Formula (LC), X^{LC1} and X^{LC3} each independently represent a carbon atom or a nitrogen atom. Herein, when X^{LC1} is a carbon atom, the bond between X^{LC1} and the N atom is a double bond (X^{LC1}=N). When X^{LC3} is a carbon atom, the bond between X^{LC3} and the N atom is a double bond (X^{LC3}=N). When X^{LC1} is a nitrogen atom, the bond between X^{LC1} and the N atom is a single bond (X^{LC1}-N). When X^{LC3} is a nitrogen atom, the bond between X^{LC3} and the N atom is a single bond (X^{LC3}-N).

Z^{LC1}, Z^{LC2} and Z^{LC3} each independently represent a group of nonmetallic atoms necessary to form a 5- or 6-membered ring. Each of Z^{LC1}, Z^{LC2} and Z^{LC3} may have a substituent, and may form a ring-closure together with an adjacent ring through a substituent. q represents 0 or 1. Examples of the substituent include the substituent T described later. Meanwhile, when q is 0, the carbon atom on a position at which X^{LC3} binds to a 5-membered ring or 6-membered ring formed by Z^{LC2} binds with a hydrogen atom or a substituent other than heterocyclic group formed by Z^{LC3}.

X is preferably a halogen ion.

The ligand represented by Formula (LC) is more preferably a ligand represented by any one of Formulas (LC-1) to (LC-4).

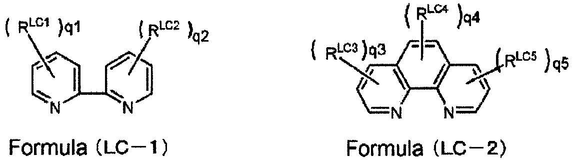

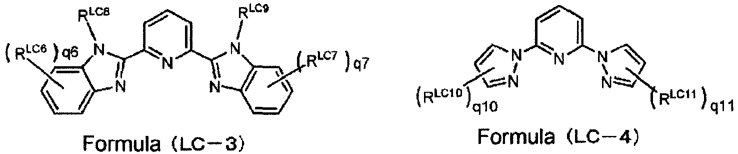

R^{LC1} to R^{LC11} each independently represent a substituent. q1, q2, q6, and q7 each independently represent an integer of 0 to 4. q3, q5, q10, and q11 each independently represent an integer of 0 to 3. q4 represents an integer of 0 to 2.

In Formulas (LC-1) to (LC-4), examples of the substituent R^{LC1} to R^{LC11} include an aliphatic group, an aromatic group, a heterocyclic group or the like. Specific examples of the substituent include an alkyl group, an alkoxy group, an alkylthio group, an aryl group, an aryloxy group, an arylthio group, and a heterocycle. Preferred examples include an alkyl group (for example, methyl, ethyl, n-butyl, n-hexyl, isobutyl, sec-butyl, t-butyl, n-dodecyl, cyclohexyl, or benzyl), an aryl group (for example, phenyl, tolyl, or naphthyl), an alkoxy group (for example, methoxy, ethoxy, isopropoxy, or butoxy), an alkylthio group (for example, methylthio, n-butylthio, n-hexylthio, or 2-ethylhexylthio), an aryloxy group (for example, phenoxy, or naphthoxy), an arylthio group (for example, phenylthio, or naphthylthio), and a heterocyclic group (for example, 2-thienyl, or 2-furyl).

Specific examples of the cobalt complex represented by Formula (LC) include the followings.

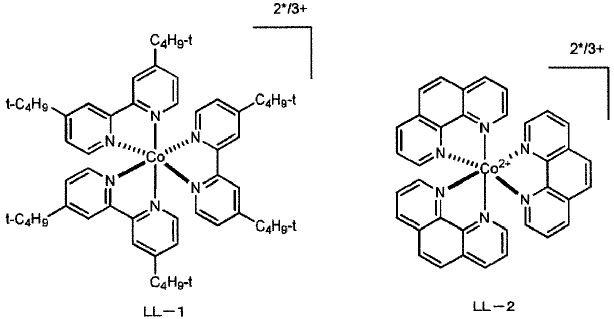

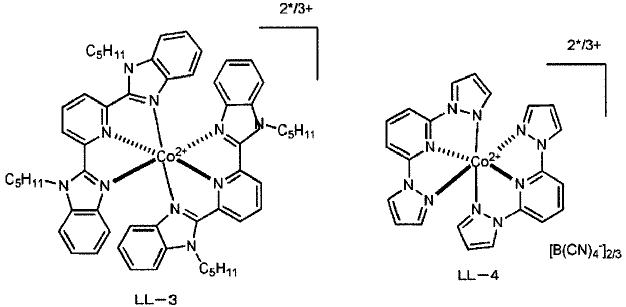

In the case where iodine and an iodide are used in combination, as an electrolyte, it is preferred that a 5- or 6-membered-ring nitrogen-containing aromatic cation iodide salt is additionally used in combination with them.

Preferred examples of the organic solvent that dissolves these redox pairs include aprotic polar solvents (for example, acetonitrile, propylene carbonate, ethylene carbonate, dimethylformamide, dimethylsulfoxide, sulfolane, 1,3-dimethylimidazolinone, and 3-methyloxazolidinone). Examples of the polymer used for a matrix of a gel electrolyte include polyacrylonitrile, polyvinylidene fluoride, and the like. Examples of the molten salts include, for example, a molten salt to which fluidity at room temperature has been imparted by mixing lithium iodide and at least one kind of other lithium salt (for example, lithium acetate or lithium perchlorate) with polyethylene oxide. The amount of addition of the polymer in this case is 1 to 50% by mass. Furthermore, the electrolyte liquid may contain γ-butyrolactone, and this γ-butyrolactone increases the diffusion efficiency of iodide ions, and thereby the conversion efficiency is enhanced.

Examples of the additives that are added to the electrolyte include 4-tert-butylpyridine mentioned above, as well as aminopyridine-based compounds, benzimidazole-based compounds, aminotriazole-based compounds, aminothiazole-based compounds, imidazole-based compounds, aminotriazine-based compounds, urea derivatives, amide compounds, pyrimidine-based compounds, and heterocycles that do not contain nitrogen.

It is also preferable to employ a method of controlling the water content of the electrolyte liquid, in order to enhance the photoelectric conversion efficiency. Preferred examples of the method of controlling the water content include a method of controlling the concentration, and a method of adding a dehydrating agent. In order to reduce the toxicity of iodine, a clathrate compound of iodine with cyclodextrin may be used. Alternatively, a method of supplying moisture on a steady basis may be used. Furthermore, a cyclic amidine may be used; or an oxidation inhibitor, a hydrolysis inhibitor, a decomposition inhibitor or zinc iodide may be added.

A molten salt may also be used as the electrolyte, and preferred examples of the molten salt include an ionic liquid containing an imidazolium or triazolium type cation, an oxazolium-based salt, a pyridinium-based salt, a guanidium-based salt, and combinations of these. These cations may be used in combination with particular anions. Additives may be added to these molten salts. The molten salt may have a substituent having liquid crystalline properties. Furthermore, the quaternary ammonium salt-based molten salt may also be used.

Molten salts other than those described above include, for example, a molten salt to which fluidity at room temperature has been imparted by mixing lithium iodide and at least one kind of other lithium salt (for example, lithium acetate or lithium perchlorate) with polyethylene oxide.

The electrolyte may be quasi-solidified by adding a gelling agent to an electrolyte liquid formed from an electrolyte and a solvent, and gelling the electrolyte liquid thereby. Examples of the gelling agent include an organic compound having a molecular weight of 1000 or less, an Si-containing compound having a molecular weight in the range of 500 to 5000, an organic salt obtained from a particular acidic compound and a particular basic compound, a sorbitol derivative, and polyvinylpyridine.

Furthermore, a method of confining a matrix polymer, a crosslinking type polymer compound or monomer, a crosslinking agent, an electrolyte and a solvent, in a polymer may be used.

Preferred examples of the matrix polymer include a polymer having a nitrogen-containing heterocyclic ring in a repeating unit in the main chain or in a side chain, and a crosslinked structure formed by reacting the polymer with an electrophilic compound; a polymer having a triazine structure, a polymer having a ureide structure, a polymer containing a liquid crystalline compound, a polymer having an ether bond, a polyvinylidene fluoride-based polymer, a methacrylate/acrylate-based polymer, a thermosetting resin, crosslinked polysiloxane, polyvinyl alcohol (PVA), a clathrate compound of polyalkylene glycol and dextrin, a system incorporated with an oxygen-containing or sulfur-containing polymer, and a naturally occurring polymer. An alkali-swellable polymer, a polymer having a cation moiety and a component capable of forming a charge transfer complex with iodine within one polymer molecule, or the like may be added to those matrix polymers.

A system containing, as a matrix polymer, a crosslinked polymer formed by reacting a bifunctional or higher-functional isocyanate as one component with a functional group such as a hydroxyl group, an amino group or a carboxyl group, may also be used. Furthermore, a crosslinked polymer based on a hydrosilyl group and a double-bonded compound, a crosslinking method involving reacting polysulfonic acid, polycarboxylic acid or the like with a divalent or higher-valent metal ion compound, and the like may also be used.

Examples of the solvent that can be used with preference in combination with the quasi-solid electrolyte described above, include particular phosphates, a mixed solvent containing ethylene carbonate, a solvent having a particular relative permittivity, and the like. A liquid electrolyte solution may be retained in a solid electrolyte membrane or in pores, and preferred examples of the method include the usage of an electrically conductive polymer membrane, a fibrous solid, and a fabric-like solid such as filter.

A solid-state charge-transport layer, such as a p-type semiconductor or a hole-transporting material, for example, CuI or CuNCS, may also be used in place of a liquid electrolyte and a quasi-solid-state electrolyte as described above. Moreover, electrolytes described in Nature, vol. 486, p. 487 (2012) and the like may be used. For a solid charge-transport layer, an organic hole-transporting material may be used. Preferred examples of the hole-transport layer include electrically conductive polymers such as polythiophene, polyaniline, polypyrrole, and polysilane; a spiro compound in which two rings share a central element adopting a tetrahedral structure, such as C and Si; aromatic amine derivatives such as triarylamine; triphenylene derivatives; nitrogen-containing heterocycle derivatives; and liquid crystalline cyano derivatives.

The redox pair serves as an electron carrier, and the concentration of the redox pair is preferably 0.01 mol/l or more, more preferably 0.1 mol/l or more, and particularly preferably 0.3 mol/l or more. The upper limit of the concentration of the redox pair in total is not particularly limited, and generally about 5 mol/1.

### - Co-adsorbent -

In the photoelectric conversion element of the present invention, a co-adsorbent is preferably used in combination with the metal complex dye of the present invention or another dye to be used if necessary. As such a co-adsorbent, a co-adsorbent having at least one acidic group (preferably a carboxyl group or a salt thereof) is preferable, and examples of the co-adsorbent include a fatty acid and a compound having a steroid skeleton. The fatty acid may be a saturated fatty acid or an unsaturated fatty acid. Examples thereof include a butanoic acid, a hexanoic acid, an octanoic acid, a decanoic acid, a hexadecanoic acid, a dodecanoic acid, a palmitic acid, a stearic acid, an oleic acid, a linoleic acid, and a linolenic acid.

Examples of the compound having a steroid skeleton include a cholic acid, a glycocholic acid, a chenodeoxycholic acid, a hyocholic acid, a deoxycholic acid, a lithocholic acid, and ursodeoxycholic acid. Among these, a cholic acid, a deoxycholic acid, and a chenodeoxycholic acid are preferable; and a chenodeoxycholic acid is more preferable.

A preferred co-adsorbent is a compound represented by Formula (CA).

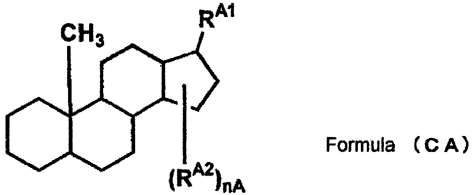

In Formula (CA), R^{A1} represents a substituent having an acidic group. R^{A2} represents a substituent. nA represents an integer of 0 or more.

The acidic group has the same meaning as described above, and the preferable range is also the same.

Of these, R^{A1} is preferably an alkyl group substituted with any one of a carboxyl group, a sulfo group, and a salt thereof; and further preferably -CH(CH₃)CH₂CH₂CO₂H, or -CH(CH₃)CH₂CH₂CONHCH₂CH₂SO₃H.

Examples of R^{A2} include those exemplified as the substituent T described later. Of these, an alkyl group, a hydroxyl group, an acyloxy group, an alkylaminocarbonyloxy group, and an arylaminocarbonyloxy group are preferable; and an alkyl group, a hydroxyl group, and an acyloxy group are more preferable.

nA is preferably from 2 to 4.

Specific compounds of these include compounds that are exemplified as the compound having a steroid skeleton.

By adsorbing on the semiconductor fine-particles, the co-adsorbent that can be used in the present invention exhibits an effect of suppressing the inefficient association of the dye and an effect of preventing reverse electron transfer from the semiconductor fine-particle surface to the redox system in the electrolyte. The amount to be used of the co-adsorbent is not particularly limited, and it is preferred, from the viewpoint of exhibiting the effects effectively, that the amount is preferably from 1 to 200 moles, more preferably from 10 to 150 moles, and particularly preferably from 20 to 50 moles, with respect to 1 mole of the above described dye.

### <Substituent T>

This specification uses an expression "compound" (including complex and dye) to mean, in addition to the compound itself, its salts, its complex and its ion. Further, it is used in the sense that the compound includes a derivative thereof which is modified in a predetermined part, in the range of achieving a desired effect. Further, a substituent with which whether being substituted or unsubstituted is not explicitly described in the present specification (the same applies to a linking group and a ligand), means that the substituent may have an arbitrary substituent. This also applies to a compound with which whether being substituted or unsubstituted is not explicitly described. Preferable examples of the substituent include the following substituent T.

In the present specification, the simple description only as a "substituent" means to refer to this substituent T. Further, in a case where each of the substituents, for example, like an alkyl group, is described in a simplistic form, both a preferable range and specific examples for the corresponding group for the substituent T are applied to.

The substituent T includes the followings:
an alkyl group (preferably an alkyl group having 1 to 20 carbon atoms, e.g. methyl, ethyl, isopropyl, t-butyl, pentyl, heptyl, 1-ethylpentyl, benzyl, 2-ethoxyethyl, 1-carboxymethyl, or trifluoromethyl), an alkenyl group (preferably an alkenyl group having 2 to 20 carbon atoms, e.g. vinyl, allyl, or oleyl), an alkynyl group (preferably an alkynyl group having 2 to 20 carbon atoms, e.g. ethynyl, butadiynyl, or phenylethynyl), a cycloalkyl group (preferably a cycloalkyl group having 3 to 20 carbon atoms, e.g. cyclopropyl, cyclopentyl, cyclohexyl, or 4-methylcyclohexyl), an cycloalkenyl group (preferably a cycloalkenyl group having 5 to 20 carbon atoms, e.g. cyclopentenyl, or cyclohexenyl), an aryl group (preferably an aryl group having 6 to 26 carbon atoms, e.g. phenyl, 1-naphthyl, 4-methoxyphenyl, 2-chlorophenyl, or 3-methylphenyl), a heterocyclic group (preferably a 5- or 6-membered heterocyclic group having 2 to 20 carbon atoms and at least one oxygen atom, sulfur atom, or nitrogen atom as the ring-forming atom, e.g. 2-pyridyl, 4-pyridyl, 2-imidazolyl, 2-benzimidazolyl, 2-thiazolyl, or 2-oxazolyl), an alkoxy group (preferably an alkoxy group having 1 to 20 carbon atoms, e.g. methoxy, ethoxy, isopropyloxy, or benzyloxy), an alkenyloxy group (preferably an alkenyloxy group having 2 to 20 carbon atoms, e.g. vinyloxy or allyloxy), an alkynyloxy group (preferably an alkynyloxy group having 2 to 20 carbon atoms, e.g. 2-propynyloxy or 4-butynyloxy), a cycloalkyloxy group (preferably a cycloalkyloxy group having 3 to 20 carbon atoms, e.g. cyclopropyloxy, cyclopentyloxy, cyclohexyloxy, or 4-methylcyclohexyloxy), an aryloxy group (preferably an aryloxy group having 6 to 26 carbon atoms, e.g. phenoxy,1-naphthyloxy, 3-methylphenoxy, or 4-methoxyphenoxy), a heterocyclic oxy group (e.g. imidazolyloxy, benzoimidazolyloxy, thiazolyloxy, benzothiazolyloxy, triazinyloxy, or purinyloxy);
an alkoxycarbonyl group (preferably an alkoxycarbonyl group having 2 to 20 carbon atoms, e.g. ethoxycarbonyl, or 2-ethylhexyloxycarbonyl), a cycloalkoxycarbonyl group (preferably a cycloalkoxycarbonyl group having 4 to 20 carbon atoms, e.g. cyclopropyloxycarbonyl, cyclopentyloxycarbonyl, or cyclohexyloxycarbonyl), an aryloxycarbonyl group (preferably an aryloxycarbonyl group having 6 to 20 carbon atoms, e.g. phenyloxycarbonyl, or naphthyloxycarbonyl), an amino group (preferably an amino group having 0 to 20 carbon atoms including an alkylamino group, an alkenylamino group, an alkynylamino group, a cycloalkylamino group, a cycloalkenylamino group, an arylamino group, and a heterocyclic amino group, e.g. amino, N,N-dimethylamino, N,N-diethylamino, N-ethylamino, N-allylamino, N-(2-propinyl)amino, N-cyclohexylamino, N-cyclohexenylamino, anilino, pyridylamino, imidazolylamino, benzimidazolylamino, thiazolylamino, benzothiazolylamino, or triazinylamino), a sulfamoyl group (preferably a sulfamoyl group having 0 to 20 carbon atoms, preferably an alkyl-, cycloalkyl-, or aryl-sulfamoyl group, e.g. N,N-dimethylsulfamoyl, N-cyclohexylsulfamoyl, or N-phenylsulfamoyl), an acyl group (preferably an acyl group having 1 to 20 carbon atoms, e.g. acetyl, cyclohexylcarbonyl, or benzoyl), an acyloxy group (preferably an acyloxy group having 1 to 20 carbon atoms, e.g. acetyloxy, cyclohexylcarbonyloxy, or benzoyloxy), a carbamoyl group (preferably a carbamoyl group having 1 to 20 carbon atoms, preferably an alkyl-, cycloalkyl-, or aryl-carbamoyl group, e.g. N,N-dimethylcarbamoyl, N-cyclohexylcarbamoyl, or N-phenylcarbamoyl),
an acylamino group (preferably an acylamino group having 1 to 20 carbon atoms, e.g. acetylamino, cyclohexylcarbonylamino, or benzoylamino), a sulfonamide group (preferably a sulfonamide group having 0 to 20 carbon atoms, preferably an alkyl-, cycloalkyl-, or aryl-sulfonamide group, e.g. methane sulfonamide, benzene sulfonamide, N-methyl methane sulfonamide, N-cyclohexyl sulfonamide, or N-ethyl benzene sulfonamide), an alkylthio group (preferably an alkylthio group having 1 to 20 carbon atoms, e.g. methylthio, ethylthio, isopropylthio, or benzylthio), a cycloalkylthio group (preferably a cycloalkylthio group having 3 to 20 carbon atoms, e.g. cyclopropylthio, cyclopentylthio, cyclohexylthio, or 4-methylcyclohexylthio), an arylthio group (preferably an arylthio group having 6 to 26 carbon atoms, e.g. phenylthio, 1-naphthylthio, 3-methylphenylthio, or 4-methoxyphenylthio), an alkyl-, cycloalkyl-, or aryl-sulfonyl group (preferably a sulfonyl group having 1 to 20 carbon atoms, e.g. methylsulfonyl, ethylsulfonyl, cyclohexylsulfonyl, or benzene sulfonyl),
a silyl group (preferably a silyl group having 1 to 20 carbon atoms, preferably an alkyl-, aryl-, alkoxy-, or aryloxy-substituted silyl group, e.g. triethylsilyl, triphenylsilyl, diethylbenzylsilyl, or dimethylphenylsilyl), a silyloxy group (preferably a silyloxy group having 1 to 20 carbon atoms, preferably an alkyl-, aryl-, alkoxy-, or aryloxy-substituted silyloxy group, e.g. triethylsilyloxy, triphenylsilyloxy, diethylbenzylsilyloxy, or dimethylphenylsilyloxy), a hydroxyl group, a cyano group, a nitro group, a halogen atom (e.g. fluorine, chlorine, bromine, or iodine atom), a carboxyl group, a sulfo group, a phosphonyl group, a phosphoryl group, and a boric-acid group.

When the compound, the substituent or the like contains an alkyl group or an alkenyl group, these may be a straight chain or a branched chain, and these may be substituted or unsubstituted. Further, in the case of containing an aryl group, a heterocyclic group or the like, these may be a single ring or a fused ring, and may be substituted or unsubstituted.

### <Counter electrode (opposite electrode)>

The counter electrode is preferably an electrode working as a positive electrode in the dye-sensitized solar cell (photoelectrochemical cell). The counter electrode usually has the same meaning as the electrically-conductive support described above, but in a construction which is likely to maintain a sufficient strength, a support is not necessarily required. A preferred structure of the counter electrode is a structure having a high charge collecting effect. At least one of the electrically-conductive support and the counter electrode as mentioned above should be substantially transparent, in order for light to reach the photoconductor layer. In the dye-sensitized solar cell of the present invention, the electrically-conductive support is preferably transparent to allow sunlight to inject from the support side. In this case, the counter electrode further preferably has properties of reflecting light. As the counter electrode of the dye-sensitized solar cell, a glass or plastic plate on which a metal or an electrically-conductive oxide is deposited is preferable, and a glass plate on which platinum is deposited is particularly preferable. In the dye-sensitized solar cell, a lateral side of the cell is preferably sealed with a polymer, an adhesive, or the like, in order to prevent evaporation of components.

The present invention can be applied to the photoelectric conversion elements and the dye-sensitized solar cells described, for example, in Japanese Patent No. 4260494, JP-A-2004-146425, JP-A-2000-340269, JP-A-2002-289274, JP-A-2004-152613, and JP-A-9-27352. In addition, the present invention can be applied to the photoelectric conversion elements and the dye-sensitized solar cells described, for example, in JP-A-2004-152613, JP-A-2000-90989, JP-A-2003-217688, JP-A-2002-367686, JP-A-2003-323818, JP-A-2001-43907, JP-A-2000-340269, JP-A-2005-85500, JP-A-2004-273272, JP-A-2000-323190, JP-A-2000-228234, JP-A-2001-266963, JP-A-2001-185244,JP-T-2001-525108,JP-A-2001-203377,JP-A-2000-100483, JP-A-2001-210390, JP-A-2002-280587, JP-A-2001-273937, JP-A-2000-285977, and JP-A-2001-320068.

### <<Dye solution, dye-adsorbed electrode using the same, and production method of dye-sensitized solar cell>>

In the present invention, the dye-adsorbed electrode is preferably produced using a dye solution containing the metal complex dye of the present invention.

The foregoing dye solution contains the metal complex dye of the present invention dissolved in an organic solvent, and may comprise a co-adsorbent and other ingredients as needed.

Examples of the solvent to be used include solvents described in JP-A-2001-291534, but the solvent is not particularly limited thereto. In the present invention, organic solvents are preferred. More preferred are alcohols, amides, nitriles, alcohols, hydrocarbons, and a mixed solvent of two or more kinds of these solvents. As the mixed solvent, preferred are mixed solvents of alcohols and a solvent selected from amides, nitriles, and hydrocarbons. More preferred are mixed solvents of alcohols and amides and mixed solvents of alcohols and hydrocarbons, and particularly preferred are mixed solvents of alcohols and amides. In specific, methanol, ethanol, propanol, butanol, dimethylformamide, and dimethylacetamide are preferred.

The dye solution preferably contains a co-adsorbent, and the co-adsorbent is preferably the aforementioned ones. Among them, the compound represented by Formula (CA) is preferred.

The dye solution of the present invention is preferably one in which the concentrations of the metal complex dye and the co-adsorbent have been adjusted so that the dye solution can be used as it is at the time of preparation of a photoelectric conversion element or a dye-sensitized solar cell. In the present invention, the metal complex dye of the present invention is preferably contained in an amount of from 0.001 to 0.1% by mass.

In the dye solution, it is particularly preferable to adjust the water content, and thus in the present invention, it is preferred that the content (content rate) of water is adjusted to the range of from 0 to 0.1% by mass.

Similarly, it is also preferable to adjust the water content in the electrolyte in a photoelectric conversion element and a dye-sensitized solar cell, in order to achieve the effects of the present invention effectively. Thus, it is preferred that the content (content rate) of water in the electrolyte solution is adjusted to the range of from 0 to 0.1 % by mass. The foregoing adjustment of the electrolyte is particularly preferably carried out with the dye solution.

In the present invention, a dye-adsorbed electrode is preferably a semiconductor electrode for dye-sensitized solar cell, which is prepared by allowing the surface of the semiconductor fine particles provided on the semiconductor electrode, to carry the metal complex dye, with using the above dye solution.

In other words, the dye-adsorbed electrode for dye-sensitized solar cell preferably has a photoconductor layer which is obtained by coating a composition obtained from the aforementioned dye solution, on an electrically-conductive support provided with semiconductor fine particles, and curing the composition after coating.

In the present invention, it is preferable that a dye-sensitized solar cell be produced by using the dye-adsorbed electrode for dye-sensitized solar cell, preparing an electrolyte and a counter electrode, and performing an assembly with using them.

### EXAMPLES

The present invention will be described in more detail based on examples given below, but the invention is not meant to be limited by these.

### [Synthesis of metal complex dyes]

### Example 1 [Synthesis of Metal complex dye Ru^{II}(LD-1-1)(LA-1-1)]

According to the following reaction scheme, a ligand LD-1-1 was synthesized, and then a metal complex dye Ru^{II}(LD-1-1)(LA-1-1) was synthesized.

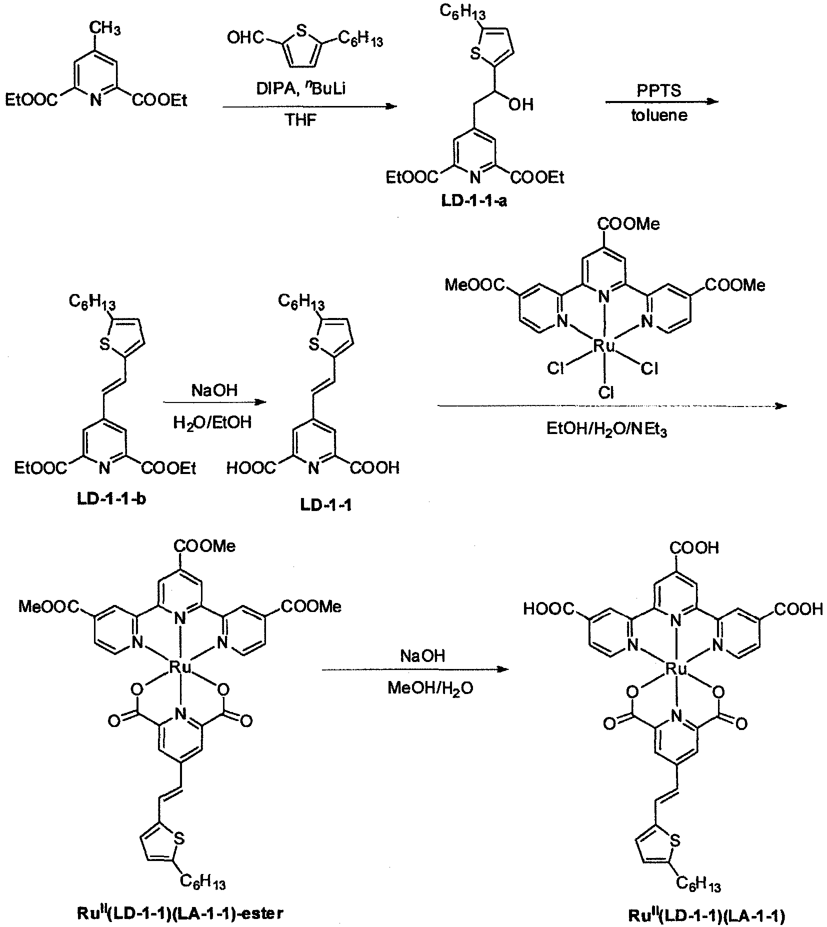

### (1) Synthesis of compound LD-1-1-a

Under a nitrogen atmosphere, 5.4 g of diisopropylamine (DIPA) was dissolved into 25 mL of tetrahydrofuran (THF), and the resultant mixture was cooled to -20°C. Then, 35 mL of n-butyllithium (1.6 mol/L hexane solution) was added dropwise thereto, and the resultant mixture was stirred at -20°C for 30 minutes. Then, 50 mL of tetrahydrofuran solution of 6.4 g of 2,6-diethylcarboxy-4-methylpyridine was added dropwise thereto, and the resultant mixture was stirred at 0°C for 75 minutes. To this suspension, 10 mL of tetrahydrofuran solution of 3.5 g of 5-hexylthiophene-2-carboxyaldehyde was added dropwise, and the resultant mixture was stirred at room temperature for 90 minutes. Then, 100 mL of saturated aqueous solution of ammonium chloride was added thereto, and then subjected to liquid separation, and the resultant organic layer was condensed under reduced pressure. The resulting crude product was purified by silica gel column chromatography, to obtain 5.2 g of the target compound.

### (2) Synthesis of compound LD-1-1-b

Under a nitrogen atmosphere, 4.9 g of compound LD-1-1-a and 3.7 g of PPTS (pyridinium p-toluenesulfonate) were added to 50 mL of toluene, and the resultant mixture was heated and refluxed for 4 hours. Liquid separation was performed using 50 mL of saturated aqueous solution of sodium hydrogencarbonate and 100 mL of ethyl acetate, and the resultant organic layer was condensed. The resulting crude product was recrystallized from methanol, to obtain 4.5 g of the target compound.

### (3) Synthesis of ligand LD-1-1

4.2 g of the compound LD-1-1-b was dissolved in 50 mL of ethanol at 50°C. Thereto, a solution in which 1.0 g of sodium hydroxide was dissolved in a mixture liquid having 50 ml of ethanol and 5 ml of water was added, and stirred for 2 hours at room temperature. While cooling the reaction solution on ice, 0.2 M hydrochloride acid aqueous solution was gradually added thereto, until pH became 2. Precipitated crystals were filtered, and the obtained crude product was recrystallized from methanol, to obtain 3.8 g of the target compound.

### (4) Synthesis of Ru^{II}(LD-1-1)(LA-1-1)-ester

Under a nitrogen atmosphere, 305 mg of (Me₃-tctpy)RuCl₃ (note: Me₃-tctpy is 4,4',4"-tri(methoxycarbonyl)-2,2':6',2"-terpyridyl), 260 mg of compound LD-1-1, and 1.5 mL of triethylamine were added to a mixed solution of 500 mL of ethanol and 100 mL of water, and the resultant mixture was heated and refluxed for 3 hours. The resultant reaction solution was concentrated under reduced pressure. 20 mL of ethyl acetate and 200 mL of water were added thereto, and the resultant mixture was subjected to liquid separation. The resultant organic layer was condensed under reduced pressure. The resulting crude product was purified by silica gel column chromatography, to obtain 294 mg of the target compound.

### (5) Synthesis of metal complex dye Ru^{II}(LD-1-1)(LA-1-1)

Under a nitrogen atmosphere, 100 mg of Ru^{II}(LD-1-1)(LA-1-1)-ester was added to 30 mL of methanol, and completely dissolved thereinto. Then, 10 mL of 1 N aqueous solution of sodium hydroxide was added thereto, and the resultant mixture was stirred at room temperature for 12 hours. The resultant reaction mixture was concentrated under reduced pressure, the resulting solid was dissolved into 30 mL of water, and 10 mL of 1 N aqueous solution of trifluoromethanesulfonic acid was added to this solution. A precipitated solid was collected by filtration, and washed with 10 mL of water, and subsequently with 10 mL of diethyl ether, to obtain 82 mg of the target compound.

### Example 2 [Synthesis of metal complex dye Ru^{II}(LD-1-26)(LA-1-1)]

A ligand LD-1-26 was synthesized according to the following reaction scheme, and a metal complex dye Ru^{II}(LD-1-26)(LA-1-1) was synthesized in the same manner as in the synthesis of the metal complex dye Ru^{II}(LD-1-1)(LA-1-1).

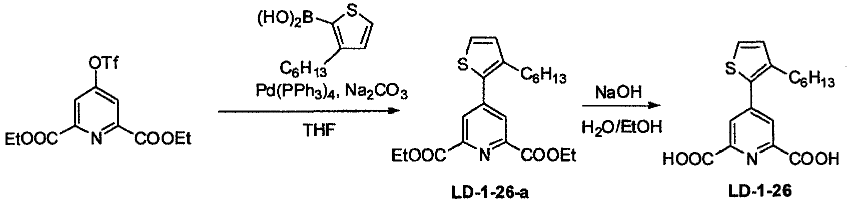

### Example 3 [Synthesis of metal complex dye Ru^{II}(LD-3-1)(LA-1-1)NCS]

According to the following reaction scheme, a ligand LD-3-1 was synthesized, and then a metal complex dye Ru^{II}(LD-3-1)(LA-1-1)NCS was synthesized.

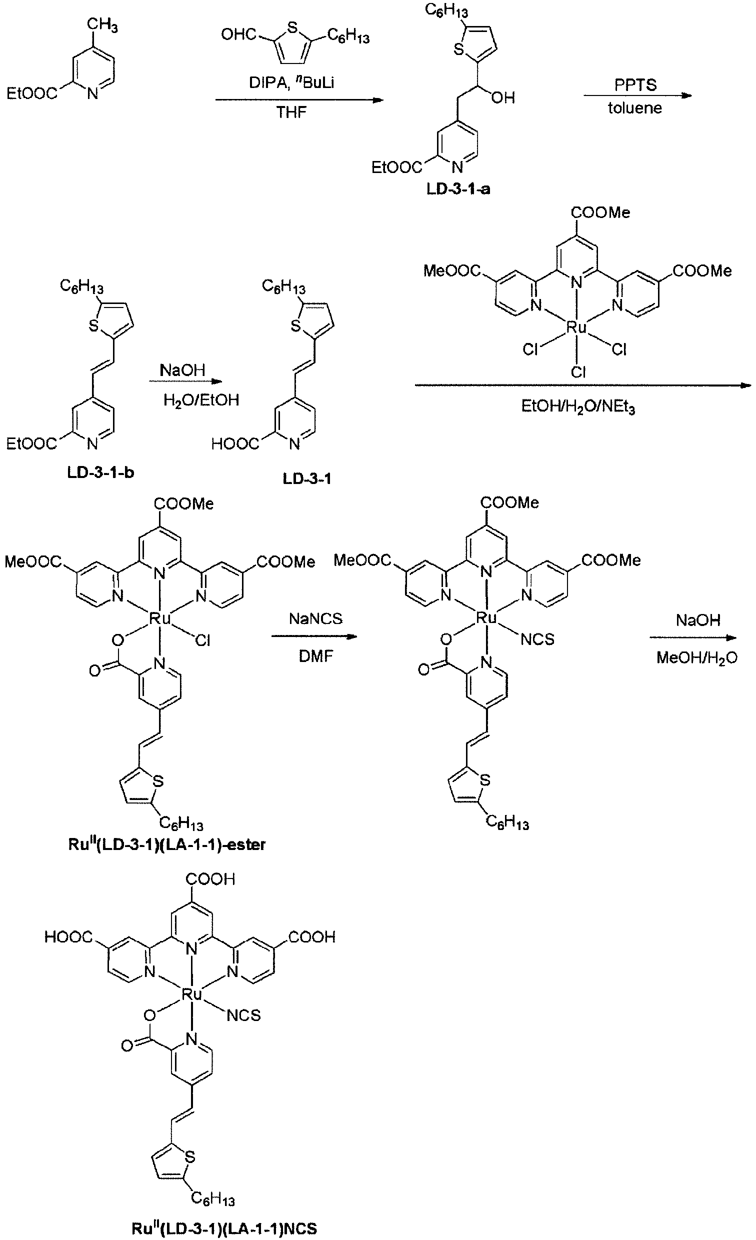

### Example 4 [Synthesis of metal complex dye Ru^{II}(LD-3-5)(LA-1-1)NCS]

A ligand LD-3-5 was synthesized according to the following reaction scheme, and a metal complex dye Ru^{II}(LD-3-5)(LA-1-1)NCS was synthesized in the same manner as the synthesis of the metal complex dye Ru^{II}(LD-3-1)(LA-1-1)NCS.

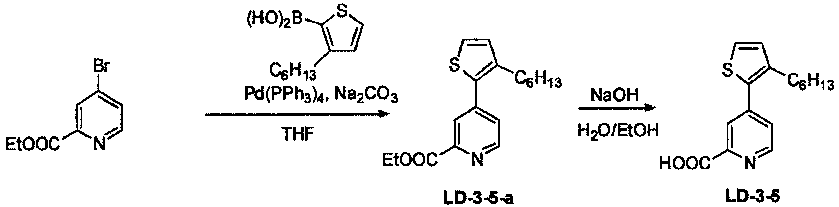

### Example 5 [Synthesis of metal complex dye Ru^{II}(LD-1-6)(LA-1-1)]

A ligand LD-1-6 was synthesized in the same manner as the ligand LD-1-1, and a metal complex dye Ru^{II}(LD-1-6)(LA-1-1) was synthesized in the same manner as in the synthesis of the metal complex dye Ru^{II}(LD-1-1)(LA-1-1).

### Example 6 [Synthesis of metal complex dye Ru^{II}(LD-1-9)(LA-1-1)]

A ligand LD-1-9 was synthesized in the same manner as the ligand LD-1-1, and a metal complex dye Ru^{II}(LD-1-9)(LA-1-1) was synthesized in the same manner as in the synthesis of the metal complex dye Ru^{II}(LD-1-1)(LA-1-1).

### Example 7 [Synthesis of metal complex dye Ru^{II}(LD-1-24)(LA-1-1)]

A ligand LD-1-24 was synthesized in the same manner as the ligand LD-1-26, and a metal complex dye Ru^{II}(LD-1-24)(LA-1-1) was synthesized in the same manner as in the synthesis of the metal complex dye Ru^{II}(LD-1-26)(LA-1-1).

### Example 7 [Synthesis of metal complex dye Ru^{II}(LD-2-12)(LA-1-1)]

A ligand LD-2-12 was synthesized according to the following reaction scheme, and a metal complex dye Rul^{II}(LD-2-12)(LA-1-1) was synthesized in the same manner as in the synthesis of the metal complex dye Ru^{II}(LD-1-1)(LA-1-1).

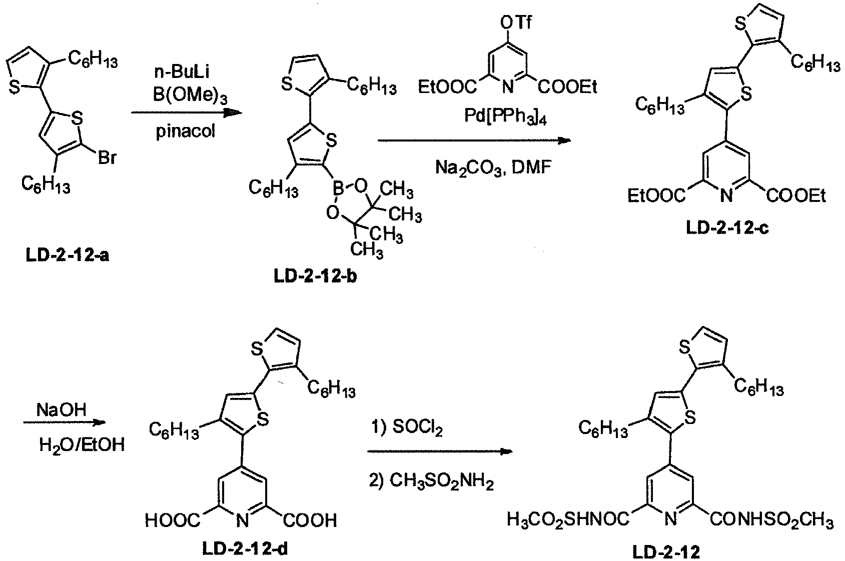

### Example 8 [Synthesis of metal complex dye Ru^{II}(LD-1-24)(LA-3-1)]

A ligand LA-3-1Me was synthesized according to the following method, and a metal complex dye Ru^{II}(LD-1-24)(LA-3-1) was synthesized in the same manner as in the synthesis of the metal complex dye Ru^{II}(LD-1-24)(LA-1-1).

### (Synthesis of ligand LA-3-1Me)

The stannylation reaction described below was applied to 5 g of 2-bromopyrimidine, to give the stannylated LA-3-1A. The stille coupling was applied to, using 2 g of dimethyl 6-bromo-2,2'-bipyridine-4,4'-dicarboxylate and the above-described stannylated LA-3-1A, to give Ligand LA-3-1Me which was a dimethyl ester of Ligand LA-3-1.

### (Stannylation reaction)

To about 5 g of a substrate with which a halogen had been substituted, 1.2 times mole of bis(tributyltin) and 0.05 times mole of tetrakis(triphenylphosphineparadium) were refluxed in 100ml of toluene under a nitrogen atmosphere. Completion of the reaction was confirmed by a thin-layer chromatography. After cooling to room temperature, the reaction liquid was filtrated and concentrated, followed by separation and refinement, using partition column chromatography equipment (AI-580, manufactured by Yamazen) and a mixed solvent of n-hexane, ethyl acetate and methanol as an eluent, by flowing the eluent while controlling the concentration gradient. By concentration of the target fraction, the stannylated product was obtained.

### Example 9 [Synthesis of metal complex dye Ru^{II}(LD-1-26)(LA-3-3)N⁺(C₄H₉)₄]

A ligand LA-3-3Me was synthesized according to the following reaction scheme, and a metal complex dye Ru^{II}(LD-1-26)(LA-3-3)N⁺(C₄H₉)₄ was synthesized in the same manner as in the synthesis of the metal complex dye Ru^{II}(LD-1-26)(LA-1-1).

### (Synthesis of ligand LA-3-3Me)

Ligand LA-3-3Me, which was a dimethyl ester of Ligand LA-3-3, was obtained in the same manner as Ligand LA-3-1Me, except that 5g of 2-bromopyrimidine in the synthesis of Ligand LA-3-1Me was replaced with an equimolar amount of 2-bromobenzothiazole.

Other metal complex dyes used in Example 10 were also synthesized in the same manner as in the above Examples 1 to 9.

The structure of each metal complex dye was determined by MS (mass spectrum) measurement. The MS measurement results of the metal complex dyes are summarized and shown in the following Table 1.

**Table 1**

| Metal complex dye | MS |
|---|---|
| Ru^{II}(LD-1-1)(LA-1-1) | 824.07 |
| Ru^{II}(LD-1-2)(LA-1-1) | 840.07 |
| Ru^{II}(LD-1-3)(LA-1-1) | 856.04 |
| Ru^{II}(LD-1-6)(LA-1-1) | 880.14 |
| Ru^{II}(LD-1-9)(LA-1-1) | 882.08 |
| Ru^{II}(LD-1-24)(LA-1-1) | 964.25 |
| Ru^{II}(LD-1-26)(LA-1-1) | 798.06 |
| Ru^{II}(LD-1-15)(LA-2-3) | 1002.14 |
| Ru^{II}(LD-1-30)(LA-1-1)N⁺(C₄H₉)₄ | 1026.33 |
| Ru^{II}(LD-2-7)(LA-1-1) Cl₂ | 866.10 |
| Ru^{II}(LD-2-7)(LA-1-2) Cl₂ | 882.10 |
| Ru^{II}(LD-2-9)(LA-1-1)(NCS)₂ | 979.25 |
| Ru^{II}(LD-2-12)(LA-1-1) | 1046.19 |
| Ru^{II}(LD-3-1)(LA-1-1) NCS | 839.07 |
| Ru^{II}(LD-3-5)(LA-1-1) NCS | 799.07 |
| Ru^{II}(LD-3-6)(LA-1-1) NCS | 979.13 |
| Ru^{II}(LD-4-1)(LA-1-1) NCS N⁺(C₄H₉)₄ | 1067.35 |
| Ru^{II}(LD-4-13)(LA-1-1) NCS N⁺(C₄H₉)₄ | 1221.33 |
| Ru^{II}(LD-4-6)(LA-2-3) NCS N⁺(C₄H₉)₄ | 1405.54 |
| Ru^{II}(LD-4-7)(LA-2-1) (NCS)₂ | 999.22 |
| Ru^{II}(LD-5-1)(LA-3-1) | 992.22 |
| Ru^{II}(LD-5-3)(LA-3-4) | 938.17 |
| Ru^{II}(LD-1-24)(LA-3-1) | 921.25 |
| Ru^{II}(LD-1-26)(LA-3-3) N⁺(C₄H₉)₄ | 1039.32 |
| Ru^{II}(LD-1-26)(LA-3-4) N⁺(C₄H₉)₄ | 982.32 |
| Ru^{II}(LD-1-1)(LA-2-12) | 880.11 |

### Example 10 [Preparation of dye-sensitized solar cell]

According to the procedure described below, a photoelectrode having the same configuration as that of the photoelectrode 12 shown in Fig. 5 of JP-A-2002-289274 was produced, and using the photoelectrode, a dye-sensitized solar cell 1 of a scale of 10 mm × 10 mm having the same configuration as that of the dye-sensitized solar cell 20 shown in Fig. 3 of JP-A-2002-289274 except for the photoelectrode, was produced. The specific configuration thereof was shown in Fig. 2 attached to the present application. In Fig. 2 of the present application, 41 denotes a transparent electrode, 42 denotes a semiconductor electrode, 43 denotes a transparent electrically-conductive film, 44 denotes a substrate, 45 denotes a semiconductor layer, 46 denotes a light-scattering layer, 40 denotes a photoelectrode, 20 denotes a dye-sensitized solar cell, CE denotes a counter electrode, E denotes an electrolyte, and S denotes a spacer.

### (Preparation of paste)

(Paste A) Spherical TiO₂ particles (anatase, a mean particle diameter: 25 nm, hereinafter, referred to as spherical TiO₂ particles A) were put into a nitric acid solution, and the resultant mixture was stirred to prepare a titania slurry. Next, a cellulose-based binder was added to the titania slurry as a thickening agent, and the resultant mixture was kneaded to prepare a paste.

(Paste 1) Spherical TiO₂ particles A and spherical TiO₂ particles (anatase, a mean particle diameter: 200 nm, hereinafter, referred to as spherical TiO₂ particles B) were put into a nitric acid solution, and the resultant mixture was stirred to prepare a titania slurry. Next, a cellulose-based binder was added to the titania slurry as a thickening agent, and the resultant mixture was kneaded to prepare a paste ((mass of TiO₂ particles A):(mass of TiO₂ particles B) = 30:70).

(Paste 2) Rod-shaped TiO₂ particles (anatase, diameter: 100 nm, aspect ratio: 5, hereinafter, referred to as rod-shaped TiO₂ particles C) were mixed with the paste A, to prepare a paste having (mass of rod-shaped TiO₂ particles C):(mass of the paste A) = 30:70.

### (Production of semiconductor electrode)

A transparent electrode was prepared in which a fluorine-doped SnO₂ electrically-conductive film (thickness: 500 nm) was formed on a glass substrate. On this SnO₂ electrically-conductive film, the paste 1 was applied to by screen printing, followed by drying. Then, the paste was calcined under the conditions of 450°C in the air. Further, by repeating this screen printing and calcination with using the paste 2, the semiconductor electrode having the same configuration as that of the semiconductor electrode 42 shown in Fig. 2 of the present application (the area of the light-receiving face: 10 mm × 10 mm; the layer thickness: 10 µm; the layer thickness of the semiconductor layer: 6 µm; the layer thickness of the light-scattering layer: 4 µm; and the content of the rod-shaped TiO₂ particles C contained in the light-scattering layer: 30% by mass) was formed on the SnO₂ electrically-conductive film. Thus, the photoelectrode, which did not contain the dye, was prepared.

### (Adsorption of dye)

Next, a dye was adsorbed onto the semiconductor electrode (precursor of a dye-adsorbed electrode) as follows. First, using a 1:1 (volume ratio) mixture of anhydrous t-butanol and dimethyl formamide, dehydrated over magnesium ethoxide, as a solvent, each of metal complex dyes described in Table 2 below was dissolved to be 3 × 10⁻⁴ mol/L. Further, as a co-adsorbent, 20 mol of equimolar mixture of chenodeoxycholic acid and cholic acid was added per 1 mol of metal complex dye, to prepare each dye solution. The measurement of the moisture content in the dye solution based on Karl Fisher titration showed that water was less than 0.01% by mass. Next, the semiconductor electrode was immersed into this solution for 10 hours at 40°C and dried at 50°C after pulling out from this solution, to complete a photoelectrode 40 in which about 2 × 10⁻⁷ mol/cm² of dye was adsorbed onto the semiconductor electrode.

### (Assembly of dye-sensitized solar cell)

Then, prepared were, as a counter electrode, a platinum electrode (thickness of Pt thin film, 100nm) having the same shape and size as those of the photoelectrode, and, as an electrolyte, an iodine-based redox propionitrile solution containing 0.1 M of iodine, 0.05 M of lithium iodide, and 0.25 M of 4-t-butylpyridine. Further, a spacer-S (trade name: "Surlyn") manufactured by DuPont, which had a shape matching to the size of the semiconductor electrode, was prepared. The photoelectrode 40 and the counter electrode CE were arranged to face each other, with the spacer-S interposed therebetween, thermally compressed, and followed by filling the electrolyte in the inside thereof. Thus, each dye-sensitized solar cell (sample numbers 101 to 126, and c11 to c17) was completed. The performance evaluations of these solar cells were conducted as described below.

### (Evaluation Test 1)

Cell characteristic test was conducted to measure short-circuit current density (Jsc, unit: mA/cm²), open-circuit voltage (Voc, unit: V), and fill factor (FF) of each of the dye-sensitized solar cells, and photoelectric conversion efficiency [η (%)] was measured by dividing the cell output by incident energy. For the cell characteristic test, a solar simulator (PEC-L12 manufactured by Peccell Technologies, Inc.) was used. For the property evaluation, I-V characteristics measurement device (PECK2400-N) manufactured by Peccell Technologies, Inc. was used. The obtained results were evaluated based on the following evaluation criteria.

### Evaluation criteria

A: The photoelectric conversion efficiency was 1.3 times or higher than Comparative Compound (1).
B: The photoelectric conversion efficiency was 1.1 times or higher but less than 1.3 times
C: The photoelectric conversion efficiency was less than 1.1 times.

Meanwhile, this is shown as initial property 1 in the following Table 2.

### (Evaluation Test 2)

For evaluation of adsorption power of metal complex dye onto titanium oxide surface, a desorption rate of the metal complex dye from the titanium oxide surface was used as an index.

The desorption rate of the metal complex dye was calculated by means of a Quartz Crystal microbalance with Dissipation monitoring (QCM-D) intermolecular interaction measuring apparatus E1 (manufactured by Meiwafosis).

Paste A (anatase, average particle size: 25 nm) was printed by screen printing (film thickness: 20 µm) on a gold sensor (manufactured by Meiwafosis) for use for the QCM-D. By calcining the thus-printed gold sensor at 450°C for 1 hour in the air, to prepare a gold sensor having a semiconductor layer (photoconductor layer) adsorbed thereon.

The thus-prepared sensor was installed in the QCM-D intermolecular interaction measuring apparatus, and 0.2 mM of a dye solution (DMF/t-BuOH=1/1) was flowed therein, to make the dye adsorb on the semiconductor layer (photoconductor layer) in a dye adsorption amount of a predetermined value (200 µg/cm²). The dye adsorption amount was calculated from a resonance frequency shift (ΔF) of a quartz oscillator according to the following Sauerbrey equation.$ΔF = - 2 \times {{F}_{0}}^{2} \times Δm / A {\left(µ\times P\right)}^{1 / 2}$

In the formula, F₀ represents a frequency of a quartz oscillator alone, Δm represents a mass change, A represents a piezoelectrically active area of the Au electrode, and µ and P represent quartz density and modulus of rigidity, respectively.

Then, by flowing the above-described electrolyte E at 80°C for 4 hours, the desorption amount of the dye was measured. The desorption amount of the dye was also calculated according to the Sauerbrey equation. The desorption rate was calculated from the resultant desorption amount of the dye. The rate obtained was evaluated based on the following criteria.

### Evaluation criteria

A: 0.0 µg/cm²/hr or more but less than 2.5 µg/cm²/hr
B: 2.5 µg/cm²/hr or more but less than 10.0 µg/cm²/hr
C: 10.0 µg/cm²/hr or more

Meanwhile, this is shown as desorption rate in the following Table 2.

### (Evaluation Test 3)

Each semiconductor electrode that had been adsorbed with dye as described above was observed with a naked eye from the opposite side of the transparent electrode, and evenness of dyeing by the dye was evaluated based on the following criteria.

### Evaluation criteria

A: Uneven dye adsorption is not observed.
B: Slightly uneven dye adsorption is observed.
C: There is a great deal of uneven dye adsorption.

Meanwhile, this is shown as electrode unevenness in the following Table 2.

### (Evaluation Test 4)

The outer periphery and inlet for an electrolyte (electrolytic solution) of the prepared cell were sealed and cured with using Resin XNR-5516 manufactured by Nagase ChemteX Corporation. The photoelectric conversion efficiency values were measured before and after keeping the cell for 200 hours under an environment of 50°C, and the ratio thereof is obtained as a deterioration rate.

### Evaluation criteria

AA: The deterioration rate was minus (that is, improved)
A: The deterioration rate was between 100 to 90%
C: The deterioration rate was less than 90%

Meanwhile, this is shown as thermal deterioration in the following Table 2.

### (Evaluation Test 5)

The cell was produced in the same manner as above, except that the electrolyte was changed to a propionitirle solution of 0.2 M of tris(bipyridine)cobalt bis(hexafluorophosphate), 0.02 M of tris(bipyridine)cobalt tris(hexafluorophosphate), 0.1 M of lithium perchlorate, and 0.5 M of 4-t-butylpyridine. Photoelectric conversion efficiency was evaluated, and evaluation was made based on the following evaluation criteria, in comparison with the case of Evaluation Test 1.

### Evaluation criteria

A: The relative value was 1.3 times or more.
B: The relative value was 1.1 times or more and less than 1.3 times.
C: The relative value was less than 1.1 times.

Meanwhile, this is shown as initial property 2 in the following Table 2.

### (Evaluation Test 6)

In order to simulate a high speed cell manufacture, cell were produced with the time for immersion into a dye solution shortened, to 1/10 of Evaluation Test 1. Photoelectric conversion efficiency was evaluated for each of them, and the evaluation was made based on the following evaluation criteria, in comparison with the case of Evaluation Test 1.

### Evaluation criteria

A: The relative value was 0.9 time or more.
B: The relative value was 0.7 time or more and less than 0.9 time.
C: The relative value was less than 0.7 time

Meanwhile, this is shown as high-speed suitability in the following Table 2.

Results of each evaluation are summarized and described in the following Table 2.

**Table 2**

| Sample No. | Metal complex dye | Initial property 1 | Desorption rate | Electrode unevenness | Thermal deterioration | Initial property 2 | High-speed suitability | Remarks |
|---|---|---|---|---|---|---|---|---|
| 101 | Ru^{II}(LD-1-1)(LA-1-1) | B | A | A | A | B | B | This invention |
| 102 | Ru^{II}(LD-1-2)(LA-1-1) | B | A | A | A | B | B | This invention |
| 103 | Ru^{II}(LD-1-3)(LA-1-1) | B | A | A | A | B | B | This invention |
| 104 | Ru^{II}(LD-1-6)(LA-1-1) | B | A | A | A | B | B | This invention |
| 105 | Ru^{II}(LD-1-9)(LA-1-1) | B | A | A | A | B | B | This invention |
| 106 | Ru^{II}(LD-1-24)(LA-1-1) | B | A | A | A | B | B | This invention |
| 107 | Ru^{II}(LD-1-26)(LA-1-1) | B | A | A | A | B | B | This invention |
| 108 | Ru^{II}(LD-1-15)(LA-2-3) | B | A | A | A | B | B | This invention |
| 109 | Ru^{II}(LD-1-30)(LA-1-1)N⁺(C₄H₉)₄ | B | A | A | A | B | B | This invention |
| 110 | Ru^{II}(LD-2-7)(LA-1-1)Cl₂ | B | A | A | B | A | B | This invention |
| 111 | Ru^{II}(LD-2-7)(LA-1-2)Cl₂ | B | A | A | B | A | B | This invention |
| 112 | Ru^{II}(LD-2-9)(LA-1--1)(NCS)₂ | B | A | A | B | A | B | This invention |
| 113 | Ru^{II}(LD-2-12)(LA-1-1) | B | A | A | B | A | B | This invention |
| 114 | Ru^{II}(LD-3-1)(LA-1-1)NCS | B | A | A | B | B | A | This invention |
| 115 | Ru^{II}(LD-3-5)(LA-1-1)NCS | B | A | A | B | B | A | This invention |
| 116 | Ru^{II}(LD-3-6)(LA-1-1)NCS | B | A | A | B | B | A | This invention |
| 117 | Ru^{II}(LD-4-1)(LA-1-1)NCSN⁺(C₄H₉)₄ | A | A | A | B | B | B | This invention |
| 118 | Ru^{II}(LD-4-13)(LA-1-1)NCSN⁺(C₄H₉)₄ | A | A | A | B | B | B | This invention |
| 119 | Ru^{II}(LD-4-6)(LA-2-3)NCSN⁺(C₄H₉)₄ | A | A | A | B | B | B | This invention |
| 120 | Ru^{II}(LD-4-7)(LA-2-1)(NCS)₂ | A | A | A | B | B | B | This invention |
| 121 | Ru^{II}(LD-5-1)(LA-3-1) | A | A | A | B | B | B | This invention |
| 122 | Ru^{II}(LD-5-3)(LA-3-4) | A | A | A | B | B | B | This invention |
| 123 | Ru^{II}(LD-1-24)(LA-3-1) | A | B | A | B | B | B | This invention |
| 124 | Ru^{II}(LD-1-26)(LA-3-3)N⁺(C₄H₉)₄ | A | B | A | B | B | B | This invention |
| 125 | Ru^{II}(LD-1-26)(LA-3-4)N⁺(C₄H₉)₄ | A | B | A | B | B | B | This invention |
| 126 | Ru^{II}(LD-1-1)(LA-2-12) | A | B | A | B | B | B | This invention |
| c11 | Comparative compound (1) | C | C | C | C | C | C | Comparative example |
| c12 | Comparative compound (2) | C | C | C | C | C | C | Comparative example |
| c13 | Comparative compound (3) | C | C | C | C | C | C | Comparative example |
| c14 | Comparative compound (4) | C | C | C | C | C | c | Comparative example |
| c15 | Comparative compound (5) | C | C | C | C | C | C | Comparative example |
| c16 | Comparative compound (6) | C | C | C | C | C | C | Comparative example |
| c17 | Comparative compound (7) | C | C | C | C | C | C | Comparative example |

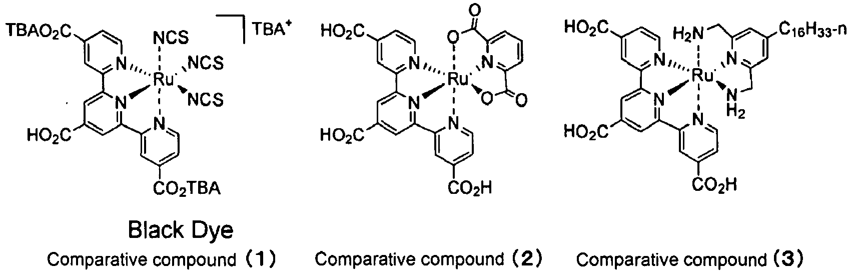

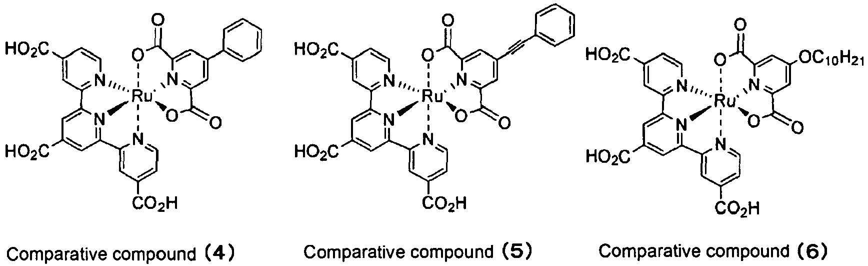

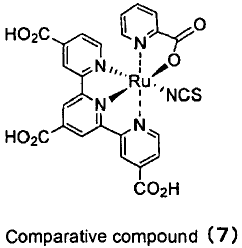

As it is evident from above Table 2, all of the dye-sensitized solar cells in which the metal complex dyes of the present invention were used had excellent photoelectric conversion efficiency and durability. They were also found to be quite excellent in that they could exhibit stable performance without causing electrode unevenness during continuous production.

### REFERENCE SIGNS LIST

1 Electrically conductive support
2 Photoconductor layer
21 Dye
22 Semiconductor fine particles
3 Charge transfer layer
4 Counter electrode
5 Light-receiving electrode
6 Circuit
10 Photoelectric conversion element
100 System utilizing a dye-sensitized solar cell
M Electric motor (Electric fan)
20 Dye-sensitized solar cell
40 Photoelectrode
41 Transparent electrode
42 Semiconductor electrode
43 Transparent electrically-conductive film
44 Substrate
45 Semiconductor layer
46 Light-scattering layer
CE Counter electrode
E Electrolyte
S Spacer

## Claims

1. A photoelectric conversion element, containing:
an electrically conductive support;
a photoconductor layer containing an electrolyte;
a charge-transfer layer containing an electrolyte; and
a counter electrode;
wherein the photoconductor layer contains semiconductor fine-particles carrying a metal complex dye represented by Formula (I):
M(LD)(LA)(X)_{mX}·(CI)_{mY} Formula (I)
wherein M represents Fe²⁺, Ru²⁺ or Os²⁺;
LD represents a ligand represented by Formula (DL);
LA represents a bidentate or tridentate ligand having a nitrogen-containing aromatic heterocyclic skeleton and at least one acidic group;
X represents a monodentate or bidentate ligand; mX represents an integer of 0 to 3;
CI represents a counter ion necessary for neutralizing an electric charge; and
mY represents an integer of 0 to 2; 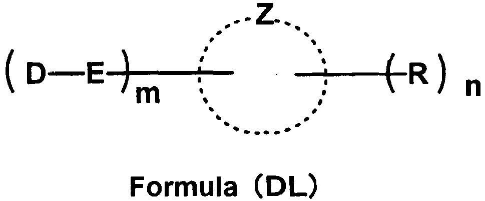
wherein Z represents a group of nonmetallic atoms necessary for forming a ring, and it may contain, as a ring-forming atom, a coordinating atom which coordinates to the aforementioned M; D represents a coordinating atom selected from an oxygen atom and a nitrogen atom and coordinates to the aforementioned M; E represents a single bond, -O-, -N(R¹)-, -C(R²)₂-, -C(=R³)-, -C(=O)-, -C(=NR¹)-, or -C(R²)₂-C(=Q)-, in which R¹ and R² each independently represent a hydrogen atom, an alkyl group, an aryl group, a heteroaryl group, an alkoxy group, or an aryloxy group; R³ represents an alkylidene group, an alkenylidene group, or a cycloalkylidene group; two R²s may be the same or different from one another; R represents a substituent; m and n each independently represent an integer of 1 or more;
wherein at least one coordinating atom is a coordinating atom A and, among Rs in the number of n, at least one is a substituent W, and D never forms a ring with E or Z;
herein, the coordinating atom A is an oxygen or nitrogen atom which does not have an unsaturated bonding arm, and the substituent W is an ethenyl group, an ethynyl group, an aryl group, or a heteroaryl group, each of which is substituted with a monovalent alkyl group having 6 to 18 carbon atoms or with a substituent having the alkyl group.

2. The photoelectric conversion element according to Claim 1, wherein the ligand represented by Formula (DL) is represented by any one of Formulas (DL-1) to (DL-3): 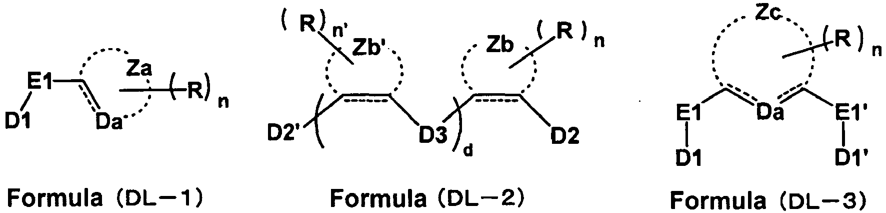 wherein R and n each have the same meaning as R and n in Formula (DL); n' represents an integer of 0 or more; Za, Zb, Zb' and Zc each represent a group of nonmetallic atoms necessary for forming a ring; the bond linking the carbon atom to which -E1-D1 is attached and Da, the bond linking the carbon atom to which D2 or D2' is attached and the carbon atom to which D3 is attached, and the bond linking the carbon atom to which -E1-D1 or -E1'-D1' is attached and Da may be either a single bond or a double bond; D1 to D3, D1', D2' and Da each independently represent a coordinating atom which coordinates to M; D1 to D3, D1' and D2' each represent an atom selected from the group consisting of an oxygen atom and a nitrogen atom; Da represents an atom selected from the group consisting of an oxygen atom, a nitrogen atom, and a carbon atom; E1 and E1' each independently represent a single bond, -O-, - N(R¹)-, -C(R²)₂-, -C(=R³)-, -C(=O)-, -C(=NR¹)-, or -C(R²)₂-C(=O)-, in which R¹ and R² each independently represent a hydrogen atom, an alkyl group, an aryl group, a heteroaryl group, an alkoxy group, or an aryloxy group; R³ represents an alkylidene group, an alkenylidene group, or a cycloalkylidene group; two R²s may be the same or different from one another; d represents 0 or 1; when d is 1, Zb and Zb' may be bonded to each other to form a ring; and when n and n' each are an integer of 2 or more, plural Rs may be bonded with each other to form a ring.

3. The photoelectric conversion element according to Claim 1 or 2, wherein the ligand represented by Formula (DL) is represented by any one of Formulas (DL-1a), (DL-1b), (DL-2a), (DL-2b), (DL-3a), and (DL-3b): 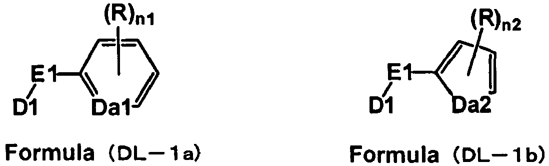 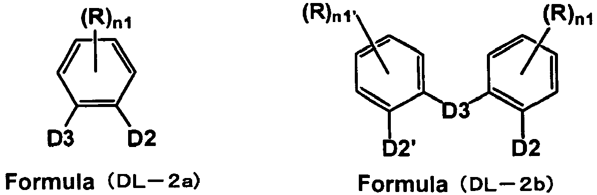 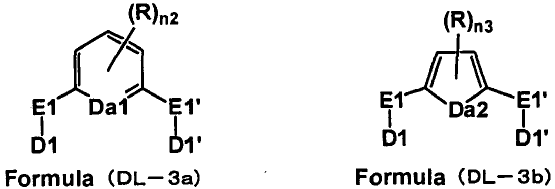 wherein R has the same meaning as R in Formula (DL); n1 represents an integer of 1 to 4; n2 represents an integer of 1 to 3; n3 represents 1 or 2; n1' represents an integer of 0 to 4; D1 to D3, D1', D2', Da1, and Da2 each independently represent a coordinating atom which coordinates to M; D1 to D3, D1', and D2' each represent an atom selected from the group consisting of an oxygen atom and a nitrogen atom; Da1 and Da2 each represent an atom selected from the group consisting of an oxygen atom, a nitrogen atom and a carbon atom; E1 and E1' each independently represent a single bond, -O-, -N(R¹)-, -C(R²)₂-, -C(=R³)-, -C(=O)-, -C(=NR¹)-, or -C(R²)₂-C(=O)-, in which R¹ and R² each independently represent a hydrogen atom, an alkyl group, an aryl group, a heteroaryl group, an alkoxy group, or an aryloxy group; R³ represents an alkylidene group, an alkenylidene group, or a cycloalkylidene group; two R²s may be the same or different from one another; and when n1 to n3 and n1' each are an integer of 2 or more, plural Rs may be bonded with each other to form a ring.

4. The photoelectric conversion element according to any one of Claims 1 to 3, wherein the substituent W is 1) an ethenyl group or ethynyl group having, at position 2, an aryl group or heteroaryl group which is substituted with an alkyl group having 6 to 18 carbon atoms, an alkylamino group having 6 to 18 carbon atoms, an alkoxy group having 6 to 18 carbon atoms, or an alkylthio group having 6 to 18 carbon atoms, or 2) an aryl group or heteroaryl group substituted with an alkyl group having 6 to 18 carbon atoms, an alkylamino group having 6 to 18 carbon atoms, an alkoxy group having 6 to 18 carbon atoms, or an alkylthio group having 6 to 18 carbon atoms.

5. The photoelectric conversion element according to any one of Claims 1 to 4, wherein the substituent W is represented by any one of Formulas (W-1) to (W-6): 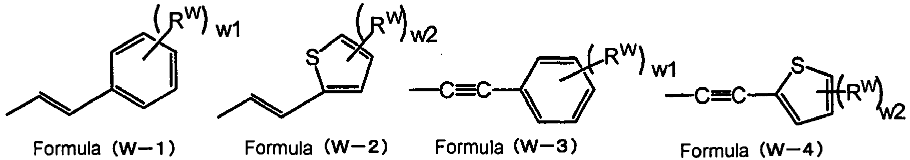 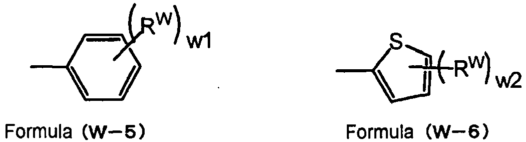 wherein R^{w} represents a monovalent alkyl group having 6 to 18 carbon atoms or a group having the alkyl group; w1 represents an integer of 1 to 5; and w2 represents an integer of 1 to 3.

6. The photoelectric conversion element according to any one of Claims 1 to 5, wherein LA is represented by any one of Formulas (AL-1) to (AL-6) : 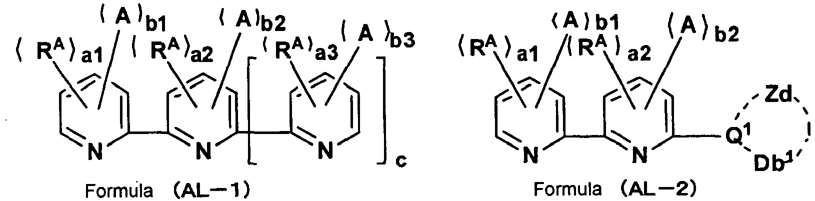 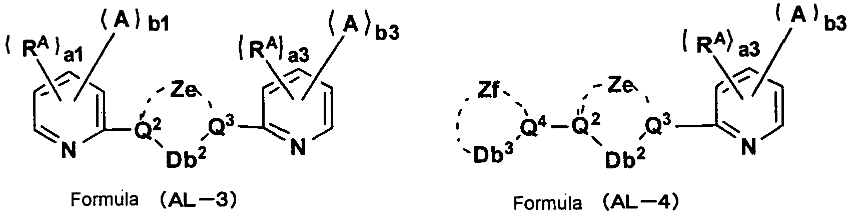 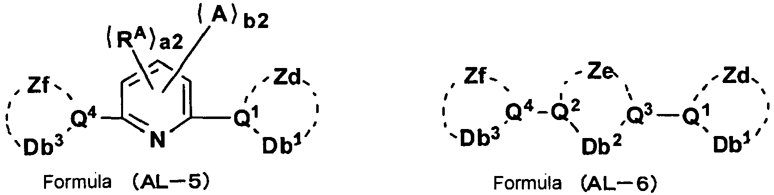
wherein Zd, Ze, and Zf each independently represent a benzene ring, a pyrrole ring, an imidazole ring, a pyrazole ring, a pyrazine ring, a pyrimidine ring, a pyridazine ring, a triazole ring, an oxazole ring, a triazine ring, a thiazole ring, an isothiazole ring, an oxazole ring, an isoxazol ring, a furan ring, a thiophene ring, a pyrrolidine ring, a piperidine ring, a morpholine ring, a piperazine ring, a tetrahydrofuran ring, a tetrahydropyran ring, a 4H-pyran ring, a 1,4-dihydropyridine ring, a tetradehydromorpholine ring, or a benzene-fused ring of these rings; A represents an acidic group; Q¹ to Q⁴ each independently represent a carbon atom or a nitrogen atom; Db¹ to Db³ each independently represent a nitrogen atom having a lone electron pair, an anionic nitrogen atom, or an anionic carbon atom; R^{A} represents a substituent; a1, a3, b1, and b3 each independently represent an integer of 0 to 4; a2 and b2 each independently represent an integer of 0 to 3; c represents 0 or 1; and
the ligands represented by Formulas (AL-1) to (AL-6) have at least one acidic group.

7. The photoelectric conversion element according to any one of Claims 1 to 6, wherein LA is represented by Formula (AL-1): 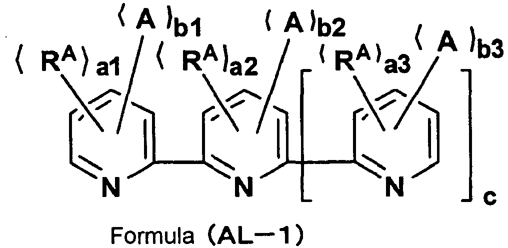
wherein A represents an acidic group; R^{A} represents a substituent; a1, a3, b1 and b3 each independently represent an integer of 0 to 4; a2 and b2 each independently represent an integer of 0 to 3; c represents 0 or 1; and
the ligand represented by Formula (AL-1) has at least one acidic group.

8. The photoelectric conversion element according to any one of Claims 1 to 7, wherein X in Formula (I) is NCS⁻, Cl⁻, Br⁻, I⁻, CN⁻, NCO⁻, or H₂O.

9. The photoelectric conversion element according to any one of Claims 1 to 8, wherein CI in Formula (I) is a halogen ion, an arylsulfonate ion, an aryldisulfonate ion, an alkylsulfate ion, a sulfate ion, a thiocyanate ion, a perchlorate ion, a tetrafluoroborate ion, a hexafluorophosphate ion, an acetate ion, a trifluoromethanesulfonate ion, an ammonium ion, an alkali metal ion, or a hydrogen ion.

10. A dye-sensitized solar cell, comprising the photoelectric conversion element according to any one of Claims 1 to 9.

11. A metal complex dye represented by Formula (I):
M(LD)(LA)(X)_{mX}·(CI)_{mY} Formula (I)
wherein M represents Fe²⁺, Ru²⁺ or Os²⁺;
LD represents a ligand represented by Formula (DL);
LA represents a bidentate or tridentate ligand having a nitrogen-containing aromatic heterocyclic skeleton and at least one acidic group.
X represents a monodentate or bidentate ligand; mX represents an integer of 0 to 3;
CI represents a counter ion necessary for neutralizing an electric charge; and
mY represents an integer of 0 to 2; 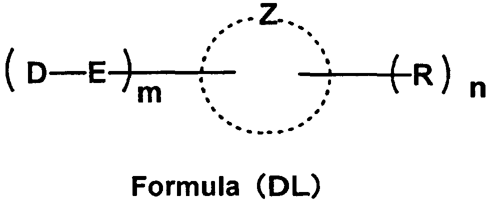
wherein Z represents a group of nonmetallic atoms necessary for forming a ring, and it may contain, as a ring-forming atom, a coordinating atom which coordinates to the aforementioned M; D represents a coordinating atom selected from an oxygen atom and a nitrogen atom and coordinates to the aforementioned M; E represents a single bond, -O-, -N(R¹)-, -C(R²)₂-, -C(=R³)-, -C(=O)-, -C(=NR¹)-, or -C(R²)₂-C(=O)-, in which R¹ and R² each independently represent a hydrogen atom, an alkyl group, an aryl group, a heteroaryl group, an alkoxy group, or an aryloxy group; R³ represents an alkylidene group, an alkenylidene group, or a cycloalkylidene group; two R²s may be the same or different from one another; R represents a substituent; m and n each independently represent an integer of 1 or more;
wherein at least one coordinating atom is a coordinating atom A and, among Rs in the number of n, at least one is a substituent W, and D never forms a ring with E or Z;
herein, the coordinating atom A is an oxygen or nitrogen atom which does not have an unsaturated bonding arm, and the substituent W is an ethenyl group, an ethynyl group, an aryl group, or a heteroaryl group, each of which is substituted with a monovalent alkyl group having 6 to 18 carbon atoms or with a substituent having the alkyl group.

12. The metal complex dye according to Claim 11, wherein the ligand represented by Formula (DL) is represented by any one of Formulas (DL-1) to (DL-3): 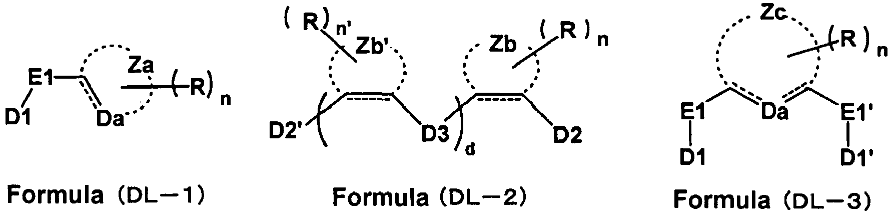 wherein R and n each have the same meaning as R and n in Formula (DL); n' represents an integer of 0 or more; Za, Zb, Zb' and Zc each represent a group of nonmetallic atoms necessary for forming a ring; the bond linking the carbon atom to which -E1-D1 is attached and Da, the bond linking the carbon atom to which D2 or D2' is attached and the carbon atom to which D3 is attached, and the bond linking the carbon atom to which -E1-D1 or -E1'-D1' is attached and Da may be either a single bond or a double bond; D1 to D3, D1', D2' and Da each independently represent a coordinating atom which coordinates to M; D1 to D3, D1' and D2' each represent an atom selected from the group consisting of an oxygen atom and a nitrogen atom; Da represents an atom selected from the group consisting of an oxygen atom, a nitrogen atom, and a carbon atom; E1 and E1' each independently represent a single bond, -O-,-N(R¹)-, -C(R²)₂-, -C(=R³)-, -C(=O)-, -C(=NR¹)-, or -C(R²)₂-C(=O)-, in which R¹ and R² each independently represent a hydrogen atom, an alkyl group, an aryl group, a heteroaryl group, an alkoxy group, or an aryloxy group; R³ represents an alkylidene group, an alkenylidene group, or a cycloalkylidene group; two R²s may be the same or different from one another; d represents 0 or 1; when d is 1, Zb and Zb' may be bonded to each other to form a ring; and when n and n' each are an integer of 2 or more, plural Rs may be bonded with each other to form a ring.

13. The metal complex dye according to Claim 11 or 12, wherein the ligand represented by Formula (DL) is represented by any one of Formulas (DL-1a), (DL-1b), (DL-2a), (DL-2b), (DL-3a) and (DL-3b): 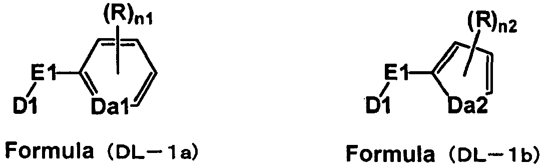 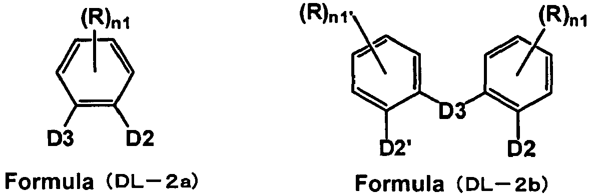 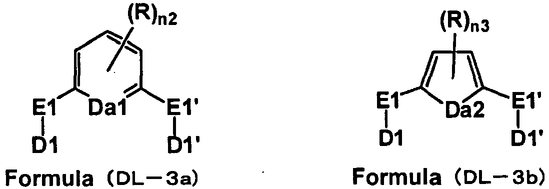 wherein R has the same meaning as R in Formula (DL); n1 represents an integer of 1 to 4; n2 represents an integer of 1 to 3; n3 represents 1 or 2; n1' represents an integer of 0 to 4; D1 to D3, D1', D2', Da1, and Da2 each independently represent a coordinating atom which coordinates to M; D1 to D3, D1', and D2' each represent an atom selected from the group consisting of an oxygen atom and a nitrogen atom; Da1 and Da2 each represent an atom selected from the group consisting of an oxygen atom, a nitrogen atom and a carbon atom; E1 and E1' each independently represent a single bond, -O-, -N(R¹)-, -C(R²)₂-, -C(=R³)-, -C(=O)-, -C(=NR¹)-, or -C(R²)₂-C(=O)-, in which R¹ and R² each independently represent a hydrogen atom, an alkyl group, an aryl group, a heteroaryl group, an alkoxy group, or an aryloxy group; R³ represents an alkylidene group, an alkenylidene group, or a cycloalkylidene group; two R²s may be the same or different from one another; and when n1 to n3 and n1' each are an integer of 2 or more, plural Rs may be bonded with each other to form a ring.

14. The metal complex dye according to any one of Claims 11 to 13, wherein the substituent W is 1) an ethenyl group or ethynyl group having, at position 2, an aryl group or heteroaryl group which is substituted with an alkyl group having 6 to 18 carbon atoms, an alkylamino group having 6 to 18 carbon atoms, an alkoxy group having 6 to 18 carbon atoms, or an alkylthio group having 6 to 18 carbon atoms, or 2) an aryl group or heteroaryl group substituted with an alkyl group having 6 to 18 carbon atoms, an alkylamino group having 6 to 18 carbon atoms, an alkoxy group having 6 to 18 carbon atoms, or an alkylthio group having 6 to 18 carbon atoms.

15. The metal complex dye according to any one of Claims 11 to 14, wherein the substituent W is represented by any one of Formulas (W-1) to (W-6): 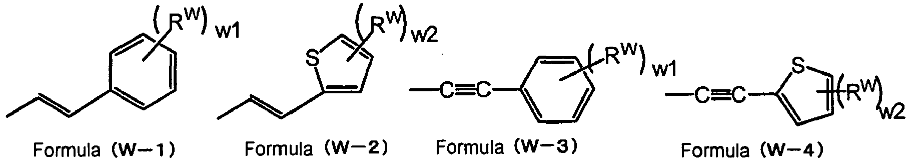 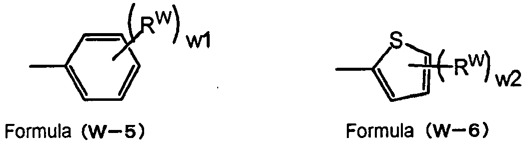 wherein R^{W} represents a monovalent alkyl group having 6 to 18 carbon atoms or a group having the alkyl group; w1 represents an integer of 1 to 5; and w2 represents an integer of 1 to 3.

16. The metal complex dye according to any one of Claims 11 to 15, wherein LA is represented by any one of Formulas (AL-1) to (AL-6): 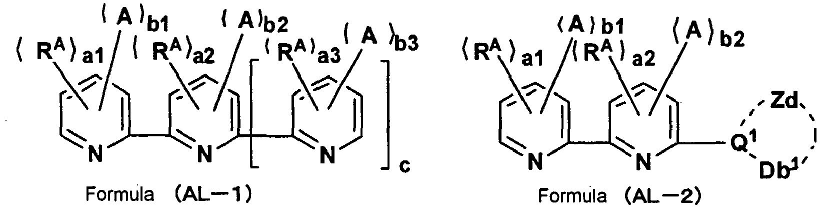 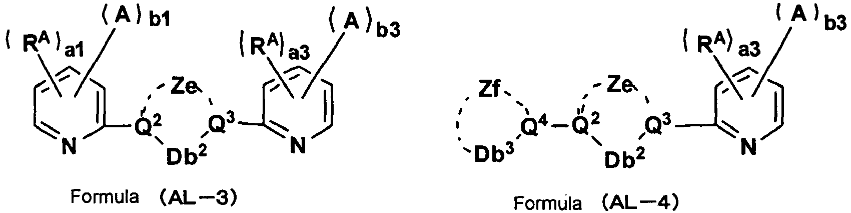 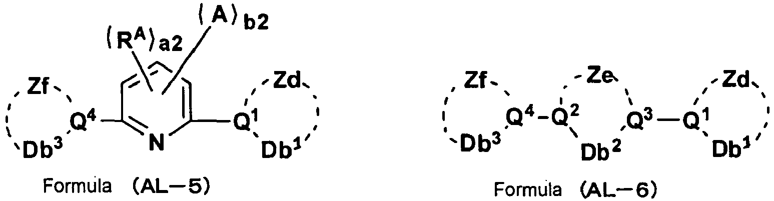
wherein Zd, Ze, and Zf each independently represent a benzene ring, a pyrrole ring, an imidazole ring, a pyrazole ring, a pyrazine ring, a pyrimidine ring, a pyridazine ring, a triazole ring, an oxazole ring, a triazine ring, a thiazole ring, an isothiazole ring, an oxazole ring, an isoxazol ring, a furan ring, a thiophene ring, a pyrrolidine ring, a piperidine ring, a morpholine ring, a piperazine ring, a tetrahydrofuran ring, a tetrahydropyran ring, a 4H-pyran ring, a 1,4-dihydropyridine ring, a tetradehydromorpholine ring, or a benzene-fused ring of these rings; A represents an acidic group; Q¹ to Q⁴ each independently represent a carbon atom or a nitrogen atom; Db¹ to Db³ each independently represent a nitrogen atom having a lone electron pair, an anionic nitrogen atom, or an anionic carbon atom; R^{A} represents a substituent; a1, a3, b1, and b3 each independently represent an integer of 0 to 4; a2 and b2 each independently represent an integer of 0 to 3; c represents 0 or 1; and
the ligands represented by Formulas (AL-1) to (AL-6) have at least one acidic group.

17. The metal complex dye according to any one of Claims 11 to 16, wherein LA is represented by Formula (AL-1): 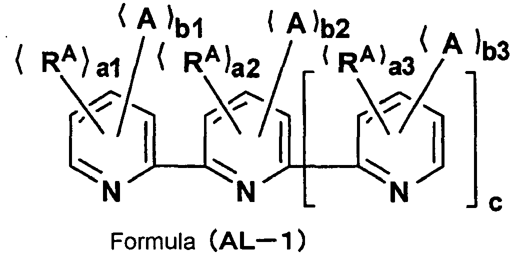
wherein A represents an acidic group; R^{A} represents a substituent; a1, a3, b1 and b3 each independently represent an integer of 0 to 4; a2 and b2 each independently represent an integer of 0 to 3; c represents 0 or 1; and
the ligand represented by Formula (AL-1) has at least one acidic group.

18. A dye solution, comprising the metal complex dye according to any one of Claims 11 to 17 dissolved in an organic solvent.

19. A dye-adsorbed electrode for a dye-sensitized solar cell, having a photoconductor layer which is obtained by coating an electrically-conductive support which has been applied with semiconductor fine particles with a dye solution according to Claim 18, followed by reacting and curing the dye solution.

## Patentansprüche

1. Fotoelektrisches Umwandlungselement, enthaltend:
einen elektrisch leitfähigen Träger;
eine. Fotoleiterschicht, die einen Elektrolyt enthält;
eine Ladungstransferschicht, die einen Elektrolyt enthält; und
eine Gegenelektrode;
worin die Fotoleiterschicht Halbleiter-Feinpartikel enthält, die einen durch die Formel (I) dargestellten Metallkomplexfarbstoff tragen:
**M(LD)(LA)(X)_{mX}·(CI)_{mY}** Formel (I)
worin M Fe²⁺, Ru²⁺ oder Os²⁺ darstellt;
LD einen durch die Formel (DL) dargestellten Liganden darstellt;
LA einen zweizähnigen oder dreizähnigen Liganden darstellt, der ein stickstoffhaltiges aromatisches heterocyclisches Gerüst und mindestens eine saure Gruppe aufweist;
X einen einzähnigen oder zweizähnigen Liganden darstellt; mX eine ganze Zahl von 0 bis 3 darstellt;
CI ein Gegenion darstellt, das zum Neutralisieren einer elektrischen Ladung notwendig ist; und
mY eine ganze Zahl von 0 bis 2 darstellt; 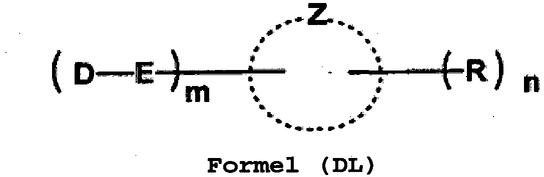
worin Z eine Gruppe von Nicht-Metallatomen darstellt, die zum Bilden eines Rings notwendig sind, und als ringbildendes Atom ein koordinierendes Atom enthalten kann, welches an das vorstehend erwähnte M koordiniert; D ein koordinierendes Atom darstellt, ausgewählt aus einem Sauerstoffatom und einem Stickstoffatom, und welches an das vorstehend erwähnte M koordiniert; E eine Einfachbindung, -O-, -N(R¹)-, -C(R²)₂-, -C(=R³)-, -C(=O)-, -C(=NR¹)- oder -C(R²)₂-C(=O) darstellt, worin R¹ und R² jeweils unabhängig ein Wasserstoffatom, eine Alkylgruppe, eine Arylgruppe, eine Heteroarylgruppe, eine Alkoxygruppe oder eine Aryloxygruppe darstellen; R³ eine Alkylidengruppe, eine Alkenylidengruppe oder eine Cycloalkylidengruppe darstellt; wobei zwei R² gleich oder voneinander verschieden sein können; R einen Substituenten darstellt; m und n jeweils unabhängig eine ganze Zahl von 1 oder größer darstellen;
worin mindestens ein koordinierendes Atom ein koordinierendes Atom A ist und unter den R in der Anzahl n zumindest eines ein Substituent W ist, und D niemals einen Ring mit E oder Z bildet;
worin das koordinierende Atom A ein Sauerstoff- oder Stickstoffatom ist, welches keinen ungesättigten Bindungsarm aufweist, und der Substituent W eine Ethenylgruppe, eine Ethinylgruppe, eine Arylgruppe oder eine Heteroarylgruppe ist, von denen jede mit einer monovalenten Alkylgruppe mit 6 bis 18 Kohlenstoffatomen oder mit einem Substituenten, der die Alkylgruppe aufweist, substituiert ist.

2. Fotoelektrisches Umwandlungselement gemäß Anspruch 1, worin der durch die Formel (DL) dargestellte Ligand durch irgendeine der Formeln (DL-1) bis (DL-3) dargestellt wird: 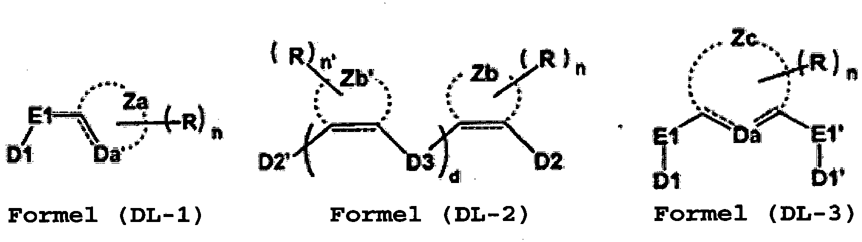 worin R und n jeweils die gleiche Bedeutung wie R und n in Formel (DL) besitzen; n' eine ganze Zahl von 0 oder größer darstellt; Za, Zb, Zb' und Zc jeweils eine Gruppe von Nicht-Metallatomen darstellen, die zum Bilden eines Rings erforderlich ist; die Bindung, die das Kohlenstoffatom, an das -E1-D1 gebunden ist, und Da verknüpft, die Bindung, die das Kohlenstoffatom, an das D2 oder D2' gebunden ist, und das Kohlenstoffatom, an welches D3 gebunden ist, verknüpft, und die Bindung, die das Kohlenstoffatom, an welches -E1-D1 oder -E1'-D1' gebunden ist, und Da verknüpft, entweder eine Einfachbindung oder eine Doppelbindung sein kann; D1 bis D3, D1', D2' und Da jeweils unabhängig ein koordinierendes Atom darstellen, welches an M koordiniert; D1 bis D3, D1' und D2' jeweils ein Atom darstellen, ausgewählt aus der Gruppe, bestehend aus einem Sauerstoffatom und einem Stickstoffatom; Da ein Atom darstellt, ausgewählt aus der Gruppe, bestehend aus einem Sauerstoffatom, einem Stickstoffatom und einem Kohlenstoffatom; E1 und E1' jeweils unabhängig eine Einfachbindung, -O-, -N(R¹)-, -C(R²)₂-, -C(=R³)-, -C(=O)-, -C(=NR¹)- oder -C(R²)₂-C(=O)- darstellen, worin R¹ und R² jeweils unabhängig ein Wasserstoffatom, eine Alkylgruppe, eine Arylgruppe, eine Heteroarylgruppe, eine Alkoxygruppe oder eine Aryloxygruppe darstellen; R³ eine Alkylidengruppe, eine Alkenylidengruppe oder eine Cycloalkylidengruppe darstellt; zwei R² gleich oder voneinander verschieden sein können; d 0 oder 1 darstellt; wenn d 1 ist, Zb und Zb' miteinander verbunden sein können, um einen Ring zu bilden; und wenn n und n' jeweils eine ganze Zahl von 2 oder größer sind, mehrere R miteinander verbunden sein können, um einen Ring zu bilden.

3. Fotoelektrisches Umwandlungselement gemäß Anspruch 1 oder 2, worin der durch die Formel (DL) dargestellte Ligand durch irgendeine der Formeln (DL-1a), (DL-1b), (DL-2a), (DL-2b), (DL-3a) und (DL-3b) dargestellt wird: 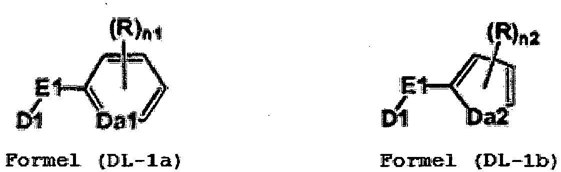 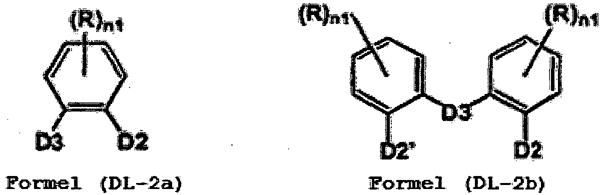 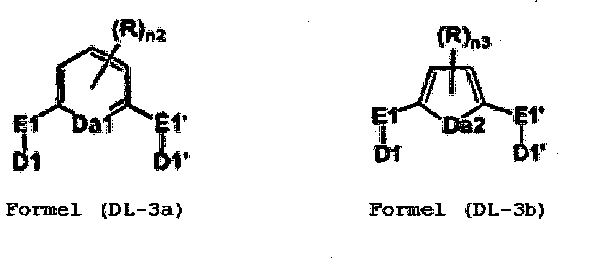 worin R die gleiche Bedeutung wie R in Formel (DL) besitzt; n1 eine ganze Zahl von 1 bis 4 darstellt; n2 eine ganze Zahl von 1 bis 3 darstellt; n3 1 oder 2 darstellt; n1' eine ganze Zahl von 0 bis 4 darstellt; D1 bis D3, D1', D2', Da1 und Da2 jeweils unabhängig ein koordinierendes Atom darstellen, welches an M koordiniert; D1 bis D3, D1' und D2' jeweils ein Atom darstellen, ausgewählt aus der Gruppe, bestehend aus einem Sauerstoffatom und einem Stickstoffatom; Da1 und Da2 jeweils ein Atom darstellen, ausgewählt aus der Gruppe, bestehend aus einem Sauerstoffatom, einem Stickstoffatom und einem Kohlenstoffatom; E1 und E1' jeweils unabhängig eine Einfachbindung, , -O-, -N(R¹)-, -C(R²)₂-, -C(=R³)-, -C(=O)-, -C(=NR¹)- oder-C(R²)₂-C(=O)- darstellen, worin R¹ und R² jeweils unabhängig ein Wasserstoffatom, eine Alkylgruppe, eine Arylgruppe, eine Heteroarylgruppe, eine Alkoxygruppe oder eine Aryloxygruppe darstellen; R³ eine Alkylidengruppe, eine Alkenylidengruppe oder eine Cycloalkylidengruppe darstellt; zwei R² gleich oder voneinander verschieden sein können; und wenn n1 bis n3 und n1' jeweils eine ganze Zahl von 2 oder größer sind, die mehreren R miteinander verbunden sein können, um einen Ring zu bilden.

4. Fotoelektrisches Umwandlungselement gemäß irgendeinem der Ansprüche 1 bis 3, worin der Substituent W 1) eine Ethenylgruppe oder eine Ethinylgruppe ist, die an Position 2 eine Arylgruppe oder eine Heteroarylgruppe aufweist, welche mit einer Alkylgruppe mit 6 bis 18 Kohlenstoffatomen, einer Alkylaminogruppe mit 6 bis 18 Kohlenstoffatomen, einer Alkoxygruppe mit 6 bis 18 Kohlenstoffatomen oder einer Alkylthiogruppe mit 6 bis 18 Kohlenstoffatomen substituiert ist, oder 2) eine Arylgruppe oder Heteroarylgruppe, die mit einer Alkylgruppe mit 6 bis 18 Kohlenstoffatomen, einer Alkylaminogruppe mit 6 bis 18 Kohlenstoffatomen, einer Alkoxygruppe mit 6 bis 18 Kohlenstoffatomen oder einer Alkylthiogruppe mit 6 bis 18 Kohlenstoffatomen substituiert ist, ist.

5. Fotoelektrisches Umwandlungselement gemäß irgendeinem der Ansprüche 1 bis 4, worin der Substituent W durch irgendeine der Formeln (W-1) bis (W-6) dargestellt wird: 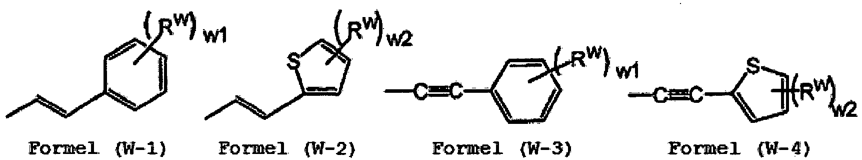 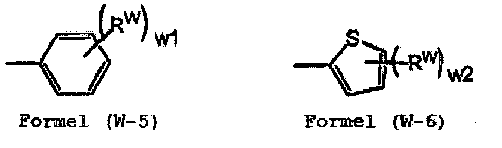 worin R^{w} eine monovalente Alkylgruppe mit 6 bis 18 Kohlenstoffatomen oder eine Gruppe, die die Alkylgruppe aufweist, darstellt; w1 eine ganze Zahl von 1 bis 5 darstellt; und w2 eine ganze Zahl von 1 bis 3 darstellt.

6. Fotoelektrisches Umwandlungselement gemäß irgendeinem der Ansprüche 1 bis 5, worin LA durch irgendeine der Formeln (AL-1) bis (AL-6) dargestellt wird: 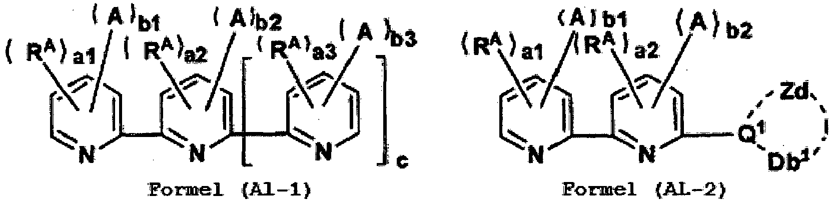 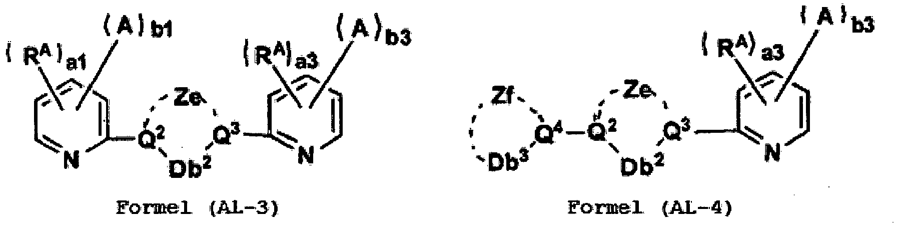 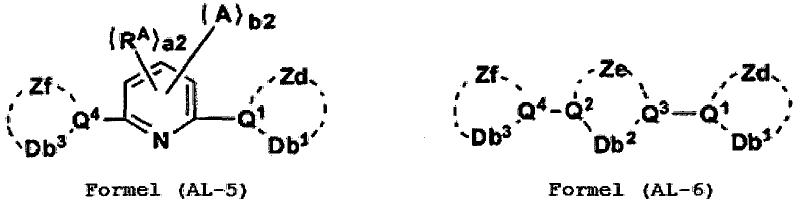
worin Zd, Ze und Zf jeweils unabhängig einen Benzolring, einen Pyrrolring, einen Imidazolring, einen Pyrazolring, einen Pyrazinring, einen Pyrimidinring, einen Pyridazinring, einen Triazolring, einen Oxazolring, einen Triazinring, einen Thiazolring, einen Isothiazolring, einen Oxazolring, einen Isoxazolring, einen Furanring, einen Thiophenring, einen Pyrrolidinring, einen Piperidinring, einen Morpholinring, einen Piperazinring, einen Tetrahydrofuranring, einen Tetrahydropyranring, einen 4H-Pyranring, einen 1,4-Dihydropyridinring, einen Tetradehydromorpholinring oder einen Benzolkondensierten Ring dieser Ringe darstellt; A eine saure Gruppe darstellt; Q¹ bis Q⁴ jeweils unabhängig ein Kohlenstoffatom oder ein Stickstoffatom darstellen; Db¹ bis Db³ jeweils unabhängig ein Stickstoffatom mit einem einsamen Elektronenpaar, ein anionisches Stickstoffatom oder ein anionisches Kohlenstoffatom darstellen; R^{A} einen Substituenten darstellt; a1, a3, b1 und b3 jeweils unabhängig eine ganze Zahl von 0 bis 4 darstellen; a2 und b2 jeweils unabhängig eine ganze Zahl von 0 bis 3 darstellen; c 0 oder 1 darstellt; und
die durch die Formeln (AL-1) bis (AL-6) dargestellten Liganden mindestens eine saure Gruppe aufweisen.

7. Fotoelektrisches Umwandlungselement gemäß irgendeinem der Ansprüche 1 bis 6, worin LA durch die Formel (AL-1) dargestellt wird: 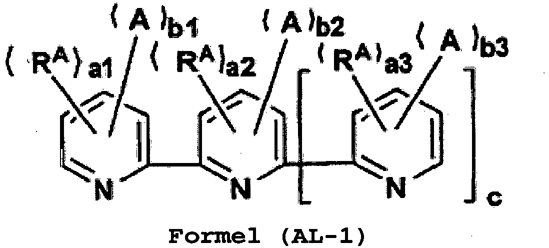
worin A eine saure Gruppe darstellt; R^{A} einen Substituenten darstellt; a1, a3, b1 und b3 jeweils unabhängig eine ganze Zahl von 0 bis 4 darstellen; a2 und b2 jeweils unabhängig eine ganze Zahl von 0 bis 3 darstellen; c 0 oder 1 darstellt; und
der durch die Formel (AL-1) dargestellte Ligand mindestens eine saure Gruppe aufweist.

8. Fotoelektrisches Umwandlungselement gemäß irgendeinem der Ansprüche 1 bis 7, worin X in der Formel (I) NCS⁻, CL⁻, Br⁻, I⁻, CN⁻, NCO⁻ oder H₂O ist.

9. Fotoelektrisches Umwandlungselement gemäß irgendeinem der Ansprüche 1 bis 8, worin CI in Formel (I) ein Halogenion, ein Arylsulfonation, ein Aryldisulfonation, ein Alkylsulfation, ein Sulfation, ein Thiocyanation, ein Perchloration, ein Tetrafluorboration, ein Hexafluorphosphation, ein Acetation, ein Trifluormethansulfonation, ein Ammoniumion, ein Alkalimetallion oder ein Wasserstoffion ist.

10. Farbstoff-sensibilisierte Solarzelle, umfassend das fotoelektrische Umwandlungselement gemäß irgendeinem der Ansprüche 1 bis 9.

11. Metallkomplexfarbstoff, dargestellt durch die Formel (I):
**M(LD)(LA)(X)_{mX}·(CI)_{mY}** Formel (I)
worin M Fe²⁺, Ru²⁺ oder Os²⁺ darstellt;
LD einen durch die Formel (DL) dargestellten Liganden darstellt;
LA einen zweizähnigen oder dreizähnigen Liganden darstellt, der ein stickstoffhaltiges aromatisches heterocyclisches Gerüst und mindestens eine saure Gruppe aufweist;
X einen einzähnigen oder zweizähnigen Liganden darstellt; mX eine ganze Zahl von 0 bis 3 darstellt;
CI ein Gegenion darstellt, das zum Neutralisieren einer elektrischen Ladung notwendig ist; und
mY eine ganze Zahl von 0 bis 2 darstellt; 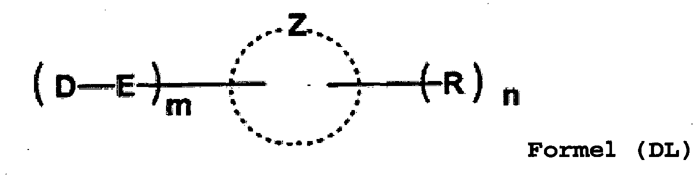
worin Z eine Gruppe von Nicht-Metallatomen darstellt, die zum Bilden eines Rings notwendig ist, und als ringbildendes Atom ein koordinierendes Atom enthalten kann, welches an das vorstehend erwähnte M koordiniert;
D ein koordinierendes Atom darstellt, ausgewählt aus einem Sauerstoffatom und einem Stickstoffatom, und welches an das vorstehend erwähnte M koordiniert; E eine Einfachbindung, -O-, -N(R¹)-, -C(R²)₂-, -C(=R³)-, -C(=O)-, -C(=NR¹)- oder -C(R²)₂-C(=O)- darstellt, worin R¹ und R² jeweils unabhängig ein Wasserstoffatom, eine Alkylgruppe, eine Arylgruppe, eine Heteroarylgruppe, eine Alkoxygruppe oder eine Aryloxygruppe darstellen; R³ eine Alkylidengruppe, eine Alkenylidengruppe oder eine Cycloalkylidengruppe darstellt; wobei zwei R² gleich oder voneinander verschieden sein können; R einen Substituenten darstellt; m und n jeweils unabhängig eine ganze Zahl von 1 oder größer darstellen;
worin mindestens ein koordinierendes Atom ein koordinierendes Atom A ist und unter den R in der Anzahl n zumindest eines ein Substituent W ist und D niemals einen Ring mit E oder Z bildet;
worin das koordinierende Atom A ein Sauerstoff- oder Stickstoffatom ist, welches keinen ungesättigten Bindungsarm aufweist, und der Substituent W eine Ethenylgruppe, eine Ethinylgruppe, eine Arylgruppe oder eine Heteroarylgruppe ist, von denen jede mit einer monovalenten Alkylgruppe mit 6 bis 18 Kohlenstoffatomen oder mit einem Substituenten, der die Alkylgruppe aufweist, substituiert ist.

12. Metallkomplexfarbstoff gemäß Anspruch 11, worin der durch die Formel (DL) dargestellte Ligand durch irgendeine der Formeln (CL-1) bis (DL-3) dargestellt wird: 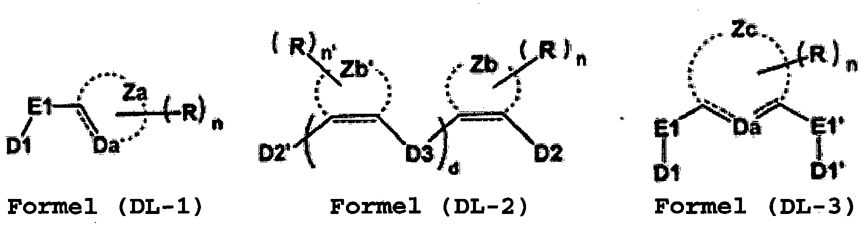 worin R und n jeweils die gleiche Bedeutung wie R und n in Formel (DL) besitzen; n' eine ganze Zahl von 0 oder größer darstellt; Za, Zb, Zb' und Zc jeweils eine Gruppe von Nicht-Metallatomen darstellen, die zum Bilden eines Rings erforderlich ist; die Bindung, die das Kohlenstoffatom, an das -E1-D1 gebunden ist, und Da verknüpft, die Bindung, die das Kohlenstoffatom, an das D2 oder D2' gebunden ist, und das Kohlenstoffatom, an welches D3 gebunden ist, verknüpft, und die Bindung, die das Kohlenstoffatom, an welches -E1-D1 oder -E1'-D1' gebunden ist, und Da verknüpft, entweder eine Einfachbindung oder eine Doppelbindung sein kann; D1 bis D3, D1', D2' und Da jeweils unabhängig ein koordinierendes Atom darstellen, welches an M koordiniert; D1 bis D3, D1' und D2' jeweils ein Atom darstellen, ausgewählt aus der Gruppe, bestehend aus einem Sauerstoffatom und einem Stickstoffatom; Da ein Atom darstellt, ausgewählt aus der Gruppe, bestehend aus einem Sauerstoffatom, einem Stickstoffatom und einem Kohlenstoffatom; E1 und E1' jeweils unabhängig eine Einfachbindung, -O-, -N(R¹)-, -C(R²)₂-, -C(=R³)-, -C(=O)-, -C(=NR¹)- oder -C(R²)₂-C(=O)- darstellt, worin R¹ und R² jeweils unabhängig ein Wasserstoffatom, eine Alkylgruppe, eine Arylgruppe, eine Heteroarylgruppe, eine Alkoxygruppe oder eine Aryloxygruppe darstellen; R³ eine Alkylidengruppe, eine Alkenylidengruppe oder eine Cycloalkylidengruppe darstellt; zwei R² gleich oder voneinander verschieden sein können; d 0 oder 1 darstellt; wenn d 1 ist, Zb und Zb' miteinander verbunden sein können, um einen Ring zu bilden; und wenn n und n' jeweils eine ganze Zahl von 2 oder größer sind, mehrere R miteinander verbunden sein können, um einen Ring zu bilden.

13. Metallkomplexfarbstoff gemäß Anspruch 11 oder 12, worin der durch die Formel (DL) dargestellte Ligand durch irgendeine der Formeln (DL-1a), (DL-1b), (DL-2a), (DL-2b), (DL-3a) und (DL-3b) dargestellt wird: 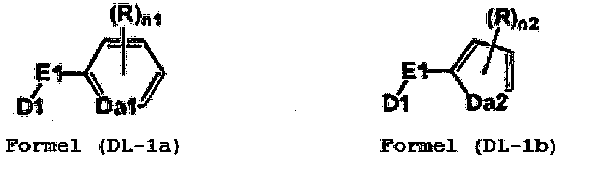 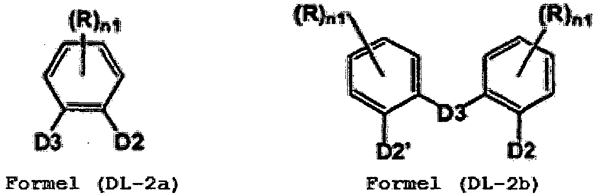 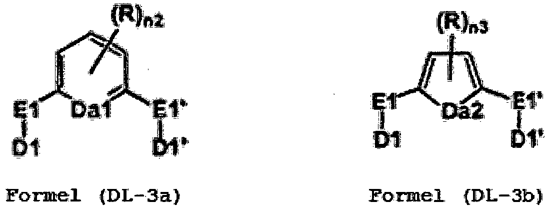 worin R die gleiche Bedeutung wie R in Formel (DL) besitzt; n1 eine ganze Zahl von 1 bis 4 darstellt; n2 eine ganze Zahl von 1 bis 3 darstellt; n3 1 oder 2 darstellt; n1' eine ganze Zahl von 0 bis 4 darstellt; D1 bis D3, D1', D2', Da1 und Da2 jeweils unabhängig ein koordinierendes Atom darstellen, welches an M koordiniert; D1 bis D3, D1' und D2' jeweils ein Atom darstellen, ausgewählt aus der Gruppe, bestehend aus einem Sauerstoffatom und einem Stickstoffatom; Da1 und Da2 jeweils ein Atom darstellen, ausgewählt aus der Gruppe, bestehend aus einem Sauerstoffatom, einem Stickstoffatom und einem Kohlenstoffatom; E1 und E1' jeweils unabhängig eine Einfachbindung, , -O-, -N(R¹)-, -C(R²)₂-, -C(=R³)-, -C(=O)-, -C(=NR¹)- oder-C(R²)₂-C(=O)- darstellen, worin R¹ und R² jeweils unabhängig ein Wasserstoffatom, eine Alkylgruppe, eine Arylgruppe, eine Heteroarylgruppe, eine Alkoxygruppe oder eine Aryloxygruppe darstellen; R³ eine Alkylidengruppe, eine Alkenylidengruppe oder eine Cycloalkylidengruppe darstellt; zwei R² gleich oder voneinander verschieden sein können; und wenn n1 bis n3 und n1' jeweils eine ganze Zahl von 2 oder größer sind, die mehreren R miteinander verbunden sein können, um einen Ring zu bilden.

14. Metallkomplexfarbstoff gemäß irgendeinem der Ansprüche 11 bis 13, worin der Substituent W 1) eine Ethenylgruppe oder eine Ethinylgruppe ist, die an Position 2 eine Arylgruppe oder eine Heteroarylgruppe aufweist, welche mit einer Alkylgruppe mit 6 bis 18 Kohlenstoffatomen, einer Alkylaminogruppe mit 6 bis 18 Kohlenstoffatomen, einer Alkoxygruppe mit 6 bis 18 Kohlenstoffatomen oder einer Alkylthiogruppe mit 6 bis 18 Kohlenstoffatomen substituiert ist, oder 2) eine Arylgruppe oder Heteroarylgruppe, die mit einer Alkylgruppe mit 6 bis 18 Kohlenstoffatomen, einer Alkylaminogruppe mit 6 bis 18 Kohlenstoffatomen, einer Alkoxygruppe mit 6 bis 18 Kohlenstoffatomen oder einer Alkylthiogruppe mit 6 bis 18 Kohlenstoffatomen substituiert ist, ist.

15. Metallkomplexfarbstoff gemäß irgendeinem der Ansprüche 11 bis 14, worin der Substituent W durch irgendeine der Formeln (W-1) bis (W-6) dargestellt wird: 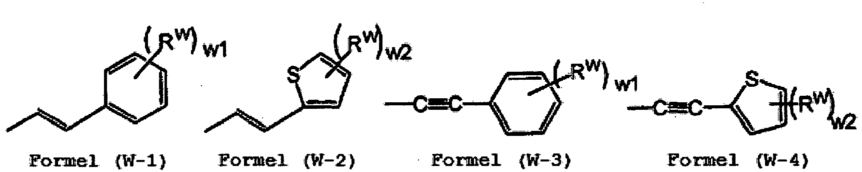 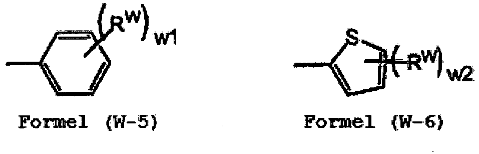 worin R^{W} eine monovalente Alkylgruppe mit 6 bis 18 Kohlenstoffatomen oder eine Gruppe, die die Alkylgruppe aufweist, darstellt; w1 eine ganze Zahl von 1 bis 5 darstellt; und w2 eine ganze Zahl von 1 bis 3 darstellt.

16. Metallkomplexfarbstoff gemäß irgendeinem der Ansprüche 11 bis 15, worin LA durch irgendeine der Formeln (AL-1) bis (AL-6) dargestellt wird: 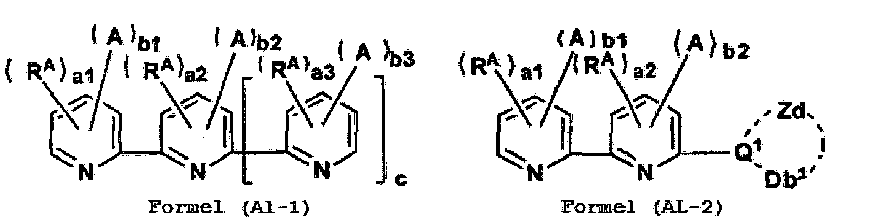 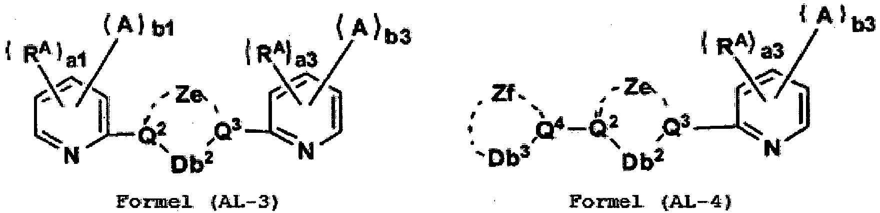 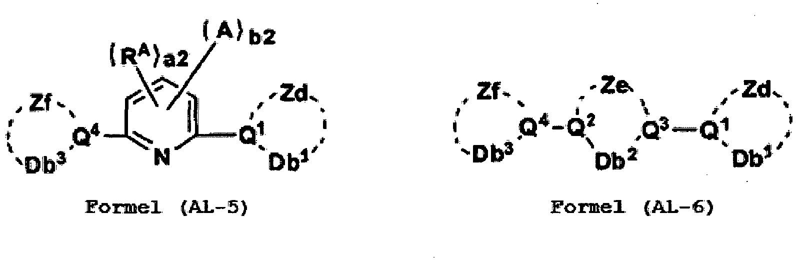
worin Zd, Ze und Zf jeweils unabhängig einen Benzolring, einen Pyrrolring, einen Imidazolring, einen Pyrazolring, einen Pyrazinring, einen Pyrimidinring, einen Pyridazinring, einen Triazolring, einen Oxazolring, einen Triazinring, einen Thiazolring, einen Isothiazolring, einen Oxazolring, einen Isoxazolring, einen Furanring, einen Thiophenring, einen Pyrrolidinring, einen Piperidinring, einen Morpholinring, einen Piperazinring, einen Tetrahydrofuranring, einen Tetrahydropyranring, einen 4H-Pyranring, einen 1,4-Dihydropyridinring, einen Tetradehydromorpholinring oder einen Benzolkondensierten Ring dieser Ringe darstellt; A eine saure Gruppe darstellt; Q¹ bis Q⁴ jeweils unabhängig ein Kohlenstoffatom oder ein Stickstoffatom darstellen; Db¹ bis Db³ jeweils unabhängig ein Stickstoffatom mit einem einsamen Elektronenpaar, ein anionisches Stickstoffatom oder ein anionisches Kohlenstoffatom darstellen; R^{A} einen Substituenten darstellt; a1, a3, b1 und b3 jeweils unabhängig eine ganze Zahl von 0 bis 4 darstellen; a2 und b2 jeweils unabhängig eine ganze Zahl von 0 bis 3 darstellen; c 0 oder 1 darstellt; und
die durch die Formeln (AL-1) bis (AL-6) dargestellten Liganden mindestens eine saure Gruppe aufweisen.

17. Metallkomplexfarbstoff gemäß irgendeinem der Ansprüche 11 bis 16, worin LA durch die Formel (AL-1) dargestellt wird: 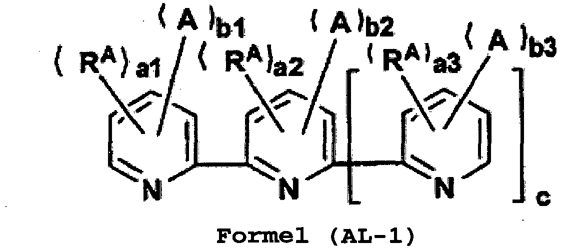
worin A eine saure Gruppe darstellt; R^{A} einen Substituenten darstellt; a1, a3, b1 und b3 jeweils unabhängig eine ganze Zahl von 0 bis 4 darstellen; a2 und b2 jeweils unabhängig eine ganze Zahl von 0 bis 3 darstellen; c 0 oder 1 darstellt; und
der durch die Formel (AL-1) dargestellte Ligand mindestens eine saure Gruppe aufweist.

18. Farbstofflösung, umfassend den Metallkomplexfarbstoff gemäß irgendeinem der Ansprüche 11 bis 17, gelöst in einem organischen Lösungsmittel

19. Farbstoff-adsorbierte Elektrode für eine Farbstoff-sensibilisierte Solarzelle, die eine Fotoleiterschicht aufweist, die durch Beschichten eines elektrisch leitfähigen Trägers, auf den Halbleiter-Feinpartikel aufgetragen worden sind, mit einer Farbstofflösung gemäß Anspruch 18, gefolgt von Umsetzen und Härten der Farbstofflösung, erhalten ist.

## Revendications

1. Élément de conversion photoélectrique, contenant :
un support électroconducteur ;
une couche photoconductrice contenant un électrolyte ;
une couche de transfert de charge contenant un électrolyte ; et
une contre-électrode ;
dans lequel la couche photoconductrice contient de fines particules de semi-conducteur portant un colorant de type complexe métallique représenté par la formule (I) :
M(LD)(LA)(X)_{mX}·(CI)_{mY} Formule (I)
dans laquelle M représente Fe²⁺, Ru²⁺ ou Os²⁺ ;
LD représente un ligand représenté par la formule (DL) ;
LA représente un ligand bidentate ou tridentate ayant un squelette hétérocyclique aromatique azoté et au moins un groupe acide;
X représente un ligand monodentate ou bidentate; mX représente un nombre entier de 0 à 3 ;
Cl représente un contre-ion nécessaire pour neutraliser une charge électrique; et
mY représente un nombre entier de 0 à 2 ; 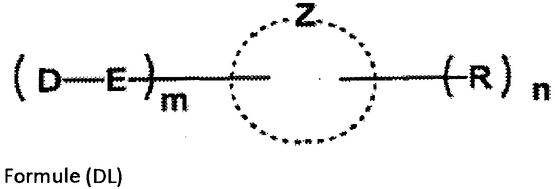
dans laquelle Z représente un groupe d'atomes non métalliques nécessaires pour former un cycle, et il peut contenir, en tant qu'atome de formation de cycle, un atome de coordination qui se coordonne au M susmentionné; D représente un atome de coordination sélectionné parmi un atome d'oxygène et un atome d'azote et se coordonne au M susmentionné; E représente une liaison simple, -O-, -N(R¹)-, -C(R²)₂-, -C(=R³)-, -C(=O)-, -C(=NR¹)-, ou -C(R²)₂-C(=O)-, dans lesquels R¹ et R² représentent chacun indépendamment un atome d'hydrogène, un groupe alkyle, un groupe aryle, un groupe hétéroaryle, un groupe alcoxy, ou un groupe aryloxy ; R³ représente un groupe alkylidène, un groupe alcénylidène, ou un groupe cycloalkylidène; deux R² peuvent être identiques ou différents l'un de l'autre; R représente un substituant; m et n représentent chacun indépendamment un nombre entier égal à 1 ou plus ;
dans lequel au moins un atome de coordination est un atome de coordination A et, parmi des R au nombre de n, au moins un est un substituant W, et D ne forme jamais un cycle avec E ou Z;
ici, l'atome de coordination A est un atome d'oxygène ou d'azote qui n'a pas un bras de liaison insaturé, et le substituant W est un groupe éthènyle, un groupe éthynyle, un groupe aryle, ou un groupe hétéroaryle, chacun parmi ceux-ci étant substitué avec un groupe alkyle monovalent contenant 6 à 18 atomes de carbone ou avec un substituant contenant le groupe alkyle.

2. Élément de conversion photoélectrique selon la revendication 1, dans lequel le ligand représenté par la formule (DL) est représenté par l'une quelconque des formules (DL-1) à (DL-3) : 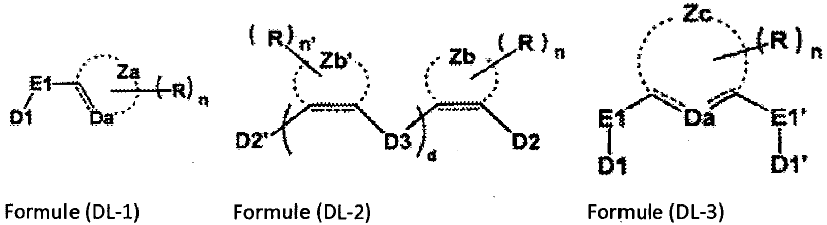 dans lesquelles R et n ont chacun la même signification que R et n dans la formule (DL); n' représente un nombre entier égal à 0 ou plus; Za, Zb, Zb' et Zc représentent chacun un groupe d'atomes non métalliques nécessaires pour former un cycle ; la liaison reliant l'atome de carbone auquel -E1-D1 est fixé et Da, la liaison reliant l'atome de carbone auquel D2 ou D2' est fixé et l'atome de carbone auquel D3 est fixé, et la liaison reliant l'atome de carbone auquel -E1-D1 ou -E1'-D1' est fixé et Da peuvent être soit une liaison simple, soit une liaison double; D1 à D3, D1', D2' et Da représentent chacun indépendamment un atome de coordination qui se coordonne à M ; D1 à D3, D1' et D2' représentent chacun un atome sélectionné dans le groupe constitué d'un atome d'oxygène et d'un atome d'azote; Da représente un atome sélectionné dans le groupe constitué d'un atome d'oxygène, d'un atome d'azote, et d'un atome de carbone; E1 et E1' représentent chacun indépendamment une liaison simple, -O-, -N(R¹)-, -C(R²)₂-, -C(=R³)-, -C(=O)-, -C(=NR¹)-, ou -C(R²)₂-C(=O)-, dans lesquels R¹ et R² représentent chacun indépendamment un atome d'hydrogène, un groupe alkyle, un groupe aryle, un groupe hétéroaryle, un groupe alcoxy, ou un groupe aryloxy ; R³ représente un groupe alkylidène, un groupe alcénylidène, ou un groupe cycloalkylidène; deux R² peuvent être identiques ou différents l'un de l'autre ; d représente 0 ou 1; quand d est 1, Zb et Zb' peuvent être liés l'un à l'autre pour former un cycle; et quand n et n' sont chacun un nombre entier égal à 2 ou plus, plusieurs R peuvent être liés les uns aux autres pour former un cycle.

3. Élément de conversion photoélectrique selon la revendication 1 ou 2, dans lequel le ligand représenté par la formule (DL) est représenté par l'une quelconque des formules (DL-1a), (DL-1b), (DL-2a), (DL-2b), (DL-3a), et (DL-3b) : 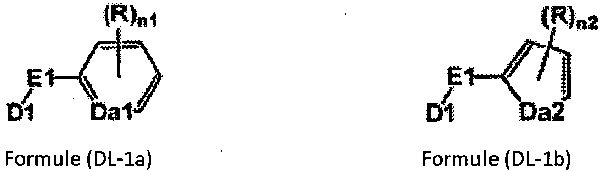 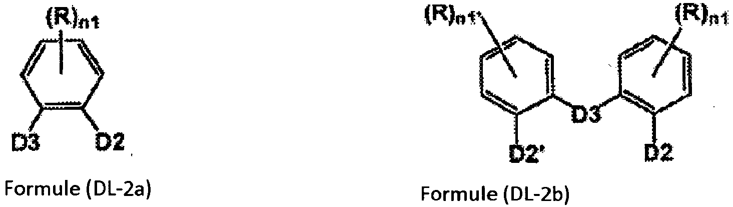 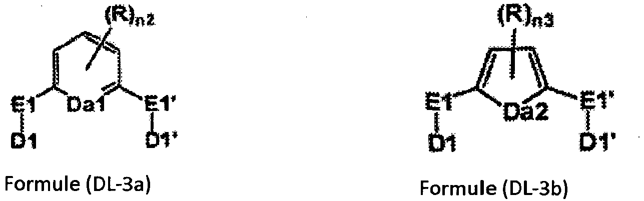 dans lesquelles R a la même signification que R dans la formule (DL); n1 représente un nombre entier de 1 à 4; n2 représente un nombre entier de 1 à 3 ; n3 représente 1 ou 2 ; n1' représente un nombre entier de 0 à 4 ; D1 à D3, D1', D2', Da1, et Da2 représentent chacun indépendamment un atome de coordination qui se coordonne à M ; D1 à D3, D1', et D2' représentent chacun un atome sélectionné dans le groupe constitué d'un atome d'oxygène et d'un atome d'azote; Da1 et Da2 représentent chacun un atome sélectionné dans le groupe constitué d'un atome d'oxygène, d'un atome d'azote et d'un atome de carbone ; E1 et E1' représentent chacun indépendamment une liaison simple, -O-, -N(R¹)-, -C(R²)₂-, -C(=R³)-, -C(=O)-, -C(=NR¹)-, ou -C(R²)₂-C(=O)-, dans lesquels R¹ et R² représentent chacun indépendamment un atome d'hydrogène, un groupe alkyle, un groupe aryle, un groupe hétéroaryle, un groupe alcoxy, ou un groupe aryloxy ; R³ représente un groupe alkylidène, un groupe alcénylidène, ou un groupe cycloalkylidène ; deux R² peuvent être identiques ou différents l'un de l'autre; et quand n1 à n3 et n1' sont chacun un nombre entier égal à 2 ou plus, plusieurs R peuvent être liés les uns aux autres pour former un cycle.

4. Élément de conversion photoélectrique selon l'une quelconque des revendications 1 à 3, dans lequel le substituant W est 1) un groupe éthènyle ou un groupe éthynyle présentant, en position 2, un groupe aryle ou un groupe hétéroaryle qui est substitué avec un groupe alkyle contenant 6 à 18 atomes de carbone, un groupe alkylamino contenant 6 à 18 atomes de carbone, un groupe alcoxy contenant 6 à 18 atomes de carbone, ou un groupe alkylthio contenant 6 à 18 atomes de carbone, ou 2) un groupe aryle ou un groupe hétéroaryle substitué avec un groupe alkyle contenant 6 à 18 atomes de carbone, un groupe alkylamino contenant 6 à 18 atomes de carbone, un groupe alcoxy contenant 6 à 18 atomes de carbone, ou un groupe alkylthio contenant 6 à 18 atomes de carbone.

5. Élément de conversion photoélectrique selon l'une quelconque des revendications 1 à 4, dans lequel le substituant W est représenté par l'une quelconque des formules (W-1) à (W-6) : 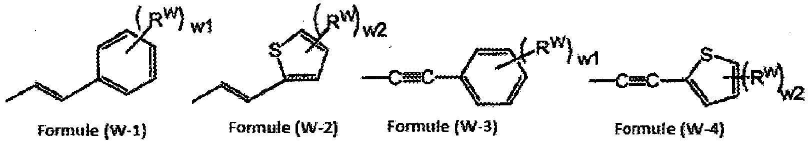 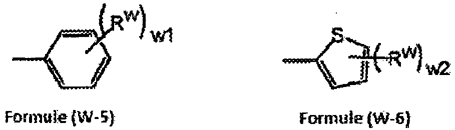 dans lesquelles R^{W} représente un groupe alkyle monovalent contenant 6 à 18 atomes de carbone ou un groupe contenant le groupe alkyle; w1 représente un nombre entier de 1 à 5 ; et w2 représente un nombre entier de 1 à 3.

6. Élément de conversion photoélectrique selon l'une quelconque des revendications 1 à 5, dans lequel LA est représenté par l'une quelconque des formules (AL-1) à (AL-6) : 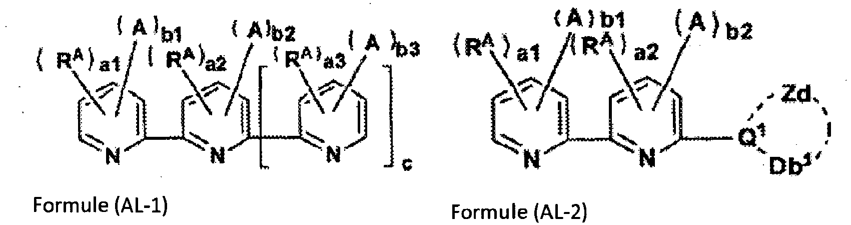 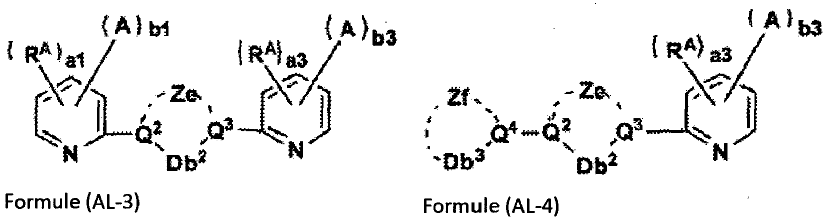 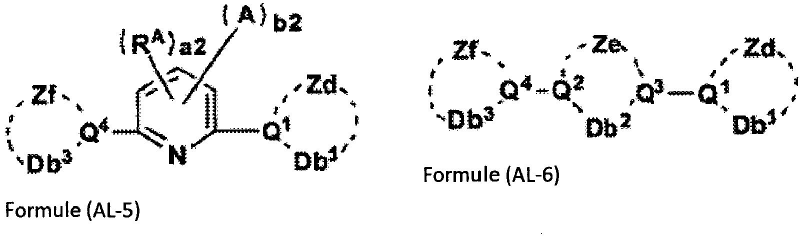
dans lesquelles Zd, Ze, et Zf représentent chacun indépendamment un cycle benzène, un cycle pyrrole, un cycle imidazole, un cycle pyrazole, un cycle pyrazine, un cycle pyrimidine, un cycle pyridazine, un cycle triazole, un cycle oxazole, un cycle triazine, un cycle thiazole, un cycle isothiazole, un cycle oxazole, un cycle isoxazole, un cycle furane, un cycle thiophène, un cycle pyrrolidine, un cycle pipéridine, un cycle morpholine, un cycle pipérazine, un cycle tétrahydrofurane, un cycle tétrahydropyrane, un cycle 4H-pyrane, un cycle 1,4-dihydropyridine, un cycle tétradéshydromorpholine, ou un cycle parmi ces cycles qui est fusionné à un benzène; A représente un groupe acide; Q¹ à Q⁴ représentent chacun indépendamment un atome de carbone ou un atome d'azote; Db¹ à Db³ représentent chacun indépendamment un atome d'azote ayant une paire d'électrons libres, un atome d'azote anionique, ou un atome de carbone anionique; R^{A} représente un substituant; a1, a3, b1, et b3 représentent chacun indépendamment un nombre entier de 0 à 4; a2 et b2 représentent chacun indépendamment un nombre entier de 0 à 3 ; c représente 0 ou 1; et
les ligands représentés par les formules (AL-1) à (AL-6) contiennent au moins un groupe acide.

7. Élément de conversion photoélectrique selon l'une quelconque des revendications 1 à 6, dans lequel LA est représenté par la formule (AL-1) : 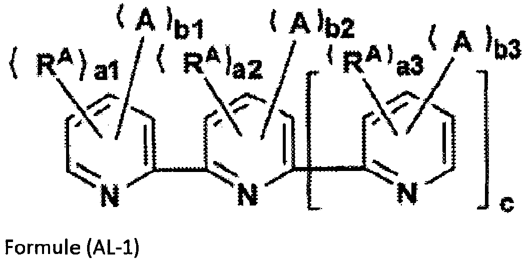
dans laquelle A représente un groupe acide; R^{A} représente un substituant; a1, a3, b1 et b3 représentent chacun indépendamment un nombre entier de 0 à 4; a2 et b2 représentent chacun indépendamment un nombre entier de 0 à 3 ; c représente 0 ou 1 ; et
le ligand représenté par la formule (AL-1) contient au moins un groupe acide.

8. Élément de conversion photoélectrique selon l'une quelconque des revendications 1 à 7, dans lequel X dans la formule (I) est NCS⁻, Cl⁻, Br⁻, I⁻, CN⁻, NCO⁻, ou H₂O.

9. Élément de conversion photoélectrique selon l'une quelconque des revendications 1 à 8, dans lequel Cl dans la formule (I) est un ion halogène, un ion arylsulfonate, un ion aryldisulfonate, un ion alkylsulfate, un ion sulfate, un ion thiocyanate, un ion perchlorate, un ion tétrafluoroborate, un ion hexafluorophosphate, un ion acétate, un ion trifluorométhanesulfonate, un ion ammonium, un ion de métal alcalin, ou un ion hydrogène.

10. Cellule solaire sensibilisée par colorant, comprenant l'élément de conversion photoélectrique selon l'une quelconque des revendications 1 à 9.

11. Colorant complexe métallique représenté par la formule (I) :
M(LD)(LA)(X)_{mX}·(CI)_{mY} Formule (I)
dans laquelle M représente Fe²⁺, Ru²⁺ ou Os²⁺ ;
LD représente un ligand représenté par la formule (DL) ;
LA représente un ligand bidentate ou tridentate ayant un squelette hétérocyclique aromatique azoté et au moins un groupe acide ;
X représente un ligand monodentate ou bidentate; mX représente un nombre entier de 0 à 3 ;
CI représente un contre-ion nécessaire pour neutraliser une charge électrique ; et
mY représente un nombre entier de 0 à 2 ; 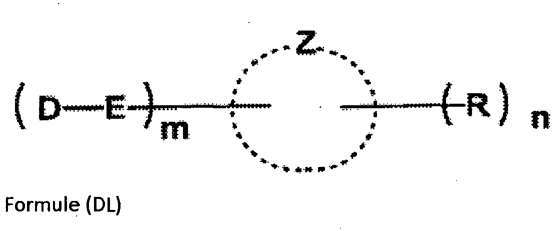
dans laquelle Z représente un groupe d'atomes non métalliques nécessaires pour former un cycle, et il peut contenir, en tant qu'atome de formation de cycle, un atome de coordination qui se coordonne au M susmentionné; D représente un atome de coordination sélectionné parmi un atome d'oxygène et un atome d'azote et se coordonne au M susmentionné; E représente une liaison simple, -O-, -N(R¹)-, -C(R²)₂-, -C(=R³)-, -C(=O)-, -C(=NR¹)-, ou -C(R²)₂-C(=O)-, dans lesquels R¹ et R² représentent chacun indépendamment un atome d'hydrogène, un groupe alkyle, un groupe aryle, un groupe hétéroaryle, un groupe alcoxy, ou un groupe aryloxy ; R³ représente un groupe alkylidène, un groupe alcénylidène, ou un groupe cycloalkylidène ; deux R² peuvent être identiques ou différents l'un de l'autre ; R représente un substituant ; m et n représentent chacun indépendamment un nombre entier égal à 1 ou plus ;
dans lequel au moins un atome de coordination est un atome de coordination A et, parmi des R au nombre de n, au moins un est un substituant W, et D ne forme jamais un cycle avec E ou Z ;
ici, l'atome de coordination A est un atome d'oxygène ou d'azote qui n'a pas un bras de liaison insaturé, et le substituant W est un groupe éthènyle, un groupe éthynyle, un groupe aryle, ou un groupe hétéroaryle, chacun parmi ceux-ci étant substitué avec un groupe alkyle monovalent contenant 6 à 18 atomes de carbone ou avec un substituant contenant le groupe alkyle.

12. Colorant complexe métallique selon la revendication 11, dans lequel le ligand représenté par la formule (DL) est représenté par l'une quelconque des formules (DL-1) à (DL-3) : 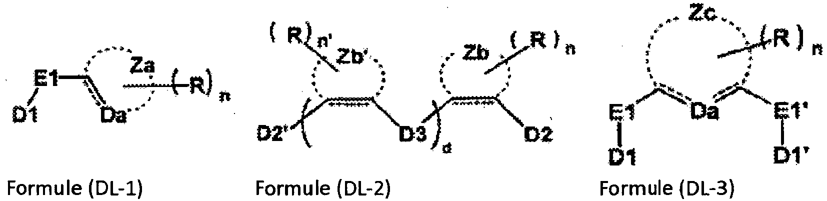 dans lesquelles R et n ont chacun la même signification que R et n dans la formule (DL) ; n' représente un nombre entier égal à 0 ou plus; Za, Zb, Zb' et Zc représentent chacun un groupe d'atomes non métalliques nécessaires pour former un cycle; la liaison reliant l'atome de carbone auquel -E1-D1 est fixé et Da, la liaison reliant l'atome de carbone auquel D2 ou D2' est fixé et l'atome de carbone auquel D3 est fixé, et la liaison reliant l'atome de carbone auquel -E1-D1 ou -E1'-D1' est fixé et Da peuvent être soit une liaison simple, soit une liaison double; D1 à D3, D1', D2' et Da représentent chacun indépendamment un atome de coordination qui se coordonne à M ; D1 à D3, D1' et D2' représentent chacun un atome sélectionné dans le groupe constitué d'un atome d'oxygène et d'un atome d'azote; Da représente un atome sélectionné dans le groupe constitué d'un atome d'oxygène, d'un atome d'azote, et d'un atome de carbone; E1 et E1' représentent chacun indépendamment une liaison simple, -O-, -N(R¹)-, -C(R²)₂-, -C(=R³)-, -C(=O)-, -C(=NR¹)-, ou -C(R²)₂-C(=O)-, dans lesquels R¹ et R² représentent chacun indépendamment un atome d'hydrogène, un groupe alkyle, un groupe aryle, un groupe hétéroaryle, un groupe alcoxy, ou un groupe aryloxy ; R³ représente un groupe alkylidène, un groupe alcénylidène, ou un groupe cycloalkylidène ; deux R² peuvent être identiques ou différents l'un de l'autre; d représente 0 ou 1; quand d est 1, Zb et Zb' peuvent être liés l'un à l'autre pour former un cycle; et quand n et n' sont chacun un nombre entier égal à 2 ou plus, plusieurs R peuvent être liés les uns aux autres pour former un cycle.

13. Colorant complexe métallique selon la revendication 11 ou 12, dans lequel le ligand représenté par la formule (DL) est représenté par l'une quelconque des formules (DL-1a), (DL-1b), (DL-2a), (DL-2b), (DL-3a), et (DL-3b) : 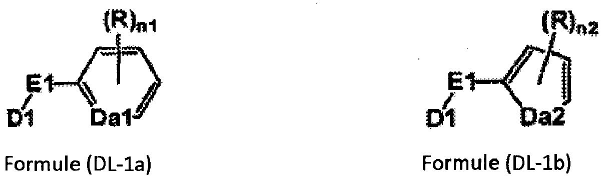 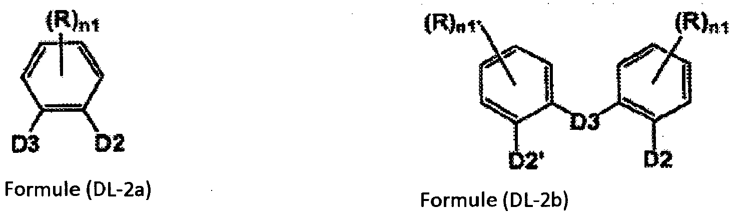 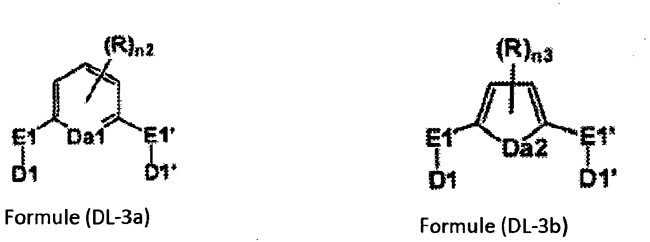 dans lesquelles R a la même signification que R dans la formule (DL); n1 représente un nombre entier de 1 à 4; n2 représente un nombre entier de 1 à 3 ; n3 représente 1 ou 2; n1' représente un nombre entier de 0 à 4; D1 à D3, D1', D2', Da1, et Da2 représentent chacun indépendamment un atome de coordination qui se coordonne à M; D1 à D3, D1', et D2' représentent chacun un atome sélectionné dans le groupe constitué d'un atome d'oxygène et d'un atome d'azote; Da1 et Da2 représentent chacun un atome sélectionné dans le groupe constitué d'un atome d'oxygène, d'un atome d'azote et d'un atome de carbone; E1 et E1' représentent chacun indépendamment une liaison simple, -O-, -N(R¹)-, -C(R²)₂-, -C(=R³)-, -C(=O)-, -C(=NR¹)-, ou -C(R²)₂-C(=O)-, dans lesquels R¹ et R² représentent chacun indépendamment un atome d'hydrogène, un groupe alkyle, un groupe aryle, un groupe hétéroaryle, un groupe alcoxy, ou un groupe aryloxy ; R³ représente un groupe alkylidène, un groupe alcénylidène, ou un groupe cycloalkylidène ; deux R² peuvent être identiques ou différents l'un de l'autre ; et quand n1 à n3 et n1' sont chacun un nombre entier égal à 2 ou plus, plusieurs R peuvent être liés les uns aux autres pour former un cycle.

14. Colorant complexe métallique selon l'une quelconque des revendications 11 à 13, dans lequel le substituant W est 1) un groupe éthènyle ou un groupe éthynyle présentant, en position 2, un groupe aryle ou un groupe hétéroaryle qui est substitué avec un groupe alkyle contenant 6 à 18 atomes de carbone, un groupe alkylamino contenant 6 à 18 atomes de carbone, un groupe alcoxy contenant 6 à 18 atomes de carbone, ou un groupe alkylthio contenant 6 à 18 atomes de carbone, ou 2) un groupe aryle ou un groupe hétéroaryle substitué avec un groupe alkyle contenant 6 à 18 atomes de carbone, un groupe alkylamino contenant 6 à 18 atomes de carbone, un groupe alcoxy contenant 6 à 18 atomes de carbone, ou un groupe alkylthio contenant 6 à 18 atomes de carbone.

15. Colorant complexe métallique selon l'une quelconque des revendications 11 à 14, dans lequel le substituant W est représenté par l'une quelconque des formules (W-1) à (W-6) : 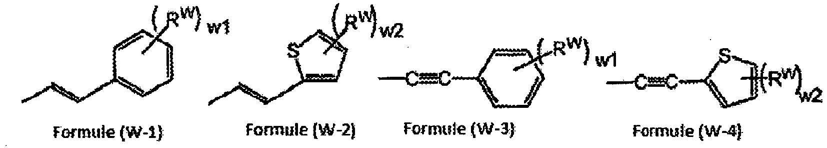 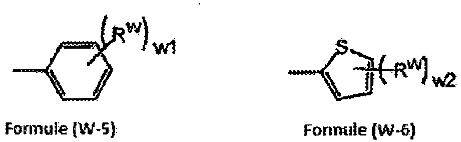 dans lesquelles R^{W} représente un groupe alkyle monovalent contenant 6 à 18 atomes de carbone ou un groupe contenant le groupe alkyle ; w1 représente un nombre entier de 1 à 5 ; et w2 représente un nombre entier de 1 à 3.

16. Colorant complexe métallique selon l'une quelconque des revendications 11 à 15, dans lequel LA est représenté par l'une quelconque des formules (AL-1) à (AL-6) : 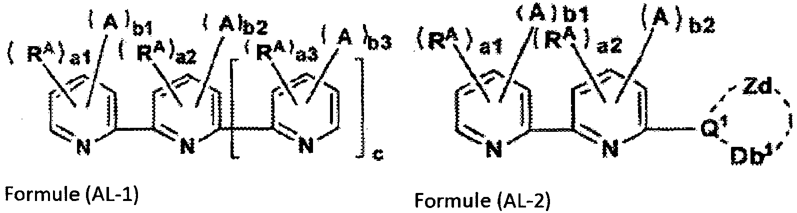 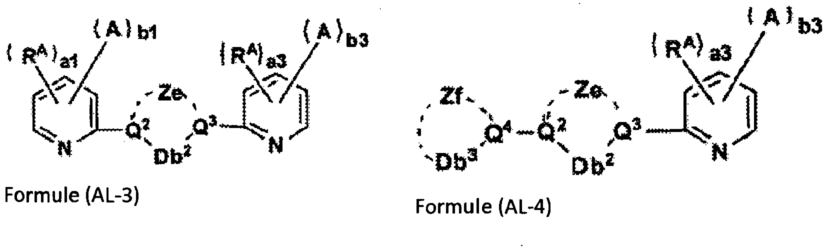 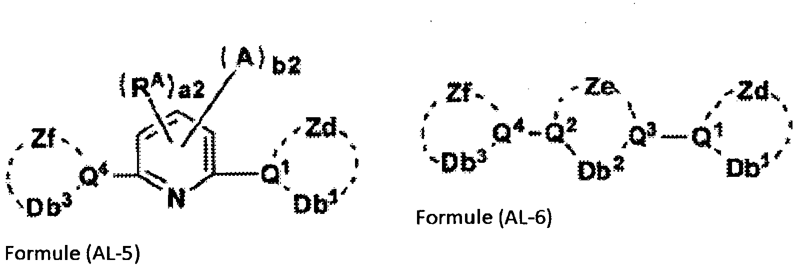
dans lesquelles Zd, Ze, et Zf représentent chacun indépendamment un cycle benzène, un cycle pyrrole, un cycle imidazole, un cycle pyrazole, un cycle pyrazine, un cycle pyrimidine, un cycle pyridazine, un cycle triazole, un cycle oxazole, un cycle triazine, un cycle thiazole, un cycle isothiazole, un cycle oxazole, un cycle isoxazole, un cycle furane, un cycle thiophène, un cycle pyrrolidine, un cycle pipéridine, un cycle morpholine, un cycle pipérazine, un cycle tétrahydrofurane, un cycle tétrahydropyrane, un cycle 4H-pyrane, un cycle 1,4-dihydropyridine, un cycle tétradéshydromorpholine, ou un cycle parmi ces cycles qui est fusionné à un benzène ; A représente un groupe acide ; Q¹ à Q⁴ représentent chacun indépendamment un atome de carbone ou un atome d'azote ; Db¹ à Db³ représentent chacun indépendamment un atome d'azote ayant une paire d'électrons libres, un atome d'azote anionique, ou un atome de carbone anionique; R^{A} représente un substituant; a1, a3, b1, et b3 représentent chacun indépendamment un nombre entier de 0 à 4; a2 et b2 représentent chacun indépendamment un nombre entier de 0 à 3 ; c représente 0 ou 1 ; et
les ligands représentés par les formules (AL-1) à (AL-6) contiennent au moins un groupe acide.

17. Colorant complexe métallique selon l'une quelconque des revendications 11 à 16, dans lequel LA est représenté par la formule (AL-1) : 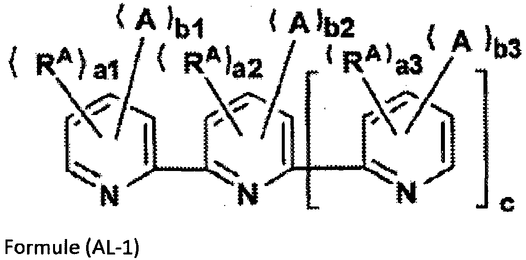
dans laquelle A représente un groupe acide ; R^{A} représente un substituant ; a1, a3, b1 et b3 représentent chacun indépendamment un nombre entier de 0 à 4; a2 et b2 représentent chacun indépendamment un nombre entier de 0 à 3 ; c représente 0 ou 1 ; et
le ligand représenté par la formule (AL-1) contient au moins un groupe acide.

18. Solution de colorant, comprenant le colorant complexe métallique selon l'une quelconque des revendications 11 à 17 dissous dans un solvant organique.

19. Électrode à colorant adsorbé pour une cellule solaire sensibilisée par colorant, ayant une couche photoconductrice qui est obtenue par revêtement d'un support électroconducteur auquel ont été appliquées de fines particules de semi-conducteur avec une solution de colorant selon la revendication 18, puis par réaction et durcissement de la solution de colorant.
